# EUROPEAN PATENT SPECIFICATION

(11) **EP 3 260 137 B1**
(45) Date of publication and mention of the grant of the patent: **12.08.2020**
(21) Application number: 17168480.6
(22) Date of filing: 27.01.2009
(51) Int. Cl.: A61K 39/145

(54) **CANINE INFLUENZA VACCINES**
IMPFSTOFF GEGEN HUNDEGRIPPE
VACCINS CONTRE LA GRIPPE CANINE

(30) Priority: 28.01.2008 US 20656
(43) Date of publication of application: 27.12.2017
(62) Divisional of application: 09706780.5
(73) Proprietor: Boehringer Ingelheim Animal Health USA Inc., Duluth, GA 30096 (US)
(72) Inventor: MINKE, Jules Maarten, 06200 Nice (FR); KARACA, Kemal, Overpark, KS 66221 (US); YAO, Jiansheng, North York, Ontario M2H 3H1 (CA)
(74) Representative: D Young & Co LLP

(56) References cited:
- WO-A-2007/024947
- WO-A2-2007/047938
- US-A1- 2008 241 184
- US-B1- 6 436 408
- KARACA KEMAL ET AL: "EVALUATION OF THE ABILITY EQUINE INFLUENZA VIRUS OF CANARYPOX-VECTORED VACCINES TO INDUCE HUMORAL IMMUNE RESPONSES AGAINST CANINE INFLUENZA VIRUSES IN DOGS", AMERICAN JOURNAL OF VETERINARY RESEARCH, AMERICAN VETERINARY MEDICINE ASSOCIATION, US, vol. 68, no. 2, 1 February 2007 (2007-02-01), pages 208-212, XP009081173, ISSN: 0002-9645
- CRAWFORD P C ET AL: "Transmission of Equine influenza virus to dogs", SCIENCE, AMERICAN ASSOCIATION FOR THE ADVANCEMENT OF SCIENCE, US, WASHINGTON, DC, vol. 310, no. 5747, 21 October 2005 (2005-10-21), pages 482-485, XP003003531, ISSN: 0036-8075
- NEWBURY SANDRA ET AL: "Prolonged intermittent virus shedding during an outbreak of canine influenza A H3N2 virus infection in dogs in three Chicago area shelters: 16 cases (March to May 2015)", JOURNAL OF THE AMERICAN VETERINARY MEDICAL ASSOCIATION, vol. 248, no. 9, May 2016 (2016-05), pages 1022-1026,

### SEQUENCE LISTING

<110> Merial, Inc.
<120> CANINE INFLUENZA VACCINES
<130> P041254EPA
<150> US 12/020,656
   <151> 2008-01-28
<160> 51
<170> PatentIn Ver. 3.5
<210> 1
   <211> 3959
   <212> DNA
   <213> Influenza A virus
<400> 1
   gtattctaaa ctaggaatag atgaaattat gtgcaaagga gataccttta gatatggatc 60
   tgatttattt ggtttttcat aatcataatc taacaacatt ttcactatac tataccttct 120
   tgcacaagtc gccattagta gtatagactt atactttgta accatagtat actttagcgc 180
   gtcatcttct tcatctaaaa cagatttaca acaataatca tcgtcgtcat cttcatcttc 240
   attaaagttt tcatattcaa taactttctt ttctaaaaca tcatctgaat caataaacat 300
   agaacggtat agagcgttaa tctccattgt aaaatatact aacgcgttgc tcatgatgta 360
   cttttttttc attatttaga aattatgcat tttagatctt tataagcggc cgtgattaac 420
   tagtcataaa aacccgggat cgattctaga ctcgagcggc cgccagtgtg atggatatct 480
   gcagaattcg gctttggtcc ttactcaaat gcaaatgttg cacctgatgt tgcctttttg 540
   gcaagcccac ataatgaaac ccaatagaac aacgcaaatt aagaagcatg atatggcgaa 600
   tgaaatccac agtatccaat ctttgtagcc tgatttcaac tcaacacttt tgatttgaaa 660
   tcggttgttt aatgcttcat ctctgtatat gtaatggtca tatgtcccat ttcttattga 720
   tccaatgcat gcattatcac atttgtggta aatcttgaaa catccacctc ccatgtcttc 780
   cgcgttttct cttaactggc gcctagtctt ctcgaataat ttattcattt ctgcatctgt 840
   taagtcaatt gtatgttgat tttctagagc caccagcaat tctgcattgt aggaccatag 900
   gtctattttg gtgtcttcta catacttctc caaatcctgg attctccctt ctacttctga 960
   gaattccttt tctatttgat ggaatttctc attggtcctt tcaatcactc tgtttaattt 1020
   tccattgatc tggtctatgg ctgcttgagt gctctttaga tctgcagctt gtcctgttcc 1080
   ttccgagttt tgatatcgga atccatacca cccatcaacc attccttccc agccgttttc 1140
   tatgaatccc gctattgctc caaagattcc tctgatttgc ttttctggta cattcctcat 1200
   cccagtggcc agctttaaag tgttttgcct gatatacttg gggcattttc catatgtaat 1260
   tttgttcaca ttttgaaatg gtttgtcgtt ggggatgctt ccatttggtg taatacattc 1320
   agacacacaa gtgtctatga gtgcatctga tctcattaca gagcttttcc ctgttctcaa 1380
   tttaaaatat ccccgcggtg caactaagtt gccattactg tttatcatta gaatatctcc 1440
   aggttttaca atggtccagt atatgcttat cctgcctgat tgacccctga cccacggcct 1500
   agatccgatg ttagggatta ctgtttgttg acttctttct gttgagactg ttactcgtcc 1560
   tgattcttgg atatataatt ttgtctgctc tttgtttgag ctcgggtgat gaatccccca 1620
   gatgtatagt ttatcgaaat ttttattgtt aggcattgtc acattcaata tggggtaaga 1680
   atttccagat tttgttagcc aattcagtcg gctaaagaaa ctatcggctg atcccctttt 1740
   gcaggctcca cttcttccgt tttgagtgac acctgtccat gtgaacccct ctgctgtgaa 1800
   ttctaatgtt cctgaggatg ctacaatgga ccggagcgat gcatagtcag ggatgtcata 1860
   tgggtagcaa ttgctgaaag cgctgcttct ttctatgaag aggtcccaat tctcatactg 1920
   aaaatcatca caatgggggt ctcctagcat tgcatctatt aatgtgcaat ttcttccatc 1980
   tagaacccta tatgagttgt tgcatatttt ccctattgaa atgctctgga ctaattcagt 2040
   agcatttgtc acctcaattt ggtcatcagt tattgttttt accaatgttc catttgctac 2100
   tgcatggtgt cccagacata atgtggctgt gttgttgcca ctggttgggt tttgactgta 2160
   gacccaatgg gtcagtagta tcaaaataat ggttgtcttc attacgatac aaacttaacg 2220
   gatatcgcga taatgaaata atttatgatt atttctcgct ttcaatttaa cacaaccctc 2280
   aagaaccttt gtatttattt tcacttttta agtatagaat aaagaagctc taattaatta 2340
   acgagcagat agtctcgttc tcgccctgcc tgatgactaa ttaattaacc cggatccttt 2400
   ttatagctaa ttagtcacgt acctttgaga gtaccacttc agctacctct tttgtgtctc 2460
   agagtaactt tctttaatca attccaaaac agtatatgat tttccatttc tttcaaagat 2520
   gtagtttaca tctgctcctt tgttgaaaag tagcctgagc acttcttttc taccatgaat 2580
   tacagctggc aagatcaatt tttcccagtt ctggacattt tatttttttt aagtagtgtg 2640
   ctacatattt caatatttcc agattgtaca gcgatcatta aaggagtacg tcccatgtta 2700
   tccagcaagt cagtatcagc acctttgttc aatagaagtt taaccattgt taaattttta 2760
   tttgatacgg ctatatgtag aggagttaac cgatccgtgt ttgaaatatc tacatccgcc 2820
   gaatgagcca atagaagttt aaccaaatta actttgttaa ggtaagctgc caaacacaaa 2880
   ggagtaaagc ctccgctgta aagaacattg tttacatagt tattcttcaa cagatctttc 2940
   actattttgt agtcgtctct caacaccgca tcatgcagac aagaagttgt gcattcagta 3000
   actacaggtt tagctccata cctcatcaag atttttatag cctcggtatt cttgaacatt 3060
   acagccattt caagaggaga ttgtagagta ccatattccg tgttagggtc gaatccattg 3120
   tccaaaaacc tatttagaga tgcattgtca ttatccatga tagcctcaca gacgtatatg 3180
   taagccatct tgaatgtata attttgttgt tttcaacaac cgctcgtgaa cagcttctat 3240
   actttttcat tttcttcatg attaatatag tttacggaat ataagtatac aaaaagttta 3300
   tagtaatctc ataatatctg aaacacatac ataaaacatg gaagaattac acgatgtcgt 3360
   tgagataaat ggctttttat tgtcatagtt tacaaattcg cagtaatctt catcttttac 3420
   gaatattgca gaatctgttt tatccaacca gtgatttttg tataatataa ctggtatcct 3480
   atcttccgat agaatgctgt tatttaacat ttttgcacct attaagttac atctgtcaaa 3540
   tccatctttc caactgactt tatgtaacga tgcgaaatag catttatcac tatgtcgtac 3600
   ccaattatca tgacaagatt ctcttaaata cgtaatctta ttatctcttg catattcgta 3660
   atagtaattg taaagagtat acgataacag tatagatata cacgtgatat aaatatttaa 3720
   ccccattcct gagtaaaata attacgatat tacatttcct tttattattt ttatgtttta 3780
   gttatttgtt aggttataca aaaattatgt ttatttgtgt atatttaaag cgtcgttaag 3840
   aataagctta gttaacatat tatcgcttag gttttgtagt atttgaatcc tttctttaaa 3900
   tggattattt ttccaatgca tatttatagc ttcatccaaa gtataacatt taacattca  3959
<210> 2
   <211> 3917
   <212> DNA
   <213> Influenza A virus
<400> 2
   gtattctaaa ctaggaatag atgaaattat gtgcaaagga gataccttta gatatggatc 60
   tgatttattt ggtttttcat aatcataatc taacaacatt ttcactatac tataccttct 120
   tgcacaagtc gccattagta gtatagactt atactttgta accatagtat actttagcgc 180
   gtcatcttct tcatctaaaa cagatttaca acaataatca tcgtcgtcat cttcatcttc 240
   attaaagttt tcatattcaa taactttctt ttctaaaaca tcatctgaat caataaacat 300
   agaacggtat agagcgttaa tctccattgt aaaatatact aacgcgttgc tcatgatgta 360
   cttttttttc attatttaga aattatgcat tttagatctt tataagcggc cgtgattaac 420
   tagtcataaa aacccgggat cgattctaga ctcgagggta cctcaaatgc aaatgttgca 480
   tctgatgttg cctttttggc aagcccacat aatgaaaccc aatagaacaa cgcaaattaa 540
   gaagcatgat atggcgaatg aaatccacag tatccaatct ttgtagcctg atttcaactc 600
   aacacctttg atttgaaatc ggttgtttaa tgcttcatct ctgtatatgt aatggtcata 660
   tgtcccattt cttattgatc caatgcatgc attatcacat ttgtggtaaa tcttgaaaca 720
   tccacctccc atgtcttccg cgttttctct taactggcgt ctagtcttct cgaataattt 780
   attcatttct gcatctgtta agtcaattgt atgttgattt tctagagcca ccagcaattc 840
   tgcattgtag gaccataggt ctattttggt gtcttctaca tacttctcca agtcctggat 900
   tctcccttct acttctgaga attccttctc tatttgatgg aatttctcat tggtcctttc 960
   aatcactctg tttaattttc cattaatctg gtcgatggct gcttgagtgc tctttagatc 1020
   tgcagcttgt cctgttcctt ccgagttttg atatcggaat ccataccacc catcaaccat 1080
   tccttcccag ccgttttcta tgaatcccgc tattgctcca aagattcctc tgatttgctt 1140
   ttctggtaca ttcctcatcc cagtggccag ctttaaagtg ttttgcctga tatacttggg 1200
   gcattttcca tatgtaactt tgttcacatt ttgaaatggt ttgtcgttgg ggatgcttcc 1260
   atttggtgta atacattcag acacacaaat gtctatgggt gcatctgatc tcattacaga 1320
   gcttttccct gttttcaatt taaaatatcc ccgcggtgca actaagttgc cattactgtt 1380
   tatcattagg atatctccag gttttacaat ggtccagtat atgcttatcc tgcctgattg 1440
   acccctgacc cacggtctag atccgatatt agggattatc gtttgttgac ttctttttgt 1500
   tgagactgtt actcgtcctg attcttggat gtacaattct gtttgctgtt ggtttgagct 1560
   cgggtgatga atcccccaga tgtatagttt gtcgaaattt ttattgttag gcattgtcac 1620
   attcaatgtg gggtaagagt ttccagattt tgttagccaa ttcagtcggc taaagaaact 1680
   atcggctgat ccccttttgc aggctccact tcttccgttt tgagtgacac ctgtccatgt 1740
   gaatccctct gctgtgaatt ccaatgttcc tgaggatgct acaatggacc ggagcgatgc 1800
   atagtcaggg atgtcatatg ggtagcaatt gctgaaagcg ctgcttcttt ctatgaagag 1860
   gtcccaattc tcatactgaa agacatcaca gtgggggtct cctagcattg catctattaa 1920
   tgtgcaattt cttccatcta gaactctata tgagttgttg catattttcc ctattgaaat 1980
   gctctgaact aattcagtag catttgtcac ctcaatttgg tcatcagtta ttgtttttac 2040
   caatgttcca tttgctactg catggtgtcc cagacataat gtggctgtgt tgttgccact 2100
   ggttgggttt tgactgtaga cccaatgggt cagtagtatc aaaataatgg ttgtcttcat 2160
   tacgatacaa acttaacgga tatcgcgata atgaaataat ttatgattat ttctcgcttt 2220
   caatttaaca caaccctcaa gaacctttgt atttattttc actttttaag tatagaataa 2280
   agaagctcta attaattaac gagcagatag tctcgttctc gccctgcctg atgactaatt 2340
   aattaacccg gatccttttt atagctaatt agtcacgtac ctttgagagt accacttcag 2400
   ctacctcttt tgtgtctcag agtaactttc tttaatcaat tccaaaacag tatatgattt 2460
   tccatttctt tcaaagatgt agtttacatc tgctcctttg ttgaaaagta gcctgagcac 2520
   ttcttttcta ccatgaatta cagctggcaa gatcaatttt tcccagttct ggacatttta 2580
   ttttttttaa gtagtgtgct acatatttca atatttccag attgtacagc gatcattaaa 2640
   ggagtacgtc ccatgttatc cagcaagtca gtatcagcac ctttgttcaa tagaagttta 2700
   accattgtta aatttttatt tgatacggct atatgtagag gagttaaccg atccgtgttt 2760
   gaaatatcta catccgccga atgagccaat agaagtttaa ccaaattaac tttgttaagg 2820
   taagctgcca aacacaaagg agtaaagcct ccgctgtaaa gaacattgtt tacatagtta 2880
   ttcttcaaca gatctttcac tattttgtag tcgtctctca acaccgcatc atgcagacaa 2940
   gaagttgtgc attcagtaac tacaggttta gctccatacc tcatcaagat ttttatagcc 3000
   tcggtattct tgaacattac agccatttca agaggagatt gtagagtacc atattccgtg 3060
   ttagggtcga atccattgtc caaaaaccta tttagagatg cattgtcatt atccatgata 3120
   gcctcacaga cgtatatgta agccatcttg aatgtataat tttgttgttt tcaacaaccg 3180
   ctcgtgaaca gcttctatac tttttcattt tcttcatgat taatatagtt tacggaatat 3240
   aagtatacaa aaagtttata gtaatctcat aatatctgaa acacatacat aaaacatgga 3300
   agaattacac gatgtcgttg agataaatgg ctttttattg tcatagttta caaattcgca 3360
   gtaatcttca tcttttacga atattgcaga atctgtttta tccaaccagt gatttttgta 3420
   taatataact ggtatcctat cttccgatag aatgctgtta tttaacattt ttgcacctat 3480
   taagttacat ctgtcaaatc catctttcca actgacttta tgtaacgatg cgaaatagca 3540
   tttatcacta tgtcgtaccc aattatcatg acaagattct cttaaatacg taatcttatt 3600
   atctcttgca tattcgtaat agtaattgta aagagtatac gataacagta tagatataca 3660
   cgtgatataa atatttaacc ccattcctga gtaaaataat tacgatatta catttccttt 3720
   tattattttt atgttttagt tatttgttag gttatacaaa aattatgttt atttgtgtat 3780
   atttaaagcg tcgttaagaa taagcttagt taacatatta tcgcttaggt tttgtagtat 3840
   ttgaatcctt tctttaaatg gattattttt ccaatgcata tttatagctt catccaaagt 3900
   ataacattta acattca                                                3917
<210> 3
   <211> 565
   <212> PRT
   <213> Influenza A virus
<400> 3
   Met Lys Thr Thr Ile Ile Leu Ile Leu Leu Thr His Trp Val Tyr Ser
   1               5                   10                  15
   Gln Asn Pro Thr Ser Gly Asn Asn Thr Ala Thr Leu Cys Leu Gly His
               20                  25                  30
   His Ala Val Ala Asn Gly Thr Leu Val Lys Thr Ile Thr Asp Asp Gln
           35                  40                  45
   Ile Glu Val Thr Asn Ala Thr Glu Leu Val Gln Ser Ile Ser Ile Gly
       50                  55                  60
   Lys Ile Cys Asn Asn Ser Tyr Arg Val Leu Asp Gly Arg Asn Cys Thr
   65                  70                  75                  80
   Leu Ile Asp Ala Met Leu Gly Asp Pro His Cys Asp Asp Phe Gln Tyr
                   85                  90                  95
   Glu Asn Trp Asp Leu Phe Ile Glu Arg Ser Ser Ala Phe Ser Asn Cys
               100                 105                 110
   Tyr Pro Tyr Asp Ile Pro Asp Tyr Ala Ser Leu Arg Ser Ile Val Ala
           115                 120                 125
   Ser Ser Gly Thr Leu Glu Phe Thr Ala Glu Gly Phe Thr Trp Thr Gly
       130                 135                 140
   Val Thr Gln Asn Gly Arg Ser Gly Ala Cys Lys Arg Gly Ser Ala Asp
   145                 150                 155                 160
   Ser Phe Phe Ser Arg Leu Asn Trp Leu Thr Lys Ser Gly Asn Ser Tyr
                   165                 170                 175
   Pro Ile Leu Asn Val Thr Met Pro Asn Asn Lys Asn Phe Asp Lys Leu
               180                 185                 190
   Tyr Ile Trp Gly Ile His His Pro Ser Ser Asn Lys Glu Gln Thr Lys
           195                 200                 205
   Leu Tyr Ile Gln Glu Ser Gly Arg Val Thr Val Ser Thr Glu Arg Ser
       210                 215                 220
   Gln Gln Thr Val Ile Pro Asn Ile Gly Ser Arg Pro Trp Val Arg Gly
   225                 230                 235                 240
   Gln Ser Gly Arg Ile Ser Ile Tyr Trp Thr Ile Val Lys Pro Gly Asp
                   245                 250                 255
   Ile Leu Met Ile Asn Ser Asn Gly Asn Leu Val Ala Pro Arg Gly Tyr
               260                 265                 270
   Phe Lys Leu Arg Thr Gly Lys Ser Ser Val Met Arg Ser Asp Ala Leu
           275                 280                 285
   Ile Asp Thr Cys Val Ser Glu Cys Ile Thr Pro Asn Gly Ser Ile Pro
       290                 295                 300
   Asn Asp Lys Pro Phe Gln Asn Val Asn Lys Ile Thr Tyr Gly Lys Cys
   305                 310                 315                 320
   Pro Lys Tyr Ile Arg Gln Asn Thr Leu Lys Leu Ala Thr Gly Met Arg
                   325                 330                 335
   Asn Val Pro Glu Lys Gln Ile Arg Gly Ile Phe Gly Ala Ile Ala Gly
               340                 345                 350
   Phe Ile Glu Asn Gly Trp Glu Gly Met Val Asp Gly Trp Tyr Gly Phe
           355                 360                 365
   Arg Tyr Gln Asn Ser Glu Gly Thr Gly Gln Ala Ala Asp Leu Lys Ser
       370                 375                 380
   Thr Gln Ala Ala Ile Asp Gln Ile Asn Gly Lys Leu Asn Arg Val Ile
   385                 390                 395                 400
   Glu Arg Thr Asn Glu Lys Phe His Gln Ile Glu Lys Glu Phe Ser Glu
                   405                 410                 415
   Val Glu Gly Arg Ile Gln Asp Leu Glu Lys Tyr Val Glu Asp Thr Lys
               420                 425                 430
   Ile Asp Leu Trp Ser Tyr Asn Ala Glu Leu Leu Val Ala Leu Glu Asn
           435                 440                 445
   Gln His Thr Ile Asp Leu Thr Asp Ala Glu Met Asn Lys Leu Phe Glu
       450                 455                 460
   Lys Thr Arg Arg Gln Leu Arg Glu Asn Ala Glu Asp Met Gly Gly Gly
   465                 470                 475                 480
   Cys Phe Lys Ile Tyr His Lys Cys Asp Asn Ala Cys Ile Gly Ser Ile
                   485                 490                 495
   Arg Asn Gly Thr Tyr Asp His Tyr Ile Tyr Arg Asp Glu Ala Leu Asn
               500                 505                 510
   Asn Arg Phe Gln Ile Lys Ser Val Glu Leu Lys Ser Gly Tyr Lys Asp
           515                 520                 525
   Trp Ile Leu Trp Ile Ser Phe Ala Ile Ser Cys Phe Leu Ile Cys Val
       530                 535                 540
   Val Leu Leu Gly Phe Ile Met Trp Ala Cys Gln Lys Gly Asn Ile Arg
   545                 550                 555                 560
   Cys Asn Ile Cys Ile
                   565
<210> 4
   <211> 565
   <212> PRT
   <213> Influenza A virus
<400> 4
   Met Lys Thr Thr Ile Ile Leu Ile Leu Leu Thr His Trp Ala Tyr Ser
   1               5                   10                  15
   Gln Asn Pro Ile Ser Gly Asn Asn Thr Ala Thr Leu Cys Leu Gly His
               20                  25                  30
   His Ala Val Ala Asn Gly Thr Leu Val Lys Thr Ile Ser Asp Asp Gln
           35                  40                  45
   Ile Glu Val Thr Asn Ala Thr Glu Leu Val Gln Ser Ile Ser Met Gly
       50                  55                  60
   Lys Ile Cys Asn Asn Ser Tyr Arg Ile Leu Asp Gly Arg Asn Cys Thr
   65                  70                  75                  80
   Leu Ile Asp Ala Met Leu Gly Asp Pro His Cys Asp Ala Phe Gln Tyr
                   85                  90                  95
   Glu Asn Trp Asp Leu Phe Ile Glu Arg Ser Ser Ala Phe Ser Asn Cys
               100                 105                 110
   Tyr Pro Tyr Asp Ile Pro Asp Tyr Ala Ser Leu Arg Ser Ile Val Ala
           115                 120                 125
   Ser Ser Gly Thr Leu Glu Phe Thr Ala Glu Gly Phe Thr Trp Thr Gly
       130                 135                 140
   Val Thr Gln Asn Gly Arg Ser Gly Ala Cys Lys Arg Gly Ser Ala Asp
   145                 150                 155                 160
   Ser Phe Phe Ser Arg Leu Asn Trp Leu Thr Lys Ser Gly Ser Ser Tyr
                   165                 170                 175
   Pro Thr Leu Asn Val Thr Met Pro Asn Asn Lys Asn Phe Asp Lys Leu
               180                 185                 190
   Tyr Ile Trp Gly Ile His His Pro Ser Ser Asn Gln Glu Gln Thr Lys
           195                 200                 205
   Leu Tyr Ile Gln Glu Ser Gly Arg Val Thr Val Ser Thr Lys Arg Ser
       210                 215                 220
   Gln Gln Thr Ile Ile Pro Asn Ile Gly Ser Arg Pro Trp Val Arg Gly
   225 230 235 240
   Gln Ser Gly Arg Ile Ser Ile Tyr Trp Thr Ile Val Lys Pro Gly Asp
   245 250 255
   Ile Leu Met Ile Asn Ser Asn Gly Asn Leu Val Ala Pro Arg Gly Tyr
   260 265 270
   Phe Lys Leu Lys Thr Gly Lys Ser Ser Val Met Arg Ser Asp Val Pro
   275 280 285
   Ile Asp Ile Cys Val Ser Glu Cys Ile Thr Pro Asn Gly Ser Ile Ser
   290 295 300
   Asn Asp Lys Pro Phe Gln Asn Val Asn Lys Val Thr Tyr Gly Lys Cys
   305 310 315 320
   Pro Lys Tyr Ile Arg Gln Asn Thr Leu Lys Leu Ala Thr Gly Met Arg
   325 330 335
   Asn Val Pro Glu Lys Gln Ile Arg Gly Ile Phe Gly Ala Ile Ala Gly
   340 345 350
   Phe Ile Glu Asn Gly Trp Glu Gly Met Val Asp Gly Trp Tyr Gly Phe
   355 360 365
   Arg Tyr Gln Asn Ser Glu Gly Thr Gly Gln Ala Ala Asp Leu Lys Ser
   370 375 380
   Thr Gln Ala Ala Ile Asp Gln Ile Asn Gly Lys Leu Asn Arg Val Ile
   385 390 395 400
   Glu Arg Thr Asn Glu Lys Phe His Gln Ile Glu Lys Glu Phe Ser Glu
   405 410 415
   Val Glu Gly Arg Ile Gln Asp Leu Glu Lys Tyr Val Glu Asp Thr Lys
   420 425 430
   Ile Asp Leu Trp Ser Tyr Asn Ala Glu Leu Leu Val Ala Leu Glu Asn
   435 440 445
   Gln His Thr Ile Asp Leu Thr Asp Ala Glu Met Asn Lys Leu Phe Glu
   450 455 460
   Lys Thr Arg Arg Gln Leu Lys Glu Asn Ala Glu Asp Met Gly Gly Gly
   465 470 475 480
   Cys Phe Lys Ile Tyr His Lys Cys Asp Asn Ala Cys Ile Gly Ser Ile
   485 490 495
   Arg Asn Gly Thr Tyr Asp His Tyr Ile Tyr Arg Asp Glu Ala Leu Asn
   500 505 510
   Asn Arg Phe Gln Ile Lys Gly Val Glu Leu Lys Ser Gly Tyr Lys Asp
   515 520 525
   Trp Ile Leu Trp Ile Ser Phe Ala Ile Ser Cys Phe Leu Ile Cys Val
   530 535 540
   Val Leu Leu Gly Phe Ile Met Trp Ala Cys Gln Lys Gly Asn Ile Arg
   545 550 555 560
   Cys Asn Ile Cys Ile
   565
<210> 5
   <211> 565
   <212> PRT
   <213> Influenza A virus
<400> 5
   Met Lys Thr Thr Ile Ile Leu Ile Leu Leu Thr His Trp Ala Tyr Ser
   1 5 10 15
   Gln Asn Pro Ile Ser Gly Asn Asn Thr Ala Thr Leu Cys Leu Gly His
   20 25 30
   His Ala Val Ala Asn Gly Thr Leu Val Lys Thr Ile Ser Asp Asp Gln
   35 40 45
   Ile Glu Val Thr Asn Ala Thr Glu Leu Val Gln Ser Ile Ser Met Gly
   50 55 60
   Lys Ile Cys Asn Asn Ser Tyr Arg Ile Leu Asp Gly Arg Asn Cys Thr
   65 70 75 80
   Leu Ile Asp Ala Met Leu Gly Asp Pro His Cys Asp Ala Phe Gln Tyr
   85 90 95
   Glu Asn Trp Asp Leu Phe Ile Glu Arg Ser Ser Ala Phe Ser Asn Cys
   100 105 110
   Tyr Pro Tyr Asp Ile Pro Asp Tyr Ala Ser Leu Arg Ser Ile Val Ala
   115 120 125
   Ser Ser Gly Thr Leu Glu Phe Thr Ala Glu Gly Phe Thr Trp Thr Gly
       130                 135                 140
   Val Thr Gln Asn Gly Arg Ser Gly Ala Cys Lys Arg Gly Ser Ala Asp
   145                 150                 155                 160
   Ser Phe Phe Ser Arg Leu Asn Trp Leu Thr Lys Ser Gly Ser Ser Tyr
                   165                 170                 175
   Pro Thr Leu Asn Val Thr Met Pro Asn Asn Lys Asn Phe Asp Lys Leu
               180                 185                 190
   Tyr Ile Trp Gly Ile His His Pro Ser Ser Asn Gln Glu Gln Thr Lys
           195                 200                 205
   Leu Tyr Ile Gln Glu Ser Gly Arg Val Thr Val Ser Thr Lys Arg Ser
       210                 215                 220
   Gln Gln Thr Ile Ile Pro Asn Ile Gly Ser Arg Pro Trp Val Arg Gly
   225                 230                 235                 240
   Gln Ser Gly Arg Ile Ser Ile Tyr Trp Thr Ile Val Lys Pro Gly Asp
                   245                 250                 255
   Ile Leu Met Ile Asn Ser Asn Gly Asn Leu Val Ala Pro Arg Gly Tyr
               260                 265                 270
   Phe Lys Leu Lys Thr Gly Lys Ser Ser Val Met Arg Ser Asp Val Pro
           275                 280                 285
   Ile Asp Ile Cys Val Ser Glu Cys Ile Thr Pro Asn Gly Ser Ile Ser
       290                 295                 300
   Asn Asp Lys Pro Phe Gln Asn Val Asn Lys Val Thr Tyr Gly Lys Cys
   305                 310                 315                 320
   Pro Lys Tyr Ile Arg Gln Asn Thr Leu Lys Leu Ala Thr Gly Met Arg
                   325                 330                 335
   Asn Val Pro Glu Lys Gln Ile Arg Gly Ile Phe Gly Ala Ile Ala Gly
               340                 345                 350
   Phe Ile Glu Asn Gly Trp Glu Gly Met Val Asp Gly Trp Tyr Gly Phe
           355                 360                 365
   Arg Tyr Gln Asn Ser Glu Gly Thr Gly Gln Ala Ala Asp Leu Lys Ser
       370                 375                 380
   Thr Gln Ala Ala Ile Asp Gln Ile Asn Gly Lys Leu Asn Arg Val Ile
   385                 390                 395                 400
   Glu Arg Thr Asn Glu Lys Phe His Gln Ile Glu Lys Glu Phe Ser Glu
                   405                 410                 415
   Val Glu Gly Arg Ile Gln Asp Leu Glu Lys Tyr Val Glu Asp Thr Lys
               420                 425                 430
   Ile Asp Leu Trp Ser Tyr Asn Ala Glu Leu Leu Val Ala Leu Glu Asn
           435                 440                 445
   Gln His Thr Ile Asp Leu Thr Asp Ala Glu Met Asn Lys Leu Phe Glu
       450                 455                 460
   Lys Thr Arg Arg Gln Leu Lys Glu Asn Ala Glu Asp Met Gly Gly Gly
   465                 470                 475                 480
   Cys Phe Lys Ile Tyr His Lys Cys Asp Asn Ala Cys Ile Gly Ser Ile
                   485                 490                 495
   Arg Asn Gly Thr Tyr Asp His Tyr Ile Tyr Arg Asp Glu Ala Leu Asn
               500                 505                 510
   Asn Arg Phe Gln Ile Lys Gly Val Glu Leu Lys Ser Gly Tyr Lys Asp
           515                 520                 525
   Trp Ile Leu Trp Ile Ser Phe Ala Ile Ser Cys Phe Leu Ile Cys Val
       530                 535                 540
   Val Leu Leu Gly Phe Ile Met Trp Ala Cys Gln Lys Gly Asn Ile Arg
   545                 550                 555                 560
   Cys Asn Ile Cys Ile
                   565
<210> 6
   <211> 3973
   <212> DNA
   <213> Influenza A virus
<220>
   <221> CDS
   <222> (1800) .. (3494)
<400> 6
   ggaaacagct atgaccatga ttacgaattg cggccgcaat tctgaatgtt aaatgttata 60
   ctttggatga agctataaat atgcattgga aaaataatcc atttaaagaa aggattcaaa 120
   tactacaaaa cctaagcgat aatatgttaa ctaagcttat tcttaacgac gctttaaata 180
   tacacaaata aacataattt ttgtataacc taacaaataa ctaaaacata aaaataataa 240
   aaggaaatgt aatatcgtaa ttattttact caggaatggg gttaaatatt tatatcacgt 300
   gtatatctat actgttatcg tatactcttt acaattacta ttacgaatat gcaagagata 360
   ataagattac gtatttaaga gaatcttgtc atgataattg ggtacgacat agtgataaat 420
   gctatttcgc atcgttacat aaagtcagtt ggaaagatgg atttgacaga tgtaacttaa 480
   taggtgcaaa aatgttaaat aacagcattc tatcggaaga taggatacca gttatattat 540
   acaaaaatca ctggttggat aaaacagatt ctgcaatatt cgtaaaagat gaagattact 600
   gcgaatttgt aaactatgac aataaaaagc catttatctc aacgacatcg tgtaattctt 660
   ccatgtttta tgtatgtgtt tcagatatta tgagattact ataaactttt tgtatactta 720
   tattccgtaa actatattaa tcatgaagaa aatgaaaaag tatagaagct gttcacgagc 780
   ggttgttgaa aacaacaaaa ttatacattc aagatggctt acatatacgt ctgtgaggct 840
   atcatggata atgacaatgc atctctaaat aggtttttgg acaatggatt cgaccctaac 900
   acggaatatg gtactctaca atctcctctt gaaatggctg taatgttcaa gaataccgag 960
   gctataaaaa tcttgatgag gtatggagct aaacctgtag ttactgaatg cacaacttct 1020
   tgtctgcatg atgcggtgtt gagagacgac tacaaaatag tgaaagatct gttgaagaat 1080
   aactatgtaa acaatgttct ttacagcgga ggctttactc ctttgtgttt ggcagcttac 1140
   cttaacaaag ttaatttggt taaacttcta ttggctcatt cggcggatgt agatatttca 1200
   aacacggatc ggttaactcc tctacatata gccgtatcaa ataaaaattt aacaatggtt 1260
   aaacttctat tgaacaaagg tgctgatact gacttgctgg ataacatggg acgtactcct 1320
   ttaatgatcg ctgtacaatc tggaaatatt gaaatatgta gcacactact taaaaaaaat 1380
   aaaatgtcca gaactgggaa aaattgatct tgccagctgt aattcatggt agaaaagaag 1440
   tgctcaggct acttttcaac aaaggagcag atgtaaacta catctttgaa agaaatggaa 1500
   aatcatatac tgttttggaa ttgattaaag aaagttactc tgagacacaa aagaggtagc 1560
   tgaagtggta ctctcaaagg tacgtgacta attagctata aaaaggatcc gggttaatta 1620
   attagtcatc aggcagggcg agaacgagac tatctgctcg ttaattaatt agagcttctt 1680
   tattctatac ttaaaaagtg aaaataaata caaaggttct tgagggttgt gttaaattga 1740
   aagcgagaaa taatcataaa ttatttcatt atcgcgatat ccgttaagtt tgtatcgta  1799
   atg aaa acc acc atc atc ctg atc ctg ctg acc cac tgg gcc tac agc   1847
   Met Lys Thr Thr Ile Ile Leu Ile Leu Leu Thr His Trp Ala Tyr Ser
   1               5                   10                  15
   cag aac cct atc agc ggc aac aac acc gcc acc ctg tgc ctg ggc cac   1895
   Gln Asn Pro Ile Ser Gly Asn Asn Thr Ala Thr Leu Cys Leu Gly His
               20                  25                  30
   cac gcc gtg gcc aac ggc acc ctg gtc aag acc atc agc gac gac cag     1943
   His Ala Val Ala Asn Gly Thr Leu Val Lys Thr Ile Ser Asp Asp Gln
           35                  40                  45
   atc gaa gtg acc aac gcc acc gag ctg gtg cag agc atc agc atg ggc     1991
   Ile Glu Val Thr Asn Ala Thr Glu Leu Val Gln Ser Ile Ser Met Gly
       50                  55                  60
   aag atc tgc aac aac agc tac cgc atc ctg gac ggc aga aac tgc acc     2039
   Lys Ile Cys Asn Asn Ser Tyr Arg Ile Leu Asp Gly Arg Asn Cys Thr
   65                  70                  75                  80
   ctg atc gac gcc atg ctg ggc gac ccc cac tgc gac gcc ttc cag tac     2087
   Leu Ile Asp Ala Met Leu Gly Asp Pro His Cys Asp Ala Phe Gln Tyr
                   85                  90                  95
   gag aac tgg gac ctg ttc atc gag agg agc agc gcc ttc agc aac tgc     2135
   Glu Asn Trp Asp Leu Phe Ile Glu Arg Ser Ser Ala Phe Ser Asn Cys
               100                 105                 110
   tac ccc tac gac atc cct gac tac gcc agc ctg aga agc atc gtg gcc     2183
   Tyr Pro Tyr Asp Ile Pro Asp Tyr Ala Ser Leu Arg Ser Ile Val Ala
           115                 120                 125
   agc agc ggc acc ctg gag ttc acc gcc gag ggc ttc acc tgg acc ggc     2231
   Ser Ser Gly Thr Leu Glu Phe Thr Ala Glu Gly Phe Thr Trp Thr Gly
       130                 135                 140
   gtg acc cag aac ggc aga agc ggc gcc tgc aag aga ggc agc gcc gac     2279
   Val Thr Gln Asn Gly Arg Ser Gly Ala Cys Lys Arg Gly Ser Ala Asp
   145                 150                 155                 160
   agc ttc ttc agc cgc ctg aac tgg ctg acc aag agc ggc agc agc tac     2327
   Ser Phe Phe Ser Arg Leu Asn Trp Leu Thr Lys Ser Gly Ser Ser Tyr
                   165                 170                 175
   ccc acc ctg aac gtg acc atg ccc aac aac aag aac ttc gac aag ctg     2375
   Pro Thr Leu Asn Val Thr Met Pro Asn Asn Lys Asn Phe Asp Lys Leu
               180                 185                 190
   tac atc tgg ggc atc cac cac ccc agc agc aac cag gag cag acc aag     2423
   Tyr Ile Trp Gly Ile His His Pro Ser Ser Asn Gln Glu Gln Thr Lys
           195                 200                 205
   ctg tac atc cag gag agc ggc aga gtg acc gtg tcc acc aag aga agc     2471
   Leu Tyr Ile Gln Glu Ser Gly Arg Val Thr Val Ser Thr Lys Arg Ser
       210                 215                 220
   cag cag acc atc atc ccc aac atc ggc agc aga cct tgg gtg cgc ggc     2519
   Gln Gln Thr Ile Ile Pro Asn Ile Gly Ser Arg Pro Trp Val Arg Gly
   225                 230                 235                 240
   cag tcc ggc agg atc agc atc tac tgg acc atc gtg aag cct ggc gac     2567
   Gln Ser Gly Arg Ile Ser Ile Tyr Trp Thr Ile Val Lys Pro Gly Asp
                   245                 250                 255
   atc ctg atg atc aac agc aac ggc aac ctg gtg gcc ccc aga ggc tac     2615
   Ile Leu Met Ile Asn Ser Asn Gly Asn Leu Val Ala Pro Arg Gly Tyr
               260                 265                 270
   ttc aag ctg aaa acc ggc aag agc agc gtg atg aga agc gac gtg ccc     2663
   Phe Lys Leu Lys Thr Gly Lys Ser Ser Val Met Arg Ser Asp Val Pro
           275                 280                 285
   atc gac atc tgc gtg tcc gag tgc atc acc cct aac ggc agc atc agc    2711
   Ile Asp Ile Cys Val Ser Glu Cys Ile Thr Pro Asn Gly Ser Ile Ser
       290                 295                 300
   aac gac aag ccc ttc cag aac gtg aac aaa gtg acc tac ggc aag tgc    2759
   Asn Asp Lys Pro Phe Gln Asn Val Asn Lys Val Thr Tyr Gly Lys Cys
   305                 310                 315                 320
   ccc aag tac atc cgc cag aac acc ctg aag ctg gcc acc ggc atg aga    2807
   Pro Lys Tyr Ile Arg Gln Asn Thr Leu Lys Leu Ala Thr Gly Met Arg
                   325                 330                 335
   aac gtg ccc gag aag cag atc aga ggc atc ttc ggc gcc atc gcc ggc    2855
   Asn Val Pro Glu Lys Gln Ile Arg Gly Ile Phe Gly Ala Ile Ala Gly
               340                 345                 350
   ttc atc gag aac ggc tgg gag ggc atg gtg gac ggc tgg tac ggc ttc    2903
   Phe Ile Glu Asn Gly Trp Glu Gly Met Val Asp Gly Trp Tyr Gly Phe
           355                 360                 365
   aga tac cag aac agc gag ggc acc ggc cag gcc gcc gac ctg aag agc    2951
   Arg Tyr Gln Asn Ser Glu Gly Thr Gly Gln Ala Ala Asp Leu Lys Ser
       370                 375                 380
   acc cag gcc gcc atc gac cag atc aac ggc aag ctg aac cgc gtg atc    2999
   Thr Gln Ala Ala Ile Asp Gln Ile Asn Gly Lys Leu Asn Arg Val Ile
   385                 390                 395                 400
   gag cgc acc aac gag aag ttc cac cag atc gag aag gag ttc agc gaa    3047
   Glu Arg Thr Asn Glu Lys Phe His Gln Ile Glu Lys Glu Phe Ser Glu
                   405                 410                 415
   gtg gag ggc aga atc cag gac ctg gag aag tac gtg gag gac acc aag    3095
   Val Glu Gly Arg Ile Gln Asp Leu Glu Lys Tyr Val Glu Asp Thr Lys
               420                 425                 430
   atc gac ctg tgg agc tac aac gcc gag ctg ctg gtc gcc ctg gag aac    3143
   Ile Asp Leu Trp Ser Tyr Asn Ala Glu Leu Leu Val Ala Leu Glu Asn
           435                 440                 445
   cag cac acc atc gac ctg acc gac gcc gag atg aac aag ctg ttc gaa    3191
   Gln His Thr Ile Asp Leu Thr Asp Ala Glu Met Asn Lys Leu Phe Glu
       450                 455                 460
   aag acc agg cgc cag ctg aag gaa aac gcc gag gac atg ggc ggc ggc    3239
   Lys Thr Arg Arg Gln Leu Lys Glu Asn Ala Glu Asp Met Gly Gly Gly
   465                 470                 475                 480
   tgc ttc aag atc tac cac aag tgc gac aac gcc tgc atc ggc tcc atc    3287
   Cys Phe Lys Ile Tyr His Lys Cys Asp Asn Ala Cys Ile Gly Ser Ile
                   485                 490                 495
   agg aac ggc acc tac gac cac tac atc tac agg gac gag gcc ctg aac    3335
   Arg Asn Gly Thr Tyr Asp His Tyr Ile Tyr Arg Asp Glu Ala Leu Asn
               500                 505                 510
   aac cgc ttc cag atc aag ggc gtg gag ctg aag agc ggc tac aag gac    3383
   Asn Arg Phe Gln Ile Lys Gly Val Glu Leu Lys Ser Gly Tyr Lys Asp
           515                 520                 525
   tgg atc ctg tgg atc agc ttc gcc atc agc tgc ttc ctg atc tgc gtg    3431
   Trp Ile Leu Trp Ile Ser Phe Ala Ile Ser Cys Phe Leu Ile Cys Val
       530                 535                 540
   gtg ctg ctg ggc ttc atc atg tgg gcc tgc cag aag ggc aac atc cgc   3479
   Val Leu Leu Gly Phe Ile Met Trp Ala Cys Gln Lys Gly Asn Ile Arg
   545                 550                 555                 560
   tgc aac atc tgc atc tgatgactcg agggttttta tgactagtta atcacggccg   3534
   Cys Asn Ile Cys Ile
                   565
   cttataaaga tctaaaatgc ataatttcta aataatgaaa aaaagtacat catgagcaac 3594
   gcgttagtat attttacaat ggagattaac gctctatacc gttctatgtt tattgattca 3654
   gatgatgttt tagaaaagaa agttattgaa tatgaaaact ttaatgaaga tgaagatgac 3714
   gacgatgatt attgttgtaa atctgtttta gatgaagaag atgacgcgct aaagtatact 3774
   atggttacaa agtataagtc tatactacta atggcgactt gtgcaagaag gtatagtata 3834
   gtgaaaatgt tgttagatta tgattatgaa aaaccaaata aatcagatcc atatctaaag 3894
   gtatctcctt tgcacataat ttcatctatt cctagtttag aatacctgca gccaagcttg 3954
   gcactggccg tcgttttac                                              3973
<210> 7
   <211> 3973
   <212> DNA
   <213> Influenza A virus
<400> 7
   gtaaaacgac ggccagtgcc aagcttggct gcaggtattc taaactagga atagatgaaa 60
   ttatgtgcaa aggagatacc tttagatatg gatctgattt atttggtttt tcataatcat 120
   aatctaacaa cattttcact atactatacc ttcttgcaca agtcgccatt agtagtatag 180
   acttatactt tgtaaccata gtatacttta gcgcgtcatc ttcttcatct aaaacagatt 240
   tacaacaata atcatcgtcg tcatcttcat cttcattaaa gttttcatat tcaataactt 300
   tcttttctaa aacatcatct gaatcaataa acatagaacg gtatagagcg ttaatctcca 360
   ttgtaaaata tactaacgcg ttgctcatga tgtacttttt ttcattattt agaaattatg 420
   cattttagat ctttataagc ggccgtgatt aactagtcat aaaaaccctc gagtcatcag 480
   atgcagatgt tgcagcggat gttgcccttc tggcaggccc acatgatgaa gcccagcagc 540
   accacgcaga tcaggaagca gctgatggcg aagctgatcc acaggatcca gtccttgtag 600
   ccgctcttca gctccacgcc cttgatctgg aagcggttgt tcagggcctc gtccctgtag 660
   atgtagtggt cgtaggtgcc gttcctgatg gagccgatgc aggcgttgtc gcacttgtgg 720
   tagatcttga agcagccgcc gcccatgtcc tcggcgtttt ccttcagctg gcgcctggtc 780
   ttttcgaaca gcttgttcat ctcggcgtcg gtcaggtcga tggtgtgctg gttctccagg 840
   gcgaccagca gctcggcgtt gtagctccac aggtcgatct tggtgtcctc cacgtacttc 900
   tccaggtcct ggattctgcc ctccacttcg ctgaactcct tctcgatctg gtggaacttc 960
   tcgttggtgc gctcgatcac gcggttcagc ttgccgttga tctggtcgat ggcggcctgg 1020
   gtgctcttca ggtcggcggc ctggccggtg ccctcgctgt tctggtatct gaagccgtac 1080
   cagccgtcca ccatgccctc ccagccgttc tcgatgaagc cggcgatggc gccgaagatg 1140
   cctctgatct gcttctcggg cacgtttctc atgccggtgg ccagcttcag ggtgttctgg 1200
   cggatgtact tggggcactt gccgtaggtc actttgttca cgttctggaa gggcttgtcg 1260
   ttgctgatgc tgccgttagg ggtgatgcac tcggacacgc agatgtcgat gggcacgtcg 1320
   cttctcatca cgctgctctt gccggttttc agcttgaagt agcctctggg ggccaccagg 1380
   ttgccgttgc tgttgatcat caggatgtcg ccaggcttca cgatggtcca gtagatgctg 1440
   atcctgccgg actggccgcg cacccaaggt ctgctgccga tgttggggat gatggtctgc 1500
   tggcttctct tggtggacac ggtcactctg ccgctctcct ggatgtacag cttggtctgc 1560
   tcctggttgc tgctggggtg gtggatgccc cagatgtaca gcttgtcgaa gttcttgttg 1620
   ttgggcatgg tcacgttcag ggtggggtag ctgctgccgc tcttggtcag ccagttcagg 1680
   cggctgaaga agctgtcggc gctgcctctc ttgcaggcgc cgcttctgcc gttctgggtc 1740
   acgccggtcc aggtgaagcc ctcggcggtg aactccaggg tgccgctgct ggccacgatg 1800
   cttctcaggc tggcgtagtc agggatgtcg taggggtagc agttgctgaa ggcgctgctc 1860
   ctctcgatga acaggtccca gttctcgtac tggaaggcgt cgcagtgggg gtcgcccagc 1920
   atggcgtcga tcagggtgca gtttctgccg tccaggatgc ggtagctgtt gttgcagatc 1980
   ttgcccatgc tgatgctctg caccagctcg gtggcgttgg tcacttcgat ctggtcgtcg 2040
   ctgatggtct tgaccagggt gccgttggcc acggcgtggt ggcccaggca cagggtggcg 2100
   gtgttgttgc cgctgatagg gttctggctg taggcccagt gggtcagcag gatcaggatg 2160
   atggtggttt tcattacgat acaaacttaa cggatatcgc gataatgaaa taatttatga 2220
   ttatttctcg ctttcaattt aacacaaccc tcaagaacct ttgtatttat tttcactttt 2280
   taagtataga ataaagaagc tctaattaat taacgagcag atagtctcgt tctcgccctg 2340
   cctgatgact aattaattaa cccggatcct ttttatagct aattagtcac gtacctttga 2400
   gagtaccact tcagctacct cttttgtgtc tcagagtaac tttctttaat caattccaaa 2460
   acagtatatg attttccatt tctttcaaag atgtagttta catctgctcc tttgttgaaa 2520
   agtagcctga gcacttcttt tctaccatga attacagctg gcaagatcaa tttttcccag 2580
   ttctggacat tttatttttt ttaagtagtg tgctacatat ttcaatattt ccagattgta 2640
   cagcgatcat taaaggagta cgtcccatgt tatccagcaa gtcagtatca gcacctttgt 2700
   tcaatagaag tttaaccatt gttaaatttt tatttgatac ggctatatgt agaggagtta 2760
   accgatccgt gtttgaaata tctacatccg ccgaatgagc caatagaagt ttaaccaaat 2820
   taactttgtt aaggtaagct gccaaacaca aaggagtaaa gcctccgctg taaagaacat 2880
   tgtttacata gttattcttc aacagatctt tcactatttt gtagtcgtct ctcaacaccg 2940
   catcatgcag acaagaagtt gtgcattcag taactacagg tttagctcca tacctcatca 3000
   agatttttat agcctcggta ttcttgaaca ttacagccat ttcaagagga gattgtagag 3060
   taccatattc cgtgttaggg tcgaatccat tgtccaaaaa cctatttaga gatgcattgt 3120
   cattatccat gatagcctca cagacgtata tgtaagccat cttgaatgta taattttgtt 3180
   gttttcaaca accgctcgtg aacagcttct atactttttc attttcttca tgattaatat 3240
   agtttacgga atataagtat acaaaaagtt tatagtaatc tcataatatc tgaaacacat 3300
   acataaaaca tggaagaatt acacgatgtc gttgagataa atggcttttt attgtcatag 3360
   tttacaaatt cgcagtaatc ttcatctttt acgaatattg cagaatctgt tttatccaac 3420
   cagtgatttt tgtataatat aactggtatc ctatcttccg atagaatgct gttatttaac 3480
   atttttgcac ctattaagtt acatctgtca aatccatctt tccaactgac tttatgtaac 3540
   gatgcgaaat agcatttatc actatgtcgt acccaattat catgacaaga ttctcttaaa 3600
   tacgtaatct tattatctct tgcatattcg taatagtaat tgtaaagagt atacgataac 3660
   agtatagata tacacgtgat ataaatattt aaccccattc ctgagtaaaa taattacgat 3720
   attacatttc cttttattat ttttatgttt tagttatttg ttaggttata caaaaattat 3780
   gtttatttgt gtatatttaa agcgtcgtta agaataagct tagttaacat attatcgctt 3840
   aggttttgta gtatttgaat cctttcttta aatggattat ttttccaatg catatttata 3900
   gcttcatcca aagtataaca tttaacattc agaattgcgg ccgcaattcg taatcatggt 3960
   catagctgtt tcc 3973
<210> 8
   <211> 890
   <212> DNA
   <213> Influenza A virus
<400> 8
   agcaaaagca gggtgacaaa aacataatgg attccaacac tgtgtcaagc tttcaggtag 60
   actgttttct ttggcatgtc cgcaaacgat tcgcagacca agaactgggt gatgccccat 120
   tccttgaccg gcttcgccga gaccagaagt ccctaagggg aagaggtatc actcttggtc 180
   tggacatcga aacagccact catgcaggaa agcagatagt ggagcagatt ctggaaaagg 240
   aatcagatga ggcacttaaa atgaccattg cctctgttcc tacttcacgc tacttaactg 300
   acatgactct tgatgagatg tcaagagact ggttcatgct catgcccaag caaaaagtaa 360
   caggctccct atgtataaga atggaccagg caatcatgga taagaacatc atacttaaag 420
   caaactttag tgtgattttc gaaaggctgg aaacactaat actacttaga gccttcaccg 480
   aagaaggagc agtcgttggc gaaatttcac cattaccttc tcttccagga catactaatg 540
   aggatgtcaa aaatgcaatt ggggtcctca tcggaggact taaatggaat gataatacgg 600
   ttagaatctc tgaaactcta cagagattcg cttggagaag cagtcatgag aatgggagac 660
   cttcattccc ttcaaagcag aaatgaaaaa tggagagaac aattaagcca gaaatttgaa 720
   gaaataagat ggttgattga agaagtgcga catagattga aaaatacaga aaatagtttt 780
   gaacaaataa catttatgca agccttacaa ctattgcttg aagtagaaca agagataaga 840
   actttctcgt ttcagcttat ttaatgataa aaaacaccct tgtttctact            890
<210> 9
   <211> 1027
   <212> DNA
   <213> Influenza A virus
<400> 9
   agcaaaagca ggtagatatt taaagatgag tcttctgacc gaggtcgaaa cgtacgttct 60
   ctctatcgta ccatcaggcc ccctcaaagc cgagatcgcg cagagacttg aagatgtctt 120
   tgcagggaag aacaccgatc ttgaggcact catggaatgg ctaaagacaa gaccaatcct 180
   gtcacctttg actaaaggga ttttaggatt tgtattcacg ctcaccgtgc ccagtgagcg 240
   aggactgcag cgtagacgct ttgtccaaaa tgcccttagt ggaaacggag atccaaacaa 300
   catggacaga gcagtaaaac tgtacaggaa gcttaaaaga gaaataacat tccatggggc 360
   aaaagaggtg gcactaagct attccactgg tgcactagcc agctgcatgg gactcatata 420
   caacagaatg ggaactgtga caaccgaagt ggcatttggc ctggtatgcg ccacatgtga 480
   acagattgct gattcccagc atcgatctca caggcagatg gtgacaacaa ccaacccatt 540
   aatcagacat gaaaacagaa tggtattagc cagtaccacg gctaaagcca tggaacagat 600
   ggcaggatca agtgagcagg cagcagaggc catggaggtt gctagtaagg ctaggcagat 660
   ggtacaggca atgagaacca ttgggaccca ccctagctcc agtgccggtt tgaaagatga 720
   tctccttgaa aatttacagg cctaccagaa acggatggga gtgcaaatgc agcgattcaa 780
   gtgatcctct cgttattgca gcaagtatca ttgggatctt gcacttgata ttgtggattc 840
   ttgatcgtct tttcttcaaa ttcatttatc gtcgccttaa atacgggttg aaaagagggc 900
   cttctacgga aggagtacct gagtctatga gggaagaata tcggcaggaa cagcagaatg 960
   ctgtggatgt tgacgatggt cattttgtca acatagagct ggagtaaaaa actaccttgt 1020
   ttctact 1027
<210> 10
   <211> 1460
   <212> DNA
   <213> Influenza A virus
<400> 10
   agcaaaagca ggagtttaaa atgaatccaa atcaaaagat aatagcaatt ggatttgcat 60
   cattggggat attaatcatt aacgtcattc tccatgtagt cagcattata gtaacagtac 120
   tggtcctcaa taacaatgga acaggtctga actgcaaagg gacgatcata agagagtaca 180
   atgaaacagt aagagtagaa aaaattactc aatggtataa taccagtgca attaagtaca 240
   tagagagacc tccaaatgaa tactacatga acaacaccga accactttgt gaggcccaag 300
   gctttgcacc attttccaaa gataatggaa tacgaattgg gtcgagaggc catgtttttg 360
   tgataagaga accttttgta tcatgttcgc cctcagaatg tagaaccttt ttcctcacac 420
   agggctcatt actcaatgac aaacattcta acggcacagt aaaggaccga agtccatata 480
   ggactttgat gagtgtcaaa atagggcaat cacctaatgt gtatcaagct aggtttgaat 540
   cggtggcatg gtcagcaaca gcatgccatg atggaaaaaa atggatgaca gttggagtca 600
   cagggcccga caatcaagca attgcagtag tgaactatgg aggtgttccg gttgatatta 660
   ttaattcatg ggcaggggat atcttaagaa cccaagaatc atcatgcacc tgcattaaag 720
   gagactgtta ttgggtaatg actgatggac cggcaaatag gcaagctaaa tataggatat 780
   tcaaagcaaa agatggaaga gtaattggac agactgatat aagtttcaat gggggacaca 840
   tagaggagtg ttcttgttac cccaatgaag ggaaggtgga atgcatatgc agggacaatt 900
   ggactggaac aaatagacca attctggtaa tatcttctga tctatcgtac acagttggat 960
   atttgtgtgc tggcattccc actgacactc ctaggggaga ggatagtcaa ttcacaggct 1020
   catgtacaag acctttggga aataaaggat acggtgtaaa aggtttcggg tttcgacaag 1080
   gaactgacgt atgggccgga aggacaatta gtaggacttc aagatcagga ttcgaaataa 1140
   taaaaatcag gaatggttgg acacagaaca gtaaagacca aatcaggagg caagtgatta 1200
   tcgatgaccc aaattggtca ggatatagcg gttctttcac attgccggtt gaactaacaa 1260
   aaaagggatg tttggtcccc tgtttctggg ttgaaatgat tagaggtaaa cctgaagaaa 1320
   caacaatatg gacctctagc agctccattg tgatgtgtgg agtagatcat aaaattgcca 1380
   gttggtcatg gcacgatgga gctattcttc cctttgacat cgataagatg taatttacga 1440
   aaaaactcct tgtttctact 1460
<210> 11
   <211> 1565
   <212> DNA
   <213> Influenza A virus
<400> 11
   agcaaaagca gggtagataa tcactcactg agtgacatca aagtcatggc gtctcaaggc 60
   accaaacgat cctatgaaca gatggaaact gatggggaac gccagaatgc aactgaaatc 120
   agagcatctg tcggaaggat ggtgggagga atcggccggt tttatgttca gatgtgtact 180
   gagcttaaac taaacgacca tgaagggcgg ctgattcaga acagcataac aatagaaagg 240
   atggtacttt cggcattcga cgaaagaaga aacaagtatc tcgaggagca tcccagtgct 300
   ggaaaagacc ctaagaaaac gggaggcccg atatacagaa ggaaagatgg gaaatggatg 360
   agggaactca tcctccatga taaagaagaa atcatgagaa tctggcgtca ggccaacaat 420
   ggtgaagacg ctactgctgg tcttactcat atgatgatct ggcactccaa tctcaatgac 480
   accacatacc aaagaacaag ggctcttgtt cggactggga tggatcccag aatgtgctct 540
   ctgatgcaag gctcaaccct cccacggaga tctggagccg ctggtgctgc agtaaaaggt 600
   gttggaacaa tggtaatgga actcatcaga atgatcaaac gcggaataaa tgatcggaat 660
   ttctggagag gtgaaaatgg tcgaaggacc agaattgctt atgaaagaat gtgcaatatc 720
   ctcaaaggga aatttcagac agcagcacaa cgggctatga tggaccaggt gagggaaggc 780
   cgcaatcctg gaaacgctga gattgaggat ctcattttct tagcacgatc agcacttatt 840
   ttgagaggat cagtagccca taaatcatgc ctacctgcct gtgtttatgg ccttgcagta 900
   accagtgggt atgactttga gaaggaagga tactctctgg ttggaattga tcctttcaaa 960
   ctactccaga acagtcaaat tttcagtcta atcagaccaa aagaaaaccc agcacacaag 1020
   agccagttgg tgtggatggc atgccattct gcagcatttg aggacctgag agttttgaat 1080
   ttcattagag gaaccaaagt aatcccaaga ggacagttaa caaccagagg agttcaaatt 1140
   gcttcaaatg aaaacatgga gacaatagat tctagcacac ttgaactgag aagcaaatat 1200
   tgggcaataa ggaccagaag tggaggaaac accagtcaac agagagcatc tgcaggacag 1260
   ataagtgtgc aacctacttt ctcagtacag agaaatcttc cctttgagag agcaaccatt 1320
   atggctgcat tcactggtaa cactgaaggg aggacttccg acatgagaac ggaaatcata 1380
   aggatgatgg aaagtgccaa atcagaagat gtgtctttcc aggggcgggg agtcttcgag 1440
   ctctcggacg aaaaggcaac gaacccgatc gtgccttcct ttgacatgag caatgaaggg 1500
   tcttatttct tcggagacaa tgctgaggaa tttgacagtt aaagaaaaat acccttgttt 1560
   ctact 1565
<210> 12
   <211> 1762
   <212> DNA
   <213> Influenza A virus
<400> 12
   agcaaaagca ggggatattt ctgtcaatca tgaagacaac cattattttg atactactga 60
   cccattgggc ctacagtcaa aacccaatca gtggcaacaa cacagccaca ttgtgtctgg 120
   gacaccatgc agtagcaaat ggaacattgg taaaaacaat aagtgatgat caaattgagg 180
   tgacaaatgc tacagaatta gttcagagca tttcaatggg gaaaatatgc aacaactcat 240
   atagaattct agatggaaga aattgcacat taatagatgc aatgctagga gacccccact 300
   gtgacgtctt tcagtatgag aattgggacc tctttataga aagaagcagc gctttcagca 360
   attgctaccc atatgacatc cctgactatg catcgctccg atccattgta gcatcctcag 420
   gaacattgga attcacagca gagggattca catggacagg tgtcactcaa aacggaagaa 480
   gtggagcctg caaaagggga tcagccgata gtttctttag ccgactgaat tggctaacaa 540
   aatctggaaa ctcttacccc acattgaatg tgacaatgcc taacaataaa aatttcgaca 600
   agctatacat ctgggggatt catcacccga gctcaaatca agagcagaca aaattgtaca 660
   tccaagaatc aggacgagta acagtctcaa caaaaagaag tcaacaaaca ataatcccta 720
   acatcggatc tagaccgtgg gtcagaggtc aatcaggcag gataagcata tactggacca 780
   ttgtaaaacc tggagatatc ctaatgataa acagtaatgg caacttagtt gcaccgcggg 840
   gatattttaa attgaaaaca gggaaaagct ctgtaatgag atcagatgca cccatagaca 900
   tttgtgtgtc tgaatgtatt acaccaaatg gaagcatctc caacgacaag ccattccaaa 960
   atgtgaacaa agttacatat ggaaaatgcc ccaagtatat caggcaaaac actttaaagt 1020
   tggccactgg gatgaggaat gtaccagaaa agcaaatcag aggaatcttt ggagcaatag 1080
   cgggattcat cgaaaacggc tgggaaggaa tggttgatgg gtggtatggg ttccgatatc 1140
   aaaactctga aggaacaggg caagctgcag atctaaagag cactcaagca gccatcgacc 1200
   agattaatgg aaagttaaac agagtgattg aaagaaccaa tgagaaattc catcaaatag 1260
   agaaggaatt ctcagaagta gaaggaagaa ttcaggactt ggagaaatat gtagaagaca 1320
   ccaaaataga cctatggtcc tacaatgcag aattgctggt ggctctagaa aatcaacata 1380
   caattgactt aacagatgca gaaatgaata aattattcga gaagactaga cgccagttaa 1440
   gagaaaacgc agaagacatg ggaggtggat gtttcaagat ttaccacaaa tgtgataatg 1500
   catgcattgg atcaataaga aatgggacat atgaccatta catatacaga gatgaagcat 1560
   taaacaaccg atttcaaatc aaaggtgttg agttgaaatc aggctacaaa gattggatac 1620
   tgtggatttc attcgccata tcatgcttct taatttgcgt tgttctattg ggtttcatta 1680
   tgtgggcttg ccaaaaaggc aacatcagat gcaacatttg catttgagta aactgatagt 1740
   taaaaacacc cttgtttcta ct 1762
<210> 13
   <211> 2233
   <212> DNA
   <213> Influenza A virus
<400> 13
   agcgaaagca ggtactgatc caaaatggaa gactttgtgc gacagtgctt caatccaatg 60
   atcgtcgagc ttgcggaaaa ggcaatgaaa gaatatggag aggacccgaa aatcgaaaca 120
   aacaaatttg cagcaatatg cactcacttg gaagtctgct tcatgtactc ggatttccac 180
   tttattaatg aactgggtga gtcagtggtc atagagtctg gtgacccaaa tgctcttttg 240
   aaacacagat ttgaaatcat tgaggggaga gatcgaacaa tggcatggac agtggtaaac 300
   agcatctgca acaccacaag agctgaaaaa cctaaatttc ttccagattt atacgactat 360
   aaggagaaca gatttgttga aattggtgtg acaaggagag aagttcacat atactacctg 420
   gagaaggcca acaaaataaa gtctgagaaa acacatatcc acattttctc atttacagga 480
   gaggaaatgg ctacaaaagc ggactatact cttgatgaag agagtagagc caggatcaag 540
   accagactat tcactataag acaagaaatg gccagtagag gcctctggga ttcctttcgt 600
   cagtccgaga gaggcgaaga gacaattgaa gaaagatttg aaatcacagg gacgatgcgc 660
   aagcttgcca attacagtct cccaccgaac ttctccagcc ttgaaaattt tagagtctat 720
   gtggatggat tcgaaccgaa cggctgcatt gagagtaagc tttctcaaat gtccaaagaa 780
   gtaaatgcca gaatcgaacc attttcaaag acaacacccc gaccactcaa aatgccaggt 840
   ggtccaccct gccatcagcg atctaaattc ttgctaatgg atgctctgaa actgagcatt 900
   gaggacccaa gtcacgaggg agagggaata ccactatatg atgcaatcaa atgcatgaaa 960
   actttctttg gatggaaaga gcccagtatt gttaaaccac atgaaaaggg tataaacccg 1020
   aactatctcc aaacttggaa gcaagtatta gaagaaatac aagaccttga gaacgaagaa 1080
   aggaccccca agaccaagaa tatgaaaaaa acaagccaat tgaaatgggc actaggtgaa 1140
   aatatggcac cagagaaagt ggattttgag gattgtaaag acatcagtga tttaaaacag 1200
   tatgacagcg atgagccaga aacaaggtct cttgcaagtt ggattcaaag tgagttcaac 1260
   aaagcttgtg agctgacaga ttcaagctgg atagagctcg atgaaattgg ggaggatgtc 1320
   gccccaatag aatacattgc gagcatgagg agaaattatt ttactgctga gatttcccat 1380
   tgtagagcaa cagaatatat aatgaaagga gtgtacatca acactgctct actcaatgca 1440
   tcctgtgctg cgatggatga atttcaatta attccgatga taagtaaatg caggaccaaa 1500
   gaagggagaa ggaaaacaaa tttatatgga ttcataataa agggaaggtc ccatttaaga 1560
   aatgatactg acgtggtgaa ctttgtaagt atggaatttt ctctcactga tccaagattt 1620
   gagccacaca aatgggaaaa atactgcgtt ctagaaattg gagacatgct tctaaggact 1680
   gctgtaggtc aagtgtcaag acccatgttt ttgtatgtaa ggacaaatgg aacctctaaa 1740
   attaaaatga aatggggaat ggaaatgagg cgctgcctcc ttcagtctct gcaacagatt 1800
   gaaagcatga tcgaagctga gtcctcagtc aaagaaaagg acatgaccaa agaatttttt 1860
   gagaacaaat cagagacatg gcctatagga gagtccccca aaggagtgga agagggctca 1920
   atcgggaagg tttgcaggac cttattagca aaatctgtgt ttaacagttt atatgcatct 1980
   ccacaactgg aagggttttc agctgaatct aggaaattac ttctcattgt tcaggctctt 2040
   agggataacc tggaacctgg aacctttgat attggggggt tatatgaatc aattgaggag 2100
   tgcctgatta atgatccctg ggttttgctt aatgcatctt ggttcaactc cttccttaca 2160
   catgcactga agtagttgtg gcaatgctac tatttgctat ccatactgtc caaaaaagta 2220
   ccttgtttct act 2233
<210> 14
   <211> 2341
   <212> DNA
   <213> Influenza A virus
<400> 14
   agcgaaagca ggcaaaccat ttgaatggat gtcaatccga ctctactttt cttaaaggtg 60
   ccagcgcaaa atgctataag cacaacattc ccttatactg gagatcctcc ctacagtcat 120
   ggaacaggga caggatacac catggatact gtcaacagaa cacaccaata ttcagaaaag 180
   gggaaatgga caacaaacac tgagattgga gcaccacaac ttaatccaat cgatggacca 240
   cttcctgaag acaatgaacc aagtgggtac gcccaaacag attgtgtatt ggaagcaatg 300
   gctttccttg aagaatccca tcccggaatc tttgaaaatt cgtgtcttga aacgatggag 360
   gtgattcagc agacaagagt ggacaaacta acacaaggcc gacaaactta tgattggacc 420
   ttgaatagga atcaacctgc cgcaacagca cttgctaata caattgaagt gttcagatca 480
   aatggtctga cttccaatga atcagggagg ttgatggact tcctcaaaga tgtcatggag 540
   tccatgaaca aggaagaaat ggaaataaca acacacttcc aacgaaagag aagagtaaga 600
   gacaacatga caaagagaat ggtaacacag agaaccatag ggaagaaaaa acaacgatta 660
   aacagaaaga gttatctaat cagaacatta accctaaaca caatgaccaa ggacgctgag 720
   agagggaaat tgaaacgacg agcaatcgca accccaggga tgcagataag aggatttgta 780
   tattttgttg aaacactagc ccgaagaata tgtgaaaagc ttgaacaatc aggattgcca 840
   gttggcggta atgagaaaaa ggccaaactg gctaatgtcg tcagaaaaat gatgactaat 900
   tcccaagaca ctgaactctc cttcaccatc actggggaca ataccaaatg gaatgaaaat 960
   cagaacccac gcatattcct ggcaatgatc acatacataa ctagaaacca gccagaatgg 1020
   ttcagaaatg ttctaagcat tgcaccgatt atgttctcaa ataaaatggc aagactgggg 1080
   aaaggatata tgtttgaaag caaaagtatg aaactgagag ctcaaatacc agcagaaatg 1140
   ctagcaagca ttgacctgaa atatttcaat gattcaacaa aaaagaaaat taaaaagata 1200
   cgaccacttc tggttgacgg gactgcttca ctgagtcctg gcatgatgat gggaatgttc 1260
   aacatgttga gcactgtgct gggtgtatcc atattaaacc tgggccagag gaaatacaca 1320
   aagaccacat actggtggga tggtctgcaa tcatccgatg actttgcttt gatagtgaat 1380
   gcgcctaatc atgaaggaat acaagctgga gtagacagat tctatagaac ttgcaaactg 1440
   gtcgggatca acatgagcaa aaagaagtcc tacataaata gaaccggaac attcgaattc 1500
   acaagctttt tctaccggta tggttttgta gccaatttca gcatggaact acccagtttt 1560
   ggggtttccg gaataaatga atctgcagac atgagcattg gagtgacagt catcaaaaac 1620
   aacatgataa ataatgatct cggtcctgcc acggcacaaa tggcactcca actcttcatt 1680
   aaggattacc ggtacacata ccggtgccat agaggtgata cccagataca aaccagaaga 1740
   tcttttgagt tgaagaaact gtgggaacag actcgatcaa agactggtct actggtatca 1800
   gatgggggtc caaacctata taacatcaga aacctacaca tcccggaagt ttgtttaaaa 1860
   tgggagctaa tggatgaaga ttataagggg aggctatgca atccattaaa tcctttcgtt 1920
   agtcacaaag aaattgaatc agtcaacagt gcagtagtaa tgcctgcgca tggccctgcc 1980
   aaaagcatgg agtatgatgc tgttgcaaca acacattctt ggatccccaa gaggaaccgg 2040
   tccatattga acacaagcca aaggggaata ctcgaagatg agcagatgta tcagaaatgc 2100
   tgcaacctgt ttgaaaaatt cttccccagc agctcataca gaagaccagt cggaatttct 2160
   agtatggttg aggccatggt gtccagggcc cgcattgatg cacgaattga cttcgaatct 2220
   ggacggataa agaaggatga gttcgctgag atcatgaaga tctgttccac cattgaagag 2280
   ctcagacggc aaaaatagtg aatttagctt gatcttcatg aaaaaatgcc ttgtttctac 2340
   t 2341
<210> 15
   <211> 2341
   <212> DNA
   <213> Influenza A virus
<400> 15
   agcgaaagca ggtcaaatat attcaatatg gagagaataa aagaactgag agatctgatg 60
   ttacaatccc gcacccgcga gatactaaca aaaactactg tggaccacat ggccataatc 120
   aagaaataca catcaggaag acaagagaag aaccctgcac ttaggatgaa atggatgatg 180
   gcaatgaaat acccaattac agcagataag aggataatgg agatgattcc tgagagaaat 240
   gaacagggac aaaccctttg gagcaaaacg aacgatgctg gctcagaccg cgtaatggta 300
   tcacctctgg cagtgacatg gtggaatagg aatggaccaa caacgagcac aattcattat 360
   ccaaaagtct acaaaactta ttttgaaaag gttgaaagat tgaaacacgg aacctttggc 420
   cccgttcatt ttaggaatca agtcaagata agacgaagag ttgatgtaaa ccctggtcac 480
   gcggacctca gtgccaaaga agcacaagat gtgatcatgg aagttgtttt cccaaatgaa 540
   gtgggagcca gaattctaac atcggaatca caactaacaa taaccaaaga gaaaaaggaa 600
   gaacttcagg actgcaaaat tgctcccttg atggtagcat acatgctaga aagagagttg 660
   gtccgaaaaa caaggttcct cccagtggca ggcggaacaa gcagtgtata cattgaagtg 720
   ttgcatctga ctcagggaac atgctgggag caaatgtaca ccccaggagg agaagttaga 780
   aacgatgata ttgatcaaag tttaattatt gcagcccgga acatagtgag aagagcgaca 840
   gtatcagcag atccactagc atccctactg gaaatgtgcc acagtacaca gattggtgga 900
   ataaggatgg tagacatcct taagcagaat ccaacagagg aacaagctgt ggatatatgc 960
   aaagcagcaa tgggattgag aattagctca tcattcagct ttggtggatt caccttcaaa 1020
   agaacaagtg gatcatcagt caagagagaa gaagaaatgc ttacgggcaa ccttcaaaca 1080
   ttgaaaataa gagtgcatga gggctatgaa gaattcacaa tggtcggaag aagagcaaca 1140
   gccattctca gaaaggcaac cagaagattg attcaattga tagtaagtgg gagagatgaa 1200
   caatcaattg ctgaagcaat aattgtagcc atggtgtttt cgcaagaaga ttgcatgata 1260
   aaagcagttc gaggcgattt gaactttgtt aatagagcaa atcagcgttt gaaccccatg 1320
   catcaactct tgaggcattt ccaaaaagat gcaaaagtgc ttttccaaaa ttggggaatt 1380
   gaacccatcg acaatgtaat gggaatgatt ggaatattgc ctgacatgac cccaagcacc 1440
   gagatgtcat tgagaggagt gagagtcagc aaaatgggag tggatgagta ctccagcact 1500
   gagagagtgg tggtgagcat tgaccgtttt ttaagagttc gggatcaaag gggaaacata 1560
   ctactgtccc ctgaagaagt cagtgaaaca caaggaacgg aaaagctgac aataatttat 1620
   tcgtcatcaa tgatgtggga gattaatggt cccgaatcag tgttggtcaa tacttatcaa 1680
   tggatcatca ggaactggga aattgtaaaa attcagtggt cacaggaccc cacaatgtta 1740
   tacaataaga tagaatttga gccattccag tccctggtcc ctagggccac cagaagccaa 1800
   tacagcggtt tcgtaagaac cctgtttcag caaatgcgag atgtacttgg aacatttgat 1860
   actgctcaaa taataaaact cctccctttt gccgctgctc ctccggaaca gagtaggatg 1920
   cagttctctt ctttgactgt taatgtaaga ggatcgggaa tgaggatact tgtaagaggc 1980
   aattccccag tgttcaacta caataaagcc actaaaaggc tcacagtcct cggaaaggat 2040
   gcaggtgcgc ttactgagga cccagatgaa ggtacggctg gagtagaatc tgctgttcta 2100
   agagggtttc tcattttagg taaagaaaac aagagatatg gcccagcact aagcatcaat 2160
   gaactaagca aacttgcaaa aggggagaaa gccaatgtac taattgggca aggggacgta 2220
   gtgttggtaa tgaaacggaa acgtgactct agcatactta ctgacagcca gacagcgacc 2280
   aaaaggattc ggatggccat caattagtgt tgaattgttt aaaaacgacc ttgtttctac 2340
   t 2341
<210> 16
   <211> 1762
   <212> DNA
   <213> Influenza A virus
<400> 16
   agcaaaagca ggggatattt ctgtcaatca tgaagacaac cattattttg atactactga 60
   cccattgggt ctacagtcaa aacccaacca gtggcaacaa cacagccaca ttatgtctgg 120
   gacaccatgc agtagcaaat ggaacattgg taaaaacaat aactgatgac caaattgagg 180
   tgacaaatgc tactgaatta gttcagagca tttcaatagg gaaaatatgc aacaactcat 240
   atagagttct agatggaaga aattgcacat taatagatgc aatgctagga gacccccact 300
   gtgatgtctt tcagtatgag aattgggacc tcttcataga aagaagcagc gctttcagca 360
   gttgctaccc atatgacatc cctgactatg catcgctccg gtccattgta gcatcctcag 420
   gaacattgga attcacagca gagggattca catggacagg tgtcactcaa aacggaagaa 480
   gtggatcctg caaaagggga tcagccgata gtttctttag ccgactgaat tggctaacaa 540
   aatctggaaa ctcttacccc acattgaatg tgacaatgcc taacaataaa aatttcgaca 600
   aactatacat ctgggggatt catcacccga gctcaaacga agagcagaca aaattgtaca 660
   tccaagaatc aggacgagta acagtctcaa caaaaagaag tcaacaaaca ataatcccta 720
   acatcggatc tagaccgtgg gtcaggggtc aatcaggcag gataagcata tactggacca 780
   ttgtaaaacc tggagatatc ctaatgataa acagtaatgg caacttagtt gcaccgcggg 840
   gatattttaa attgaaaaca gggaaaagct ctgtaatgag atcagatgca cccatagaca 900
   tttgtgtgtc tgaatgtatt acaccaaatg gaagcatccc caacgacaaa ccatttcaaa 960
   atgtgaacaa agttacatat ggaaaatgcc ccaagtatat caggcaaaac actttaaagc 1020
   tggccactgg gatgaggaat gtaccagaaa agcaaatcag aggaatcttt ggagcaatag 1080
   cgggattcat agaaaacggc tgggaaggaa tggttgatgg gtggtatgga ttccgatatc 1140
   aaaactcgga aggaacagga caagctgcag atctaaagag cactcaagca gccatcgacc 1200
   agatcaatgg aaaattaaac agagtgattg aaaggaccaa tgagaaattc catcaaatag 1260
   agaaggaatt ctcagaagta gaagggagaa tccaggattt ggagaagtat gtagaagaca 1320
   ccaaaataga cctatggtcc tacaatgcag aattgctggt ggctctagaa aatcaacata 1380
   caattgactt aacagatgca gaaatgaata aattattcga gaagactaga cgccagttaa 1440
   gagaaaacgc ggaagacatg ggaggtggat gtttcaagat ataccacaaa tgtgataatg 1500
   catgcattgg atcaataaga aatgggacat atgaccatta catatacaga gatgaagcat 1560
   taaacaaccg gtttcaaatc aaaggtgttg agttgaaatc aggctacaaa gattggatac 1620
   tgtggatttc attcgccata tcatgcttct taatttgcgt tgttctattg ggtttcatta 1680
   tgtgggcttg ccaaaaaggc aacatcagat gcaacatttg catttgagta aactgatagt 1740
   taaaaacacc cttgtttcta ct 1762
<210> 17
   <211> 1762
   <212> DNA
   <213> Influenza A virus
<400> 17
   agcaaaagca ggggatattt ctgtcaatca tgaagacaac cattattttg atactactga 60
   cccattgggt ctacagtcaa aacccaacca gtggcaacaa cacagccaca ttatgtctgg 120
   gacaccatgc agtagcaaat ggaacattgg taaaaacaat aactgatgac caaattgagg 180
   tgacaaatgc tactgaatta gttcagagca tttcaatagg gaaaatatgc aacaactcat 240
   atagggttct agatggaaga aattgcacat taatagatgc aatgctagga gacccccact 300
   gtgatgtctt tcagtatgag aattgggacc tcttcataga aagaagcagc gctttcagca 360
   attgctaccc atatgacatc cctgactatg catcgctccg gtccattgta gcatcctcag 420
   gaacattaga attcacagca gagggattca catggacagg tgtcactcaa aacggaagaa 480
   gtggagcctg caaaagggga tcagccgata gtttctttag ccgactgaat tggctaacaa 540
   aatctggaaa ctcttacccc acattgaatg tgacaatgcc taacaataaa aatttcgaca 600
   aactatacat ctgggggatt catcacccga gctcaaacaa tgagcagaca aaattgtata 660
   tccaagaaac aggacgagta acagtctcaa caaaaagaag tcaacaaaca ataatcccta 720
   acatcggatc tagaccgtgg gtcaggggtc aatcaggcag gataagcata tactggacca 780
   ttgtaaaacc tggagatatc ctaatgataa acagcaatgg caacttagtt gcaccgcggg 840
   gatattttaa attgagaaca gggagaagct ctgtaatgag atcagatgca cccatagaca 900
   tttgtgtgtc tgaatgtatt acaccaaatg gaagcatccc caacgacaaa ccatttcaaa 960
   atgtgaacaa agttacatat ggaaaatgcc ccaaatatat caggcaaaac actttaaagc 1020
   tggccactgg gatgaggaat gtaccagaaa agcaaatcag aggaatcttt ggagcaatag 1080
   cgggattcat agaaaacggc tgggaaggaa tggttgatgg gtggtatgga ttccgatatc 1140
   aaaactcgga aggaacagga caagctgcag atctaaagag cactcaagca gccatcgacc 1200
   agatcaatgg aaaattaaac agagtgattg aaaggaccaa tgagaaattc catcaaatag 1260
   agaaggaatt ctcagaagta gaagggagaa tccaggattt ggagaagtat gtagaagaca 1320
   ccaaaataga cctatggtcc tacaatgcag aattgctagt ggctctagaa aatcaacata 1380
   caattgactt aacagacgca gaaatgaata aattattcga gaagactaga cgccagttaa 1440
   gagaaaacgc ggaagacatg ggaggtggat gtttcaagat ttaccacaaa tgtgataatg 1500
   catgcattgg atcaataaga aatgggacat atgaccatta catatacaga gatgaagcat 1560
   taaacaaccg atttcaaatc aaaggtgttg aattaaaatc aggctacaaa gattggatac 1620
   tgtggatttc attcgccata tcatgcttct taatttgtgt tgttctattg ggtttcatta 1680
   tgtgggcttg ccaaaaaggc aacatcagat gcaacatttg catttgagta aactgatagt 1740
   taaaaacacc cttgtttcta ct 1762
<210> 18
   <211> 1762
   <212> DNA
   <213> Influenza A virus
<400> 18
   agcaaaagca ggggatattt ctgtcaatca tgaaaacaac cattattttg atactactga 60
   cccattgggt ctacagtcaa aacccaacca gtggcaacaa cacagccaca ttatgtctgg 120
   gacaccatgc agtagcaaat ggaacattgg taaaaacaat aactgatgac caaattgagg 180
   tgacaaatgc tactgaatta gttcagagca tttcaatagg aaaaatatgc aacaactcat 240
   atagggttct agatggaaga aattgcacat taatagatgc aatgctagga gaccctcact 300
   gtgatgtctt tcagtatgag aattgggacc tcttcataga aagaagcagc gctttcagca 360
   attgctaccc atatgacatc cctgactatg catcgctccg gtccattgta gcatcctcag 420
   gaacattaga attcacagca gagggattca catggacagg tgtcactcaa aacggaggaa 480
   gtggagcctg caaaagagga tcagccgata gtttctttag ccgactgaat tggctaacaa 540
   aatctggaaa ctcttacccc acattgaatg tgacaatgcc taacaataaa aatttcgaca 600
   aactatacat ctgggggatt catcacccga gctcaaacaa tgagcagaca aaattgtata 660
   tccaagaatc aggacgagta acagtctcaa caaaaagaag tcaacaaaca ataatcccta 720
   acatcggatc tagaccgtgg gtcaggggtc aatcaggcag gataagcata tactggacca 780
   ttgtaaaacc tggagatatc ctaatgataa acagtaatgg caacttagtt gcaccgcggg 840
   gatattttaa attgagaaca gggaaaagct ctgtaatgag atcagatgca cccatagaca 900
   cttgtgtgtc tgaatgtatt acaccaaatg gaagcatccc caacgacaaa ccatttcaaa 960
   atgtgaacaa agttacatat ggaaaatgcc ccaagtatat caggcaaaac actttaaagc 1020
   tggccactgg gatgaggaat gtaccagaaa agcaaatcag aggaatcttt ggagcaatag 1080
   cgggattcat agaaaacggc tgggaaggaa tggttgatgg gtggtatgga ttccgatatc 1140
   aaaactcgga aggaacagga caagctgcag atctaaagag cactcaagca gccatcgacc 1200
   agatcaatgg aaaattaaac agagtgattg aaaggaccaa tgagaaattc catcaaatag 1260
   agaaggaatt ctcagaagta gaagggagaa tcaaggactt ggagaagtat gtagaagaca 1320
   ccaaaataga cctatggtcc tacaatgcag aattgctggt ggctctagaa aatcaacata 1380
   caattgactt aacagatgca gaaatgaata aattattcga gaagactaga cgccagttaa 1440
   gagaaaacgc ggaagacatg ggaggtggat gtttcaagat ctaccacaaa tgtgataatg 1500
   catgcattgg atcaataaga aatgggacat atgaccatta catatacaaa gatgaagcat 1560
   taaacaaccg atttcaaatc aaaggtgttg agttgaaatc aggctacaaa gattggatac 1620
   tgtggatttc attcgccata tcatgcttct taatttgcgt tgttctattg ggtttcatta 1680
   tgtgggcttg ccaaaaaggc aacatcagat gcaacatttg catttgagta aactgatagt 1740
   taaaaacacc cttgtttcta ct                                          1762
<210> 19
   <211> 1762
   <212> DNA
   <213> Influenza A virus
<400> 19
   agcaaaagca ggggatattt ctgtcaatca tgaagacaac cattattttg atactactga 60
   cccattgggt ctacagtcaa aacccaacca gtggcaacaa cacagccaca ttatgtctgg 120
   gacaccatgc agtagcaaat ggaacattgg taaaaacaat aactgatgac caaattgagg 180
   tgacaaatgc tactgaatta gttcagagca tttcaatagg gaaaatatgc aacaactcat 240
   atagagttct agatggaaga aattgcacat taatagatgc aatgctagga gacccccact 300
   gtgatgtctt tcagtatgag aattgggacc tcttcataga aagaagcagc gctttcagca 360
   attgctaccc atatgacatc cctgactatg catcgctccg gtccattgta gcatcctcag 420
   gaacattgga attcacagca gagggattca catggacagg tgtcactcaa aacggaagaa 480
   gtggagcctg caaaagggga tcagccgata gtttctttag ccgactgaat tggctaacaa 540
   aatctggaaa ctcttacccc acattgaatg tgacaatgcc taacaataaa aatttcgaca 600
   aactatacat ctgggggatt catcacccga gctcaaacca acagcaaaca gaattgtaca 660
   tccaagaatc aggacgagta acagtctcaa caaaaagaag tcaacaaacg ataatcccta 720
   atatcggatc tagaccgtgg gtcaggggtc aatcaggcag gataagcata tactggacca 780
   ttgtaaaacc tggagatatc ctaatgataa acagtaatgg caacttagtt gcaccgcggg 840
   gatattttaa attgaaaaca gggaaaagct ctgtaatgag atcagatgca cccatagaca 900
   tttgtgtgtc tgaatgtatt acaccaaatg gaagcatccc caacgacaaa ccatttcaaa 960
   atgtgaacaa agttacatat ggaaaatgcc ccaagtatat caggcaaaac actttaaagc 1020
   tggccactgg gatgaggaat gtaccagaaa agcaaatcag aggaatcttt ggagcaatag 1080
   cgggattcat agaaaacggc tgggaaggaa tggttgatgg gtggtatgga ttccgatatc 1140
   aaaactcgga aggaacagga caagctgcag atctaaagag cactcaagca gccatcgacc 1200
   agattaatgg aaaattaaac agagtgattg aaaggaccaa tgagaaattc catcaaatag 1260
   agaaggaatt ctcagaagta gaagggagaa tccaggactt ggagaagtat gtagaagaca 1320
   ccaaaataga cctatggtcc tacaatgcag aattgctggt ggctctagaa aatcaacata 1380
   caattgactt aacagatgca gaaatgaata aattattcga gaagactaga cgccagttaa 1440
   gagaaaacgc ggaagacatg ggaggtggat gtttcaagat ttaccacaaa tgtgataatg 1500
   catgcattgg atcaataaga aatgggacat atgaccatta catatacaga gatgaagcat 1560
   taaacaaccg atttcaaatc aaaggtgttg agttgaaatc aggctacaaa gattggatac 1620
   tgtggatttc attcgccata tcatgcttct taatttgcgt tgttctattg ggtttcatta 1680
   tgtgggcttg ccaaaaaggc aacatcagat gcaacatttg catttgagta aactgatagt 1740
   taaaaacacc cttgtttcta ct                                          1762
<210> 20
   <211> 1565
   <212> DNA
   <213> Influenza A virus
<400> 20
   agcaaaagca gggtagataa tcactcactg agtgacatca aagtcatggc gtctcaaggc 60
   accaaacgat cttatgagca gatggaaact ggtggggaac gccagaatgc aactgaaatc 120
   agagcatctg ttggaaggat ggtgggagga atcggccggt tctatgttca aatgtgtact 180
   gagcttaaac tcaacgacca tgaagggcgg ctgattcaga acagcataac aatagaaagg 240
   atggtacttt cggcattcga cgaaagaaga aacaagtacc tcgaggagca tcccagtgct 300
   gggaaagacc ccaagaaaac gggaggcccg atatacagaa ggagagatgg gaaatggatg 360
   agagaactca tcctccatga taaagaagaa atcatgagga tctggcgtca ggccaacaat 420
   ggtgaagacg ctactgctgg tcttactcat atgatgatct ggcactccaa tctcaatgac 480
   accacctacc aaagaacaag ggctcttgtt cgggctggga tggatcccag aatgtgctct 540
   ctgatgcaag gatcaactct cccacggaga tctggagctg ccggtgctgc agtgaagggt 600
   gttggaacaa tggtaatgga actcatcagg atgatcaaac gcgggataaa tgatcgaaac 660
   ttctggagag gtgaaaatgg tcgaagaacc agaattgctt atgaaagaat gtgcaacatc 720
   ctcaagggga aattccaaac agcagcacaa cgagcaatga tggaccaagt gagggagggc 780
   cgcaatcctg gaaatgctga gattgaggat ctcattttct tggcacgatc agcactcatt 840
   ctgagaggat cagtagccca taaatcatgc ctacctgcct gtgtttatgg ccttgcagta 900
   gccagtgggt atgactttga gaaagaggga tactctctgg ttggaattga tcctttcaaa 960
   ctactccaga acagccaaat tttcagtcta atcagaccga aagaaaatcc agcacacaag 1020
   agccagctgg tgtggatggc atgccattct gcagcatttg aggacctgag agtttcgaat 1080
   ttcattagag gaaccaaagt aatcccaaga ggacagttag caaccagggg agtgcaaatt 1140
   gcttcaaatg aaaacatgga gacaatagat tctagcacac tcgaactgag gagcagatat 1200
   tgggcaataa ggaccaggag tgggggaaac accagtcaac agagagcatc tgcaggacag 1260
   ataagtgtgc aacccacttt ctcagtgcag agaaatcttc cctttgaaag agcaaccatt 1320
   atggctgcat tcactggaaa cactgagggg aggacttccg acatgagaac ggaaatcata 1380
   aggatgatgg aaaatgccag atcagaagat gtgtctttcc aggggcgggg agtcttcgag 1440
   ctctcggacg aaaaggcaac gaacccgatc gtgccttcct ttgacatgag caatgaaggg 1500
   tcttatttct tcggagacaa tgctgaggag tttgacagtt aaagaaaaat acccttgttt 1560
   ctact                                                             1565
<210> 21
   <211> 1096
   <212> DNA
   <213> Influenza A virus
<400> 21
   gtcaatcatg aagacaacca ttattttgat actactgacc cattgggtct acagtcaaaa 60
   cccaaccagt ggcaacaaca cagccacatt atgtctggga caccatgcag tagcaaatgg 120
   aacattggta aaaacaataa ctgatgacca aattgaggtg acaaatgcta ctgaattagt 180
   tcagagcatt tcaataggga aaatatgcaa caactcatat agggttctag atggaagaaa 240
   ttgcacatta atagatgcaa tgctaggaga cccccactgt gatgtctttc agtatgagaa 300
   ttgggacctc ttcatagaaa gaagcagcgc tttcagcaat tgctacccat atgacatccc 360
   tgactatgca tcgctccggt ccattgtagc atcctcagga acattagaat tcacagcaga 420
   gggattcaca tggacaggtg tcactcaaaa cggaggaagt ggagcctgca aaaggggatc 480
   agccgatagt ttctttagcc gactgaattg gctaacaaaa tctggaaact cttaccccac 540
   attgaatgtg acaatgccta acaataaaaa tttcgacaaa ctatacatct gggggattca 600
   tcacccgagc tcaaacaatg agcagacaaa attgtatatc caagaaacag gacgagtaac 660
   agtctcaaca aaaagaagtc aacaaacaat aatccctaac atcggatcta gaccgtgggt 720
   caggggtcaa tcaggcagga taagcatata ctggaccatt gtaaaacctg gagatatcct 780
   aatgataaac agcaatggca acttagttgc accgcgggga tattttaaat tgagaacagg 840
   gagaagctct gtaatgagat cagatgcacc catagacatt tgtgtgtctg aatgtattac 900
   accaaatgga agcatcccca acgacaaacc atttcaaaat gtgaacaaag ttacatatgg 960
   aaaatgcccc aaatatatca ggcaaaacac tttaaagctg gccactggga tgaggaatgt 1020
   accagaaaag caaatcagag gaatctttgg agcaatagcg ggattcatag aaaacggctg 1080
   ggaaggaatg gttgat 1096
<210> 22
   <211> 1061
   <212> DNA
   <213> Influenza A virus
<400> 22
   agcaaaagca ggggatattt ctgtcaatca tgaagacaac cattattttg atactactga 60
   cccattgggc ctacagtcaa aacccaatca gtggcaacaa cacagccaca ttgtgtctgg 120
   gacaccatgc agtagcaaat ggaacattgg taaaaacaat aactgatgat caaattgagg 180
   tgacaaatgc tactgaatta gttcagagca tttcaatggg gaaaatatgc aacaactcat 240
   atagagttct agatggaaga aattgcacat taatagatgc aatgctagga gacccccact 300
   gtgatgtctt tcagtatgag aattgggacc tctttataga aagaagcagc gctttcagca 360
   attgctaccc atatgacatc cctgactatg catcgctccg gtccattgta gcatcctcag 420
   gaacattgga attcacagca gagggattca catggacagg tgtcactcaa aacggaagaa 480
   gtggagcctg caaaagggga tcagccgata gtttctttag ccgactgaat tggctaacaa 540
   aatctggaaa ctcttacccc acattgaatg tgacaatgcc taacaataaa aatttcgaca 600
   aactatacat ctgggggatt catcacccga gctcaaacca agagcagaca aaattgtaca 660
   tccaagaatc aggacgagta acagtctcaa caaaaagaag tcaacaaaca ataatcccta 720
   acatcggatc tagaccgtgg gtcaggggtc aatcaggcag gataagcata tactggacca 780
   ttgtaaaacc tggagatatc ctaatgataa acagtaatgg caacttagtt gcaccgcggg 840
   gatattttaa attgaaaaca gggaaaagct ctgtaatgag atcagatgca cccatagaca 900
   tttgtgtgtc tgaatgtatt acaccaaatg gaagcatctc caacgacaaa ccattccaaa 960
   atgtgaacaa agttacatat ggaaaatgcc ccaagtatat caggcaaaac actttaaagc 1020
   tggccactgg gatgaggaat gaaccagaaa agcaaatcag a 1061
<210> 23
   <211> 1061
   <212> DNA
   <213> Influenza A virus
<400> 23
   agcaaaagca ggggatattt ctgtcaatca tgaagacaac cattattttg atactactga 60
   cccattgggt ctacagtcaa aacccaacca gtggcaacaa cacagccaca ttatgtctgg 120
   gacaccatgc agtagcaaat ggaacattgg taaaaacaat aactgatgac caaattgagg 180
   tgacaaatgc tactgaatta gttcagagca tttcaatagg gaaaatatgc aacaactcat 240
   atagagttct agatggaaga aattgcacat taatagatgc aatgctagga gacccccact 300
   gtgatgtctt ccagtatgag aattgggacc tcttcataga aagaagcagc gctttcagca 360
   attgctaccc atatgacatc cctgactatg catcgctccg gtccattgta gcatcctcag 420
   gaacattaga attcacagca gagggattca catggacagg tgtcactcaa aacggaagaa 480
   gtggagcctg caaaagggga tcagccgata gtttctttag ccgactgaat tggctaacaa 540
   aatctggaaa ctcttacccc acattgaatg tgacaatgcc taacaataaa aatttcgaca 600
   aactatacat ctgggggatt catcacccga gctcaaacca aaagcagaca gaattgtaca 660
   tccaagaatc aggacgagta acagtctcaa caaaaagaag tcaacaaacg ataatcccta 720
   atatcggatc tagaccgtgg gttaggggtc aatcaggcag gataagcata tactggacca 780
   ttgtaaaacc tggagatatc ctaatgataa acagtaatgg caacttagtt gcaccgcggg 840
   gatattttaa attgaaaaca gggaaaagct ctgtaatgag atcagatgca cccatagaca 900
   tttgtgtgtc tgaatgtatt acaccaaatg gaagcatccc caacgacaaa ccatttcaaa 960
   atgtgaacaa agttacatat ggaaaatgcc ccaagtatat caggcaaaac actttaaagc 1020
   tggccactgg gatgaggaat gtaccagaaa agcaaatcag a                     1061
<210> 24
   <211> 1061
   <212> DNA
   <213> Influenza A virus
<400> 24
   agcaaaagca ggggatattt ctgtcaatca tgaagacaac cattattttg atactactga 60
   cccattgggt ctacagtcaa aacccaacca gtggaaacaa cacagccaca ttatgtctgg 120
   gacaccatgc agtagcaaat ggaacattgg taaaaacaat aactgatgac caaattgagg 180
   tgacaaatgc tactgaatta gttcagagca tttcaatagg gaaaatatgc aacaactcat 240
   atagagttct agatggaaga aattgcacat taatagatgc aatgctagga gacccccact 300
   gtgatgtctt ccagtatgag aattgggacc tcttcataga aagaagcagc gctttcagca 360
   attgctaccc atatgacatc cctgactatg catcgctccg gtccattgta gcatcctcag 420
   gaacattgga attcacagca gagggattca catggacagg tgtcactcaa aacggaagaa 480
   gtggagcctg caaaagggga tcagccgata gtttctttag ccgactgaat tggctaacaa 540
   aatctggaaa ctcttacccc acattgaatg tgacaatgcc taacaataaa aatttcgaca 600
   aactatacat ctgggggatt catcacccga gctcaaacca aaagcagaca gaattgtaca 660
   tccaagaatc aggacgagta acagtctcaa caaaaagaag tcaacaaacg ataatcccta 720
   atatcggatc tagaccgtgg gtcaggggtc aatcaggcag gataagcata tactggacca 780
   ttgtaaaacc tggagatatc ctaatgataa acagtaatgg caacttagtt gcaccgcggg 840
   gatattttaa attgaaaaca gggaaaagct ctgtaatgag atcagatgca cccatagaca 900
   tttgtgtgtc tgaatgtatt acaccaaatg gaagcatccc caacgacaaa ccatttcaaa 960
   atgtgaacaa agttacatat ggaaaatgcc ccaagtatat caggcaaaac actttaaagc 1020
   tggccactgg gatgaggaat gtaccagaaa agcaaatcag a                     1061
<210> 25
   <211> 1061
   <212> DNA
   <213> Influenza A virus
<400> 25
   agcaaaagca ggggatattt ctgtcaatca tgaagacaac cattattttg atactactga 60
   cccattgggt ctacagtcaa aacccaacca gtggaaacaa cacagccaca ttatgtctgg 120
   gacaccatgc agtagcaaat ggaacattgg taaaaacaat aactgatgac caaattgagg 180
   tgacaaatgc tactgaatta gttcagagca tttcaatagg gaaaatatgc aacaactcat 240
   ataaagttct agatggaaga aattgcacat taatagatgc aatgctagga gacccccact 300
   gtgatgtctt ccagtatgag aattgggacc tcttcataga aagaagcagc gctttcagca 360
   attgctaccc atatgacatc cctgactatg catcgctccg gtccattgta gcatcctcag 420
   gaacattaga attcacagca gagggattca catggacagg tgtcactcaa aacggaagaa 480
   gtggagcctg caaaagggga tcagccgata gtttctttag ccgactgaat tggctaacaa 540
   aatctggaaa ctcttacccc acattgaatg tgacaatgcc taacaataaa aatttcgaca 600
   aactatacat ctgggggatt catcacccga gctcaaacca acagcagaca gaattgtaca 660
   tccaagaatc aggacgagta acagtctcaa caaaaagaag tcaacaaacg atagtcccta 720
   atatcggatc tagaccgtgg gttaggggtc aatcaggcag gataagcata tactggacca 780
   ttgtaaaacc tggagatatc ctaatgataa acagtaatgg caacttagtt gcaccgcggg 840
   gatattttaa attgaaaaca gggaaaagct ctgtaatgag atcagatgca cccatagaca 900
   tttgtgtgtc tgaatgtatt acaccaaatg gaagcatccc caacgacaaa ccatttcaaa 960
   atgtgaacaa agttacatat ggaaaatgcc ccaagtatat caggcaaaac actttaaagc 1020
   tggccactgg gatgaggaat ataccagaaa agcaaatcag a                     1061
<210> 26
   <211> 1763
   <212> DNA
   <213> Influenza A virus
<400> 26
   agcaaaagca ggggatactt tctgtcaatc atgaagacaa ccattatttt gatactactg 60
   acccattggg tctacagtca aaacccaacc agtggcaaca acacagccac actatgtctg 120
   ggacaccatg cagtagcaaa tggaacattg gtaaaaacaa taactgatga ccaaattgag 180
   gtgacaaatg ctactgaatt agttcagagc acttcaatag ggaaaatatg caacaaccca 240
   tatagggttc tagatggaag aaactgcaca ttaatagatg caatgctagg agatccccac 300
   tgtgatgttt ttcagtatga gaattgggac ctcttcatag aaagaagcag cgctttcagc 360
   aattgctacc catatgacat ccctgactat gcatcgctcc ggtctattgt ggcatcttca 420
   ggaacattag aattcacagc agagggattc acatggacag gtgtcactca aaacggagga 480
   agtggagcct gcagaagggg gtcagccgat agtttcttta gccgactgaa ttggctaaca 540
   aaatctggaa attcttaccc cacattgaat gtaacaatgc ctaacaataa caatttcgat 600
   aaactataca tctgggggat ccatcacccg agcacaaaca atgagcagac aaaattgtat 660
   atccaagaat cagggcgagt aacagtctca acaaaaagaa gtcaacaaac aataatcccc 720
   aacatcggat ctagaccgtg ggtcaggggt caatcaggca ggataagcat atattggacc 780
   attgtgaaac ctggagatat cctaatgata aacagtaatg gcaacttagt tgcaccgcgg 840
   ggatatttta aaatgcgaac agggaaaagc tctgtaatga gatcagatgc acccatagac 900
   acttgtgtgt ccgagtgtat tacaccaaat ggaagcatcc ccaacgacaa accatttcaa 960
   aatgtgaaca aagttacata tggaaaatgc cccaagtata tcaagcagaa tactttgaag 1020
   ctggccactg ggatgaggaa tgtaccagaa aagcaaatca gaggaatctt tggagcaata 1080
   gcgggattca tagaaaacgg ctgggaagga atggttgatg ggtggtatgg attccgatat 1140
   cagaattcgg aaggaacagg acaagctgca gatctaaaga gcactcaagc agccatcgac 1200
   cagatcaatg gaaaattgaa cagagtgatt gaaaggacca atgagaaatt ccatcaaata 1260
   gagaaggaat tctcagaagt agaagggaga atccaggact tggagaagta tgtagaagac 1320
   accaaaatag acctatggtc ctacaatgca gagttactgg tggctctaga aaatcaacat 1380
   acgattgact taacagatgc agaaatgaat aaattattcg agaagactag gcgccagtta 1440
   agagaaaacg cggaagacat ggggggtgga tgtttcaaga tttatcacaa atgtgataat 1500
   gcatgcattg gatcaataag aaatgggaca tatgaccatt acatatacag agatgaagca 1560
   ttaaacaacc gatttcaaat taaaggtgtt gaattgaaat caggctacaa agattggata 1620
   ctgtggattt cattcgccat atcatgcttc ttaatttgcg ttgttctatt gggtttcatc 1680
   atgtgggctt gccaaaaagg caacatcaga tgcaacattt gcatttgagt aaactgataa 1740
   ttaaaaacac ccttgtttct act 1763
<210> 27
   <211> 1027
   <212> DNA
   <213> Influenza A virus
<400> 27
   agcaaaagca ggtagatatt taaagatgag tcttctaacc gaggtcgaaa cgtacgttct 60
   ctctattgta ccatcaggcc ccctcaaagc cgagatcgcg cagagacttg aagatgtctt 120
   tgcagggaag aacaccgatc ttgaggcact catggaatgg ctaaagacaa gaccaatcct 180
   gtcacctctg actaaaggga ttttaggatt tgtgttcacg ctcaccgtgc ccagtgagcg 240
   aggactgcaa cgtagacgct ttgtccaaaa tgcccttagt ggaaacggag atccaaataa 300
   catggacaga gcagtaaaac tgtacaagaa gcttaaaaga gaaataacat tccatggggc 360
   aaaagaggtg gcactcagct attccactgg tgcactagcc agctgcatgg gactcatata 420
   caacagaatg gggactgtga caaccgaagt ggcatttggc ctggtatgcg ccacatgtga 480
   acagattgct gattcccagc atcgatctca caggcagatg gtgacaacaa ccaacccact 540
   aatcagacat gaaaacagaa tggtactagc cagtaccaca gctaaaacca tggagcaggt 600
   ggcagggtcg agtgagcagg cagcagaggc catggaggtt gctagtaagg ccaggcagat 660
   ggtgcaggca atgaggacca ttgggaccca ccctagctcc agtgccggtt tgaaagatga 720
   tcttcttgaa aatttgcagg cctaccagaa acggatggga gtgcaaatgc agcggttcaa 780
   gtgatcctct cgttattgca gcaagtatca ttgggatctt gcacttgata ttgtggattc 840
   ttgatcgcct tttcttcaaa ttcatttatc gtctccttaa atacggtttg aaaagagggc 900
   cttctacgga aggagtacct gagtctatga gggaagaata tcggcaggaa cagcagaatg 960
   ctgtggatgt tgacgatggt cattttgtca acatagagct ggagtaaaaa actaccttgt 1020
   ttctact                                                           1027
<210> 28
   <211> 890
   <212> DNA
   <213> Influenza A virus
<400> 28
   agcaaaagca gggtgacaaa aacataatgg attccaacac tgtgtcaagc tttcaggtag 60
   actgttttct ttggcatgtc cgcaaacgat ttgcagacca agaactgggt gatgccccat 120
   tccttgaccg gcttcgccga gaccagaagt ccctaaaagg aagaggcagc actcttggtc 180
   tggacatcga aacagccact cgtgcaggaa agcagatagt ggagcggatt ctggaagagg 240
   agtcagatga ggcacttaaa atgaccattg cctctgttcc tgcttcacgc tacttaactg 300
   acatgactct tgatgagatg tcaagagact ggttcatgct catgcccaag cagaaagtaa 360
   caggctccct atgtataagg atggaccagg caatcatgga taagaacatc atactaaaag 420
   caaactttag tgtgattttc gaaaggctgg agacactaat actacttaga gctttcaccg 480
   aagaaggagc agtcgttggc gaaatttcac cattgccttc tcttccagga catactaatg 540
   aggatgtcaa aaatgcaatt ggggtcctca tcggaggact taaatggaat gataacacag 600
   ttagaatctc tgaaactcta cagagattcg cttggagaag cagtcatgag aatgggagac 660
   cttcattccc tccaaagcag aaacgaaaaa tggcgagaac aattgagtca gaagtttgaa 720
   gaaataaggt ggttgattga agaagtgcga catagattga aaaatacaga aaatagtttt 780
   gaacaaataa catttatgca agccttacaa ctattgcttg aagtagaaca agagataaga 840
   actttctcgt ttcagcttat ttaatgataa aaaacaccct tgtttctact            890
<210> 29
   <211> 982
   <212> DNA
   <213> Influenza A virus
<400> 29
   atgagtcttc tgaccgaggt cgaaacgtac gttctctcta tcgtaccatc aggccccctc 60
   aaagccgaga tcgcgcagag acttgaagat gtctttgcag ggaagaacac cgatcttgag 120
   gcactcatgg aatggctaaa gacaagacca atcctgtcac ctctgactaa agggatttta 180
   ggattcgtat tcacgctcac cgtgcccagt gagcgaggac tgcagcgtag acgctttgtc 240
   caaaatgccc ttagtggaaa cggagatcca aacaacatgg acagagcagt aaaactgtac 300
   aggaaactta aaagagaaat aacattccat ggggcaaaag aggtggcact cagctattcc 360
   actggtgcac tagccagctg catgggactc atatacaaca gaatgggaac tgtgacaacc 420
   gaagtggcat ttggcctagt atgcgccaca tgtgaacaga ttgctgattc ccagcatcga 480
   tctcacaggc agatggtgac aacaaccaac ccattaatca gacatgaaaa cagaatggta 540
   ttagccagta ccacggctaa agccatggag cagatggcag ggtcgagtga gcaggcagca 600
   gaggccatgg aggttgctag taaggctagg cagatggtac aggcaatgag gaccattggg 660
   acccacccta gctccagtgc cggtttgaaa aatgatctcc ttgaaaattt gcaggcctac 720
   cagaaacgga tgggagtgca aatgcagcga ttcaagtgat cctctcgtta ttgcagcaag 780
   tatcattggg atcttgcact tgatattgtg gattcttgat cgccttttct tcaaattcat 840
   ttatcgtcgc cttaaatacg ggttgaaaag agggccttct acggaaggag tacctgagtc 900
   tatgagggaa gaatatcggc aggaacagca gaatgctgtg gatgttgacg atggtcattt 960
   tgtcaacata gagctggagt aa                                          982
<210> 30
   <211> 982
   <212> DNA
   <213> Influenza A virus
<400> 30
   atgagtcttc taaccgaggt cgaaacgtac gttctctcta tcgtaccatc aggccccctc 60
   aaagccgaga tcgcgcagag acttgaagat gtctttgcag ggaagaacac cgatcttgag 120
   gcactcatgg aatggctaaa gacaagacca atcctgtcac ctctgactaa agggatttta 180
   ggatttgtgt tcacgctcac cgtgcccagt gagcgaggac tgcagcgtag acgctttgtc 240
   caaaatgccc ttagtggaaa cggagatcca aacaacatgg acagagcagt aaaactgtac 300
   aggaagctta aaagagaaat aacattccat ggggcaaaag aggtggcact cagctattcc 360
   actggtgcac tagccagctg catgggactc atatacaaca gaatggggac tgtgacaacc 420
   gaagtggcat ttggcctggt atgcgccaca tgtgaacaga ttgctgattc ccagcatcga 480
   tctcacaggc agatggtgac aacaaccaac ccactaatca gacatgaaaa cagaatggta 540
   ctagccagta ccacagctaa agccatggaa cagatggcag ggtcgagtga gcaggcagca 600
   gaggccatgg aggttgctag taaggccagg cagatggtac aggcaatgag gaccattggg 660
   acccacccta gctccagtgc cggtttgaaa gatgatcttc ttgaaaattt gcaggcctac 720
   cagaaacgga tgggagtgca aatgcagcga ttcaagtgac cctctcgtta ttgcagcaag 780
   tatcattggg atcttgcact tgatattgtg gattcttgat cgccttttct tcaaattcat 840
   ttatcgtcgc cttaaatacg gtttgaaaag agggccttct acggaaggag tacctgagtc 900
   tatgagggaa gaatatcggc aggaacagca gaatgctgtg gatgttgacg atggtcattt 960
   tgtcaacata gagctggagt aa 982
<210> 31
   <211> 838
   <212> DNA
   <213> Influenza A virus
<400> 31
   atggattcca acactgtgtc aagctttcag gtagactgtt ttctttggca tgtccgcaaa 60
   cgattcgcag accaagaact gggtgatgcc ccattccttg accggcttcg ccgagaccag 120
   aagtccctaa aaggaagagg tagcactctt ggtctggaca tcgaaacagc cactcgtgca 180
   ggaaagcaga tagtggagca gattctggaa gaggaatcag atgaggcact taaaatgacc 240
   attgcctctg ttcctgcttc acgctactta actgacatga ctcttgatga gatgtcaaga 300
   gactggttca tgctcatgcc caagcagaaa gtaacaggct ccctatgtat aagaatggac 360
   caggcaatca tggataagaa catcatactt aaagcaaact ttagtgtgat tttcgaaagg 420
   ctggaaacac taatactact tagagccttc accgaagaag gagcagtcgt tggcgaaatt 480
   tcaccattgc cttctcttcc aggacatact aatgaggatg tcaaaaatgc aattggggtc 540
   ctcatcggag gacttaaatg gaatgataat acggttagaa tctctgaaac tctacagaga 600
   ttcgcttgga gaagcagtca tgagaatggg agaccttcat tccctccaaa gcagaaacga 660
   aaaatggaga gaacaattga gccagaagtt tgaagaaata agatggttga ttgaagaagt 720
   gcgacataga ttgaaaaata cagaaaatag ttttgaacaa ataacattta tgcaagcctt 780
   acaactattg cttgaagtag aacaagagat aagaactttc tcgtttcagc ttatttaa   838
<210> 32
   <211> 838
   <212> DNA
   <213> Influenza A virus
<400> 32
   atggattcca acactgtgtc aagctttcag gtagactgtt ttctttggca tgtccgcaaa 60
   cgatttgcag accaagaact gggtgatgcc ccattccttg accggcttcg ccgagaccag 120
   aagtccctaa aaggaagagg cagcactctt ggtctggaca tcgaaacagc cactcgtgca 180
   ggaaagcaga tagtggagca gattctggaa gaggaatcag atgaggcact taaaatgacc 240
   attgcctctg ttcctgcttc acgctactta actgacatga ctcttgatga gatgtcaaga 300
   gactggttca tgctcatgcc caagcagaaa gtaacaggct ccctatgtat aaggatggac 360
   caggcaatca tggataagaa catcatacta aaagcaaact ttagtgtgat tttcgaaagg 420
   ctggagacac taatactact tagagccttc accgaagaag gagcagtcgt tggcgaaatt 480
   tcaccattgc cttctcttcc aggacatact aatgaggatg tcaaaaatgc aattggggtc 540
   ctcatcggag gacttaaatg gaatgataat acagttagag tctctgaaac tctacagaga 600
   ttcgcttgga gaagcagtca tgagaatggg agaccttcat tccctccaaa gcagaaacga 660
   aaaatggcga gaacaattga gccagaagtt tgaagaaata agatggttga ttgaagaagt 720
   gcgacataga ttgaaaaata cagaaaatag ttttgaacaa ataacattta tgcaagcctt 780
   acaactattg cttgaagtag aacaagagat aagaactttc tcgtttcagc ttatttaa 838
<210> 33
   <211> 1565
   <212> DNA
   <213> Influenza A virus
<400> 33
   agcaaaagca gggtagataa tcactcactg agtgacatca aagtcatggc gtctcaaggc 60
   accaaacgat cttatgagca gatggaaact ggtggggaac gccagaatgc aactgaaatc 120
   agagcatctg tcggaaggat ggtgggagga atcggccggt tctatgttca gatgtgtact 180
   gagcttaaac tcaacgacca tgaagggcgg ctgattcaga acagcataac aatagaaagg 240
   atggtacttt cggcattcga cgaaagaaga aacaagtacc tcgaggagca tcccagtgct 300
   gggaaagacc ccaagaaaac gggaggcccg atatacagaa ggaaagatgg gaaatggatg 360
   agagaactca tcctccatga taaagaagaa atcatgagga tctggcgtca ggccaacaat 420
   ggtgaagacg ctactgctgg tcttactcat atgatgatct ggcactccaa tctcaatgac 480
   accacatacc aaagaacaag ggctcttgtt cgggctggga tggatcccag aatgtgctct 540
   ctgatgcaag gatcaaccct cccacggaga tctggagctg ccggtgctgc agtaaaaggt 600
   gttggaacaa tggtaatgga actcatcagg atgatcaaac gcgggataaa tgatcgaaat 660
   ttctggagag gtgaaaatgg tcgaagaacc agaattgctt atgaaagaat gtgcaatatc 720
   ctcaaaggga aattccaaac agcagcacaa cgggcaatga tggaccaagt gagggagggc 780
   cgcaatcctg gaaatgctga gattgaggat ctcattttct tggcacgatc agcactcatt 840
   ttgagaggat cagtagccca taaatcatgc ctacctgcct gtgtttatgg ccttgcagta 900
   gccagtgggt atgactttga gaaggaagga tactctctgg ttggaattga tcctttcaaa 960
   ctactccaga acagccaaat tttcagtcta atcagaccga aagaaaatcc agcacacaag 1020
   agccagttgg tgtggatggc atgccattct gcagcatttg aggacctgag agttttgaat 1080
   ttcattagag gaaccaaagt aatcccaaga ggacagttag caaccagagg agtgcaaatt 1140
   gcttcaaatg aaaacatgga gacaatagat tctagcacac tcgaactgag gagcagatat 1200
   tgggcaataa ggaccaggag tggagggaac accagtcaac agagagcatc tgcaggacag 1260
   ataagtgtgc aacccacttt ctcagtgcag agaaatcttc cctttgaaag agcaaccatt 1320
   atggctgcat tcactgggaa cactgagcgg aggacttccg acatgagaac ggaaatcata 1380
   aggatgatgg aaaatgccag atcagaagat gtgtctttcc aggggcgggg agtcttcgag 1440
   ctctcggacg aaaaggcaac gaacccgatc gtgccttcct ttgacatgag caatgaaggg 1500
   tcttatttct tcggagacaa tgctgaggag tttgacagtt aaagaaaaat acccttgttt 1560
   ctact 1565
<210> 34
   <211> 2341
   <212> DNA
   <213> Influenza A virus
<400> 34
   agcaaaagca ggtcaaatat attcaatatg gagagaataa aagaactgag agatctaatg 60
   tcacagtccc gcacccgcga gatactaaca aaaactactg tggaccatat ggccataatc 120
   aagaaataca catcaggaag acaagagaag aaccccgcac ttaggatgaa gtggatgatg 180
   gcaatgaaat acccaattac agcagataag aggataatgg aaatgattcc tgagagaaat 240
   gaacaggggc aaaccctttg gagcaaaacg aacgatgctg gctcagaccg cgtaatggta 300
   tcacctctgg cagtgacatg gtggaatagg aatggaccaa caacgagcac aattcattat 360
   ccaaaagtct acaaaactta ttttgaaaaa gttgaaaggt taaaacacgg aacctttggc 420
   cccgttcatt ttaggaatca agtcaagata agacggagag ttgacgtaaa ccctggtcac 480
   gcggacctca gtgccaaaga agcacaagat gtgatcatgg aagttgtttt cccaaatgaa 540
   gtgggagcca gaattctaac atcggaatca caactaacaa taaccaaaga gaaaaaagaa 600
   gaacttcagg actgcaaaat tgcccccttg atggtagcat acatgctaga aagagagttg 660
   gtccgaaaaa caaggttcct cccagtggct ggcggaacaa gcagtgtata cattgaggtg 720
   ttgcatctga ctcagggaac gtgctgggaa caaatgtaca ccccaggagg agaagttaga 780
   aacgatgaca ttgatcaaag tttaattatt gctgcccgga acatagtgaa aagagcgaca 840
   gtatcagcag atccactagc atccctgctg gagatgtgcc acagtacaca gattggtgga 900
   ataaggatgg tagacatcct taagcagaat ccaacagagg aacaagctgt ggatatatgc 960
   aaagcagcaa tggggttaag aattagctca tcattcagct ttggtggatt cacctttaag 1020
   agaacaagtg gatcatcagt caagagagaa gaagaaatgc ttacgggcaa ccttcaaaca 1080
   ttgaaaataa gagtgcatga gggctatgaa gaattcacaa tggtcggaag aagagcaaca 1140
   gccattctca gaaagacaac cagaagattg attcaattga tagtaagtgg gagagatgaa 1200
   cagtcaattg ctgaagcaat aattgtagcc atggtgtttt cgcaagaaga ttgcatgata 1260
   aaagcagttc gaggcgattt gaacttcgtt aatagagcaa atcagcgctt gaaccccatg 1320
   catcaactct tgaggcattt ccaaaaggat gcaaaagtgc ttttccagaa ttgggggatt 1380
   gaacccatcg acaatgtgat gggaatgatc ggaatattgc ccgacatgac cccaagcacc 1440
   gagatgtcat tgagaggagt gagagtcagc aaaatgggag tggatgagta ctccagcact 1500
   gagagagtgg tggtgagcat tgaccgtttt ttaagagttc gggatcaaag gggaaacata 1560
   ctactgtccc ctgaagaggt cagtgaaaca caaggaacgg aaaagctgac aataatttat 1620
   tcatcatcaa tgatgtggga gattaatggt cccgagtcag tgttggtcaa tacttatcaa 1680
   tggatcatca gaaactggga aattgtgaaa attcaatggt cacaggatcc cacaatgtta 1740
   tacaataaga tagaatttga gccattccag tccctggtcc ctagggccac cagaagccaa 1800
   tacagcggtt tcgtaaggac cctgtttcag caaatgcgag atgtacttgg aacatttgac 1860
   actgctcaaa taataaaact cctccctttt gccgctgctc ctccggaaca gagtagaatg 1920
   cagttctctt ctttgactgt taatgtaaga ggatcgggaa tgaggatact tgtaagaggc 1980
   aattccccag tgttcaacta caacaaagcc actaagaggc tcacagtcct cggaaaggat 2040
   gcaggtgcgc ttactgaaga cccagatgaa ggtacggctg gagtagaatc tgctgttctg 2100
   agagggtttc tcatcttagg taaagaaaac aagagatatg gcccagcact aagcatcaat 2160
   gaactgagca aacttacaaa aggggagaaa gctaatgtgc taattgggca aggggacgtg 2220
   gtgttggtaa tgaaacggaa acgtgactct agcatactta ctgacagtca gacagcgacc 2280
   aaaaggattc ggatggccat caattagtgt tgaattgttt aaaaacgacc ttgtttctac 2340
   t 2341
<210> 35
   <211> 1762
   <212> DNA
   <213> Influenza A virus
<220>
   <221> modified_base
   <222> (313)
   <223> n is a, c, g, t, unknown or other
<400> 35
   agcaaaagca ggggatattt ctgtcaatca tgaagacaac cattgttttg atactactga 60
   cccattgggt ctacagtcaa aacccaacca gtggcaacaa cacagccaca ttatgtctgg 120
   gacaccatgc agtagcaaat ggaacactgg taaaaacaat aactgatgac cagattgagg 180
   tgacaaatgc tactgaatta gttcagagca tttcaatagg gaaaatatgc aacaactcat 240
   atagggttct agatggaaga aattgcacat taatagatgc aatgctagga gacccccact 300
   gtgatgtttt tcngtatgag aattgggacc tcttcataga aagaagcagc gctttcagca 360
   attgctaccc atatgacatc cctgactatg catcgctccg gtctattgtg gcatcctcag 420
   gaacattaga attcacagca gagggattca catggacagg tgtcactcaa aacggaagaa 480
   gtggagcctg caaaagggga tcagccgata gtttctttag ccgactgaat tggctaacaa 540
   aatctggaaa ttcttacccc acattgaatg tgacaatgcc taacaataac aatttcgata 600
   aactatacat ctgggggatt catcacccga gctcaaacaa tgagcagaca aaattgtata 660
   tccaagaatc aggacgagta acagtctcaa caaaaagaag tcaacaaaca ataatcccca 720
   acatcggatc tagaccgtgg gtcaggggtc aatcaggcag gataagcata tattggacca 780
   ttgtgaaacc tggagatatc ctaatgataa acagtaatgg caacttagtt gcaccgcggg 840
   gatatttcaa attgagaaca gggaaaagct ctgtaatgag atcagatgca cccatagaca 900
   cttgtgtgtc tgaatgtatt acaccaaatg gaagcatccc caacgacaaa ccattccaaa 960
   atgtgaacaa agttacatat ggaaaatgcc ccaagtatat caggcaaaac actttgaagc 1020
   tggccactgg gatgaggaat gtaccagaaa agcaaatcag aggaatcttt ggagcaatag 1080
   cgggattcat agaaaacggc tgggaaggaa tggttgatgg gtggtatgga ttccgatatc 1140
   aaaattcgga aggaacagga caagctggag atctaaagag cactcaagca gccatcgacc 1200
   agatcaatgg aaaattaaac agagtgattg aaaggaccaa tgagaaattc catcaaatag 1260
   agaaggaatt ctcagaagta gaagggagaa tccaggactt ggagaagtat gtagaagaca 1320
   ccaaaataga cctatggtcc tacaatgcag aattgctggt ggctctagaa aatcaacata 1380
   caattgactt aacagatgca gaaatgaata aattattcga gaagactagg cgccagttaa 1440
   gagaaaacgc ggaagacatg ggaggtggat gtttcaggat ttaccacaaa tgtgataatg 1500
   catgcattgg atcaataaga aatgggacat atgaccatta catatacaga gatgaagcat 1560
   taaacaaccg atttcaaatt aaaggtgttg agttgaaatc aggctacaaa gattggatac 1620
   tgtggatttc attcgccata tcatgcttct taatttgcgt tgttctattg ggtttcatta 1680
   tgtgggcttg ccaaaaaggc aacatcagat gcaacatttg catttgagta aactgatagt 1740
   taaaaacacc cttgtttcta ct                                          1762
<210> 36
   <211> 1762
   <212> DNA
   <213> Influenza A virus
<220>
   <221> modified_base
   <222> (313)
   <223> n is a, c, g, t, unknown or other
<400> 36
   agcaaaagca ggggatattt ctgtcaatca tgaagacaac cattattttg atactactga 60
   cccattgggt ctacagtcaa aacccaacca gtggcaacaa cacagccaca ttatgtctgg 120
   gacaccatgc agtagcaaat ggaacattgg taaaaacaat aactgatgac caaattgagg 180
   tgacaaatgc tactgaatta gttcagagca tttcaatagg gaaaatatgc aacaactcat 240
   atagggttct agatggaaga aattgcacat taatagatgc aatgctagga gacccccact 300
   gtgatgtttt tcngtatgag aattgggacc tcttcataga aagaagcagc gcttccagca 360
   attgctaccc atatgacatc cctgactatg catcgctccg gtctattgtg gcatcctcag 420
   gaacattaga attcacagca gagggattca catggacagg tgtcactcaa aacggaagaa 480
   gtggagcctg caaaagggga tcagccgata gtttctttag ccgactgaat tggctaacaa 540
   aatctggaaa ttcttacccc acattgaatg tgacaatgcc taacaataac aatttcgata 600
   agctatacat ctgggggatc catcacccga gctcaaacaa tgagcagaca aaattgtata 660
   tccaagaatc aggacgagta acagtctcaa caaaaagaag tcaacaaaca ataatcccca 720
   acatcggatc tagaccgtgg gtcaggggtc aatcaggcag gataagcata tattggacca 780
   ttgtgaaacc tggagatatc ctaataataa acagtaatgg caacttagtt gcaccgcggg 840
   gatatttcaa attgcgaaca gggaaaagct ctgtaatgag atcagatgca cccatagaca 900
   cttgtgtgtc tgaatgtatt acaccaaatg gaagcatccc caacgacaaa ccatttcaaa 960
   atgtgaacaa agttacatat ggaaaatgcc ccaagtatat caggcaaaac actttgaagc 1020
   tggccactgg gatgaggaat gtaccagaaa agcaaatcag aggaatcttt ggagcaatag 1080
   cgggattcat agaaaacggc tgggaaggaa tggttgatgg gtggtatgga ttccgatatc 1140
   aaaactcgga aggaacagga caagctggag atctaaagag cactcaagca gccatcgacc 1200
   agatcaatgg aaaattgaac agagtgattg aaaggaccaa tgagaaattc catcaaatag 1260
   agaaggaatt ctcagaagta gaagggagaa tccaggactt ggagaagtat gtagaagaca 1320
   ccaaaataga cctatggtcc tacaatgcag agttgctggt ggctctagaa aatcaacata 1380
   caattgactt aacagatgca gaaatgaata aactattcga gaagactagg cgccagttaa 1440
   gagaaaacgc ggaagacatg ggaggtggat gtttcaagat ttatcacaaa tgtgataatg 1500
   catgcattgg atcaataaga aatgggacat atgaccatta catatacaga gatgaagcat 1560
   taaacaaccg atttcaaatt aaaggtgtag agctgaaatc aggctacaaa gattggatac 1620
   tgtggatttc attcgccata tcatgcttct taatttgcgt tgttctattg ggtttcatta 1680
   tgtgggcttg ccaaaaaggc aacatcagat gcaacatttg catttgagta aactgatagt 1740
   taaaaacacc cttgtttcta ct 1762
<210> 37
   <211> 693
   <212> DNA
   <213> Influenza A virus
<400> 37
   atggattcca acactgtgtc aagctttcag gtagactgtt ttctttggca tgtccgcaaa 60
   cgatttgcag accaagaact gggtgatgcc ccattccttg accggcttcg ccgagaccag 120
   aagtccctaa aaggaagagg tagcactctt ggtctggaca tcgaaacagc cactcgtgca 180
   ggaaagcaga tagtggagca gattctggaa gaggaatcag atgaggcatt taaaatgacc 240
   attgcctctg ttcctgcttc acgctactta actgacatga ctcttgatga gatgtcaaga 300
   gactggttca tgctcatgcc caagcagaaa gtaacaggct ccctatgtat aagaatggac 360
   caggcaatca tggataagaa catcatactt aaagcaaact ttagtgtgat tttcgaaagg 420
   ctggagacac taatactact cagggccttc accgaagaag gagcagtcgt tggcgaaatt 480
   tcaccattgc cttctcttcc aggacatact aatgaggatg tcaaaaatgc aattggggtc 540
   ctcatcggag gacttaaatg gaatgataat acggttagag tctctgaaac tctacagaga 600
   ttcgcttgga gaagcagtca tgagaatggg agaccttcat tccctccaaa gcagaaacga 660
   aaaatggaga gaacaattga gtcagaagtt tga                              693
<210> 38
   <211> 1032
   <212> DNA
   <213> Influenza A virus
<400> 38
   atgaagacaa ccattatttt gatactactg acccattggg tctacagtca aaacccaacc 60
   agtggcaaca acacagccac attatgtctg ggacaccatg cagtagcaaa tggaacattg 120
   gtaaaaacaa taactgatga ccaaattgag gtgacaaatg ctactgaatt agtccagagc 180
   atttcaatag ggaaaatatg caacaactca tatagggttc tagatggaag aaattgcaca 240
   ttaatagatg caatgctagg agacccccat tgtgatgatt ttcagtatga gaattgggac 300
   ctcttcatag aaagaagcag cgctttcagc aattgctacc catatgacat ccctgactat 360
   gcatcgctcc ggtccattgt agcatcctca ggaacattag aattcacagc agaggggttc 420
   acatggacag gtgtcactca aaacggagga agtggagcct gcaaaagggg atcagccgat 480
   agtttcttta gccgactgaa ttggctaaca aaatctggaa attcttaccc catattgaat 540
   gtgacaatgc ctaacaataa aaatttcgat aaactataca tctgggggat tcatcacccg 600
   agctcaaaca aagagcagac aaaattatat atccaagaat caggacgagt aacagtctca 660
   acagaaagaa gtcaacaaac agtaatccct aacatcggat ctaggccgtg ggtcaggggt 720
   caatcaggca ggataagcat atactggacc attgtaaaac ctggagatat tctaatgata 780
   aacagtaatg gcaacttagt tgcaccgcgg ggatatttta aattgagaac agggaaaagc 840
   tctgtaatga gatcagatgc actcatagac acttgtgtgt ctgaatgtat tacaccaaat 900
   ggaagcatcc ccaacgacaa accatttcaa aatgtgaaca aaattacata tggaaaatgc 960
   cccaagtata tcaggcaaaa cactttaaag ctggccactg ggatgaggaa tgtaccagaa 1020
   aagcaaatca ga                                                     1032
<210> 39
   <211> 1032
   <212> DNA
   <213> Influenza A virus
<400> 39
   atgaagacaa ccattatttt gatactactg acccattggg tctacagtca aaacccaacc 60
   agtggcaaca acacagccac attatgtctg ggacaccatg cagtagcaaa tggaacattg 120
   gtaaaaacaa taactgatga ccaaattgag gtgacaaatg ctactgaatt agttcagagc 180
   atttcaatag ggaaaatatg caacaactca tatagagttc tagatggaag aaattgcaca 240
   ttaatagatg caatgctagg agacccccac tgtgatgtct ttcagtatga gaattgggac 300
   ctcttcatag aaagaagcag cgctttcagc aattgctacc catatgacat ccctgactat 360
   gcatcgctcc ggtccattgt agcatcctca ggaacattgg aattcacagc agagggattc 420
   acatggacag gtgtcactca aaacggaaga agtggagcct gcaaaagggg atcagccgat 480
   agtttcttta gccgactgaa ttggctaaca aaatctggaa actcttaccc cacattgaat 540
   gtgacaatgc ctaacaataa aaatttcgac aaactataca tctgggggat tcatcacccg 600
   agctcaaacc aacagcagac agaattgtac atccaagaat caggacgagt aacagtctca 660
   acaaaaagaa gtcaacaaac gataatccct aatatcggat ctagaccatg ggtcaggggt 720
   caatcaggca ggataagcat atactggacc attgtaaaac ctggagatat cctaatgata 780
   aacagtaatg gcaacttagt tgcaccgcgg ggatatttta aattgaaaac agggaaaagc 840
   tctgtaatga gatcagatgc acccatagac atttgtgtgt ctgaatgtat tacaccaaat 900
   ggaagcatcc ccaacgacaa accatttcaa aatgtgaaca aagttacata tggaaaatgc 960
   cccaagtata tcaggcaaaa cactttaaag ctggccactg ggatgaggaa tgtaccagaa 1020
   aagcaaatca ga 1032
<210> 40
   <211> 1032
   <212> DNA
   <213> Influenza A virus
<400> 40
   atgaagacaa ccattatttt gatactactg acccattggg tctacagtca aaacccaacc 60
   agtggcaaca acacagccac attatgtctg ggacaccatg cagtagcaaa tggaacattg 120
   gtaaaaacaa taactgatga ccaaattgag gtgacaaatg ctactgaatt agttcagagc 180
   atttcaatag ggaaaatatg caacaactca tatagggttc tagatggaag aaattgcaca 240
   ttaatagatg caatgctagg agacccccac tgtgatgttt ttcagtatga gaattgggac 300
   ctcttcatag aaagaagcag cgctttcagc aattgctacc catatgacat ccctgactat 360
   gcatcgctcc ggtccattgt agcatcctca ggaacattag aattcacagc agagggattc 420
   acatggacag gtgtcactca aaacggaaga agtggagcct gcaaaagggg atcagccgat 480
   agtttcttta gccgactgaa ttggctaaca aaatctggaa attcttaccc catattgaat 540
   gtgacaatgc ctaacaataa aaatttcgat aaactataca tctgggggat tcatcacccg 600
   agctcaaaca aagagcagac aaaattgtat atccaagaat caggacgagt aacagtctca 660
   acagaaagaa gtcaacaaac agtaatccct aacatcggat ctagaccgtg ggtcaggggt 720
   caatcaggca ggataagcat atactggacc attgtaaaac ctggagatat tctaatgata 780
   aacagtaatg gcaacttagt tgcaccgcgg ggatatttta aattgagaac agggaaaagc 840
   tctgtaatga gatcagatgc actcataggc acttgtgtgt ctgaatgtat tacaccaaat 900
   ggaagcatcc ccaacgacaa accatttcaa aatgtgaaca aagttacata tggaaaatgc 960
   cccaagtata tcaggcaaaa cactttaaag ctggccactg ggatgaggaa tgtaccagaa 1020
   aagcaaatca ga 1032
<210> 41
   <211> 1032
   <212> DNA
   <213> Influenza A virus
<400> 41
   atgaagacaa ccattatttt gatactactg acccattggg tctacagtca aaacccaacc 60
   agtggcaaca acacagccac attatgtctg ggacaccatg cagtagcaaa tggaacattg 120
   gtaaaaacaa taactgatga ccaaattgag gtgacaaatg ctactgaatt agttcagagc 180
   atttcaatag ggaaaatatg caacaactca tatagggttc tagatggaag aaattgcaca 240
   ttaatagatg caatgctagg agacccccat tgtgatgatt ttcagtatga gaattgggac 300
   ctcttcatag aaagaagcag tgctttcagc aattgctacc catatggcat ccctgactat 360
   gcatcgctcc ggtccattgt agcatcctca ggaacattag aattcacagc agaggggttc 420
   acatggacag gtgtcactca aaacggaaga agtggagcct gcaaaagggg atcagccgat 480
   agtttcttta gccgactcaa ttggctaaca aaatctggaa attcttaccc catattgaat 540
   gtgacaatgc ctaacaataa aaatttcgat aaactataca tctgggggat tcatcacccg 600
   agctcaaaca aagagcagac aaaattatat atccaagaat caggacgagt aacagtctca 660
   acagaaagaa gtcaacaaac agtaatccct aacatcggat ctaggccgtg ggtcaggggt 720
   caatcaggca ggataagcat atactggacc attgtaaaac ctggagatat tctaatgata 780
   aacaataatg gcaacttagt tgcaccgcgg ggatatttta aattgagaac agggaaaagc 840
   tctgtaatga gatcagatgc actcatagac acttgtgtgt ctgaatgtat tacaccaaat 900
   ggaagcatcc ccaacgacaa accatttcaa aatgtgaaca aagttacata tggaaaatgc 960
   cccaagtata tcaggcaaaa cactttaaag ctggccactg ggatgaggaa tataccagaa 1020
   aagcaaatca ga 1032
<210> 42
   <211> 1032
   <212> DNA
   <213> Influenza A virus
<400> 42
   atgaagacaa ccattatttt gatactactg acccattggg tctacagtca aaacccaacc 60
   agtggcaaca acacagccac attatgtctg ggacaccatg cagtagcaaa tggaacattg 120
   gtaaaaacaa taactgatga ccaaattgag gtgacaaatg ctactgaatt agttcagagc 180
   atttcaatag ggaaaatatg caacaactca tatagggttc tagatggaag aaattgcaca 240
   ttaatagatg caatgctagg agacccccac tgtgatgttt ttcagtatga gaattgggac 300
   ctcttcatag aaagaagcag cgctttcagc aattgctacc catatgacat ctctgactat 360
   gcatcgctcc ggtccattgt agcatcctca ggaacattag aattcacagc agagggattc 420
   acatggacag gtgtcactca aaacggaaga agtggagcct gcaaaagggg atcagccgat 480
   agtttcttta gccgactgaa ttggctaaca aaatctggaa attcttaccc catattgaat 540
   gtgacaatgc ctaacaataa aaatttcgat aaactataca tctgggggat tcatcacccg 600
   agctcaaaca aagagcagac aaaattgtat atccaagaat caggacgagt aacagtctca 660
   acagaaagaa gtcaacaaac agtaatccct aacatcggat ctagaccgtg ggtcaggggt 720
   caatcaggca ggataagcat atactggacc attgtaaaac ctggagatat tctaatgata 780
   aacagtaatg gcaacttagt tgcaccgcgg ggatatttta aattgagaac agggaaaagc 840
   tctgtaatga gatcagatgc acccataggc acttgtgtgt ctgaatgtat tacaccaaat 900
   ggaagcatcc ccaacgacaa accatttcaa aatgtgaaca aagttacata tggaaaatgc 960
   cccaagtata tcaggcaaaa cactttaaag ctggccactg ggatgaggaa tgtaccagaa 1020
   aagcaaatca ga 1032
<210> 43
   <211> 1032
   <212> DNA
   <213> Influenza A virus
<400> 43
   atgaagacaa ccattatttt gatactactg acccattggg tctacagtca aaacccaacc 60
   agtggcaaca acacagccac attatgtctg ggacaccatg cagtagcaaa tggaacattg 120
   gtaaaaacaa taactgatga ccaaattgag gtgacaaatg ctactgaatt agttcagagc 180
   atttcaatag ggaaaatatg caacaactca tatagagttc tagatggaag aaattgcaca 240
   ttaatagatg caatgctagg agacccccac tgtgatgtct ttcagtatga gaattgggac 300
   ctcttcatag aaagaagcag cgctttcagc aattgctacc catatgacat ccctgactat 360
   gcatcgctcc ggtccattgt agcatcctca ggaacattag aattcacagc agagggattc 420
   acatggacag gtgtcactca aaacggaaga agtggagcct gcaaaagggg atcagccgat 480
   agtttcttta gccgactgaa ttggctaaca aaatctggaa attcttaccc catattgaat 540
   gtgacaatgc ctaacaataa aaatttcgat aaactataca tctgggggat tcatcacccg 600
   agctcaaaca aagagcagac aaaattgtac atccaagaat caggacgagt aacagtctca 660
   acaaaaagaa gtcaacaaac aataatccct aacatcggat ctagaccgtg ggtcaggggt 720
   caatcaggca ggataagcat atactggacc attgtaaaac ctggagatat cctaatgata 780
   aacagtaatg gcaacttagt tgcaccgcgg ggatatttta aattgagaac agggaaaagc 840
   tctgtaatga gatcagatgc acccatagac atttgtgtgt ctgaatgtat tacaccaaat 900
   ggaagcatcc ccagcgacaa accatttcaa aatgtaaaca aagttacata tggaaaatgc 960
   cccaagtata tcaggcaaaa cactttaaag ctggccactg ggatgaggaa tgtaccagaa 1020
   aagcaaatca ga 1032
<210> 44
   <211> 1742
   <212> DNA
   <213> Influenza A virus
<400> 44
   ctgtcaatca tgaagacaac cattattttg atactactga cccattgggt ctacagtcaa 60
   aacccaacca gtggcaacaa cacagccaca ttatgtctgg gacaccatgc agtagcaaat 120
   ggaacattgg taaaaacaat aactgatgac caaattgagg tgacaaatgc tactgaatta 180
   gttcagagca tttcaatagg gaaaatatgc aacaactcat atagggttct agatggaaga 240
   aattgcacat taatagatgc aatgctagga gacccccact gtgatgtttt tcagtatgag 300
   aattgggacc tcttcataga aagaagcagc gctttcagca attgctaccc atatgacatc 360
   cctgactatg catcgctccg gtccattgta gcatcctcag gaacattaga attcacagca 420
   gagggattca catggacagg tgtcactcaa aacggaagaa gtggagcctg caaaagggga 480
   tcagccgata gtttctttag ccgactgaat tggctaacaa aatctggaaa ttcttacccc 540
   atattgaatg tgacaatgcc taacaataaa aatttcgata aactatacat ctgggggatt 600
   catcacccga gctcaaacaa agagcagaca aaattgtata tccaagaatc aggacgagta 660
   acagtctcaa cagaaagaag tcaacaaaca gtaatcccta acatcggatc tagaccgtgg 720
   gtcaggggtc aatcaggcag gataagcata tactggacca ttgtaaaacc tggagatatt 780
   ctaacgataa acagtaatgg caacttagtt gcaccgcggg gatattttaa attgagaaca 840
   gggaaaagct ctgtaatgag atcagatgca cccatagaca cttgtgtgtc tgaatgtatt 900
   acaccaaatg gaagcatccc caacgacaaa ccatttcaaa atgtgaacaa agttacatat 960
   ggaaaatgcc ccaagtatat caggcaaaac actttaaagc tggccaccgg gatgaggaat 1020
   gtaccagaaa agcaaatcag aggaatcttt ggagcaatag cgggattcat agaaaacggc 1080
   tgggaaggaa tggttgatgg gtggtatgga ttccgatatc aaaactcgga aggaacagga 1140
   caagctgcag atctaaagag cactcaagca gccatcgacc agatcaatgg aaaattaaac 1200
   agagtgattg aaaggaccaa tgagaaattc catcaaatag agaaggaatt ctcagaagta 1260
   gaagggagaa tccaggattt ggagaagtat gtagaagaca ccaaaataga cctatggtcc 1320
   tacaatgcag aattgctggt ggctctagaa aatcaacata caattgactt aacagatgca 1380
   gaaatgaata aattattcga gaagactagg cgccagttaa gagaaaacgc ggaagacatg 1440
   ggaggtggat gtttcaagat ttaccacaaa tgtgataatg catgcattgg atcaataaga 1500
   aatgggacat atgaccatta catatacaga gatgaagcat taaacaaccg atttcaaatc 1560
   aaaggtgttg agttgaaatc aggctacaaa gattggatac tgtggatttc attcgccata 1620
   tcatgcttct taatttgcgt tgttctattg ggtttcatta tgtgggcttg ccaaaaaggc 1680
   aacatcagat gcaacatttg catttgagta aactgatagt taaaaacacc cttgtttcta 1740
   ct 1742
<210> 45
   <211> 1761
   <212> DNA
   <213> Influenza A virus
<220>
   <221> modified_base
   <222> (1)..(19)
   <223> n is a, c, g, t, unknown or other
<220>
   <221> modified_base
   <222> (1760)..(1761)
   <223> n is a, c, g, t, unknown or other
<400> 45
   nnnnnnnnnn nnnnnnnnna aatgaacact cagattctaa tattagccat ttcggcattc 60
   ctctgtgtac gtgcagataa aatctgccta ggacatcatg ctgtgtctaa tggaaccaaa 120
   gtagacaccc ttactgaaaa gggaatagaa gtcgtcaatg caacagaaac agttgaacaa 180
   aaaaacatcc ccaagatctg ctcaaaaggg aaacagacta ttgaccttgg tcaatgtgga 240
   ttactaggga ccactattgg tcccccccaa tgcgaccaat ttcttgaatt ctctgctaat 300
   ttaataattg agagaagaga aggtgatgat atttgttatc caggcaaatt tgacaatgaa 360
   gaaacattga gacaaatact cagaaaatcc ggaggaatta aaaaggagaa tatgggattc 420
   acatataccg gagtgagaac caatggagag actagcgcct gtagaaggtc aagatcttcc 480
   ttttatgcag aaatgaaatg gctcctatct aacacagaca atggggtatt cccacaaatg 540
   acaaaatcct acaagaacac taagaaggag ccagctctga taatctgggg aatccaccac 600
   tcaggatcaa ctgctgaaca gactagattg tatggaagtg gaaacaagtt gataacagtt 660
   tggagttcca aataccaaca atcttttgcc ccaaaccctg gaccaaggcc gcaaatgaat 720
   ggccaatcag gaagaattga cttttactgg ctgatgttag atcccaatga tactgttaat 780
   ttcagtttta atggggcctt tatagcacct gaccgcgcca gttttctaag aggtaaatct 840
   ctaggaattc agagtgacgc acaacttgac aacaattgtg aaggtgaatg ttatcatatt 900
   ggaggtacca taattagcaa cttgcccttt caaaacatta atagcagagc aattgggaaa 960
   tgccccagat acgtaaagca aaaaagctta atgctagcaa ccggaatgaa aaatgttcct 1020
   gaaaattcta cacacaaaca gttaactcat cacatgcgca aaaaaagagg tttatttggt 1080
   gcaatagcag gatttattga aaatggatgg gaaggattaa tagatggatg gtatggatac 1140
   agacatcaga atgcacaagg agaaggaact gctgcagact acaaaagtac acaatctgct 1200
   gtcaatcaaa taaccgggaa attaaacaga ctaatagaaa aaaccaacca gcaatttgaa 1260
   ctaatagata atgaattcaa tgaaatagaa aagcaaattg gcaatgttat taactggact 1320
   agagattcta tcatcgaaat atggtcatat aatgcagaat tcctcgtggc agtggagaat 1380
   caacacacta ttgatttaac tgattcagag atgaacaaat tatatgaaaa ggtaagaaga 1440
   cagctgagag aaaatgctga ggaagatggt aatggctgtt ttgaaatatt tcaccaatgt 1500
   gacaatgatt gcatggccag cattagaaac aatacatatg atcataaaaa atacagaaag 1560
   gaggcaatac aaaacagaat tcagattgat gcagtaaagt tgagcagcgg ttacaaagaa 1620
   ataatacttt ggtttagctt cggggcatca tgtttcttat ttcttgccat tgcaatggtt 1680
   cttgctttca tatgcataaa aaatggaaac atgcggtgca ctatttgtat ataagtttga 1740
   aaaaacaccc ttgtttctan n 1761
<210> 46
   <211> 1096
   <212> DNA
   <213> Influenza A virus
<400> 46
   agtcaaaacc caaccagtgg caacaacaca gccacattat gtctgggaca ccatgcagta 60
   gcaaatggaa cattggtaaa aacaataact gatgaccaaa ttgaggtgac aaatgctact 120
   gaattagttc agagcatttc aatagggaaa atatgcaaca actcatatag ggttctagat 180
   ggaagaaatt gcacattaat agatgcaatg ctaggagacc cccactgtga tgtttttcag 240
   tatgagaatt gggacctctt catagaaaga agcagcgctt tcagcaattg ctacccatat 300
   gacatccctg actatgcatc gctccggtcc attgtagcat cctcaggaac attagaattc 360
   acagcagagg gattcacatg gacaggtgtc actcaaaacg gaagaagtgg agcctgcaaa 420
   aggggatcag ccgatagttt ctttagccga ctgaattggc taacaaaatc tggaaattct 480
   taccccatat tgaatgtgac aatgcctaac aataaaaatt tcgataaact atacatctgg 540
   gggattcatc acccgagctc aaacaaagag cagacaaaat tgtatatcca agaatcagga 600
   cgagtaacag tctcaacaga aagaagtcaa caaacagtaa tccctaacat cggatctaga 660
   ccgtgggtca ggggtcaatc aggcaggata agcatatact ggaccattgt aaaacctgga 720
   gatattctaa tgataaacag taatggcaac ttagttgctc cgcggggata ttttaaattg 780
   agaacaggga aaagctctgt aatgagatca gatgcaccca tagacacttg tgtgtctgaa 840
   tgtattacac caaatggaag catccccaac gacaaaccat ttcaaaatgt gaacaaagtt 900
   acatatggaa aatgccccaa gtatatcagg caaaacactt taaagctggc cactgggatg 960
   aggaatgtac cagaaaagca aatcagagga atctttggag caatagaggg attcatagaa 1020
   aacggctggg aaggaatggt tgatgggtgg tatggattcc gatatcaaaa ctcggaagga 1080
   acaggacaag ctgcag 1096
<210> 47
   <211> 1086
   <212> DNA
   <213> Influenza A virus
<400> 47
   ctgtcaatca tgaagacaac cattattttg atactactga cccattgggt ctacagtcaa 60
   aacccaacca gtggcaacaa cacagccaca ctatgtctgg gacaccatgc agtagcaaat 120
   ggaacattgg taaaaacaat aactgatgac caaattgagg tgacaaatgc cactgaatta 180
   gttcagagca cttcaatagg gaaaatatgc aacaacccat atagggttct agatggaaga 240
   aactgcacat taatagatgc aatgctagga gatccccact gtgatgtttt tcagtatgag 300
   aattgggacc tcttcataga aagaagcagc gctttcagca attgctaccc atatgacatc 360
   cctgactatg catcgctccg gtctattgtg gcatcttcag gaacattaga attcacagca 420
   gagggattca catggacagg tgtcactcaa aacggaagaa gtggcgcctg cagaagggga 480
   tcagccgata gtttctttag ccgactgaat tggctaacaa aatctggaga ttcttacccc 540
   acattgaatg tgacaatgcc taacaataac aatttcgata aactatacat ctgggggatc 600
   catcacccga gcacaaacaa tgagcagaca aaattgtatg tccaagaatc agggcgagta 660
   acagtctcaa caaaaagaag tcaacaaaca ataatcccca acatcggatc tagaccgtgg 720
   gtcaggggtc aaccaggcag gataagcata tattggacca ttgtgaaacc tggagatatc 780
   ctaatgataa acagtaatgg caacttagtt gcaccgcggg gatatttcaa aatgcgaaca 840
   ggaaaaagct ctataatgag atcagatgca cccatagaca cttgtgtgtc cgagtgtatt 900
   acaccaaatg gaagcatccc caacgacaaa ccatttcaaa atgtgaacaa agttacatat 960
   gggaaatgcc ccaagtatat caagcagaat actttgaagc tggccactgg gatgaggaat 1020
   gtaccagaaa agcaaatcag aggaatcttt ggagcaatag cgggattcat agaaaatggc 1080
   tgggag                                                            1086
<210> 48
   <211> 982
   <212> DNA
   <213> Influenza A virus
<400> 48
   atgagtcttc tgaccgaggt cgaaacgtac gttctctcta tcgtaccatc aggccccctc 60
   aaagccgaga tcgcgcagag acttgaagat gtctttgcag gaaagaacac cgatcttgag 120
   gcactcatgg aatggctaaa gacaagacca atcctgtcac ctctgactaa ggggatttta 180
   ggatttgtgt tcacgctcac cgtgcccagt gagcgaggac tgcagcgtag acgctttgtc 240
   caaaatgccc ttaatgggaa cggagatcca aacaacatgg acagagcagt aaaactgtac 300
   aggaagctta aaagggaaat aacattccat ggggcaaaag aggtggcact cagctattcc 360
   actggtgcac tagccagctg catgggactc atatacaaca gaatggggac tgtgacaacc 420
   gaagtggcat ttggcctggt atgcgccaca tgtgaacaga ttgctgattc ccagcaccga 480
   tctcacagac agatggtgac aacaaccaac ccactaatca gacacgagaa cagaatggta 540
   ctagccagta ccacagctaa agccatggag cagatggcag ggtcgagtga gcaggcagca 600
   gaggccatgg aggttgctag tcaggccagg cagatggtgc aggcaatgag aaccattggg 660
   acccacccta gctccagtgc cggtttgaaa aatgatcttc ttgaaaattt gcaggcctac 720
   cagaaacgga tgggagtgca aatgcagcga ttcaagtgat cctctcgtta ttgcagcaag 780
   tatcattggg atcttgcact tgatattgtg gattcttgat cgtcttttct tcaaatgcat 840
   ttatcgtcgt cttaaatacg gtttgaaaag agggccttct acggaaggag tacctgagtc 900
   tatgagggaa gaatatcggc aggaacagca gagtgctgtg gatgttgacg atggtcattt 960
   tgtcaacata gagctggagt aa 982
<210> 49
   <211> 982
   <212> DNA
   <213> Influenza A virus
<400> 49
   atgagtcttc taaccgaggt cgaaacgtac gttctctcta tcgtaccatc aggccccctc 60
   aaagccgaga tcgcgcagag acttgaagat gtctttgcag ggaagaacac cgatcttgag 120
   gcactcatgg aatggctaaa gacaagacca atcctgtcac ctctgactaa agggatttta 180
   ggatttgtgt tcacgctcac cgtgcccagt gagcgaggac tgcagcgtag acgctttgtc 240
   caaaatgccc ttagtggaaa cggagatcca aacaacatgg acagagcagt aaaactgtac 300
   aggaagctta aaagagaaat aacattccat ggggcaaaag aggtggcact cagctattcc 360
   actggtgcac tagccagctg catgggactc atatacaaca gaatggggac tgtgacaacc 420
   gaagtggcat ttggcctggt atgcgccaca tgtgaacaga ttgctgattc ccagcatcga 480
   tctcacaggc agatggtgac aacaaccaac ccactaatca gacatgaaaa cagaatggta 540
   ctagccagta ccacagctaa agccatggag cagatggcag ggtcgagtga gcaggcagca 600
   gaggccatgg aggttgctag taaggccagg cagatggtac aggcaatgag gaccattggg 660
   acccacccta gctccagtgc cggtttgaaa gatgatcttc ttgaaaattt gcaggcctac 720
   cagaaacgga tgggagtgca aatgcagcga ttcaagtgat cctctcgtta ttgcagcaag 780
   tatcattggg atcttgcact tgatattgtg gattcttgat cgccttttct tcaaattcat 840
   ttatcgtcgc cttaaatacg gtttgaaaag agggccttct acggaaggag tacctgagtc 900
   tatgagggaa gaatatcggc aggaacagca gaatgctgtg gatgttgacg atggtcattt 960
   tgtcaacata gagctggagt aa 982
<210> 50
   <211> 838
   <212> DNA
   <213> Influenza A virus
<400> 50
   atggattcca acactgtgtc aagctttcag gtagactgtt ttctttggca tgtccgcaaa 60
   cgatttgcag accaagaact gggtgatgcc ccattccttg accggcttcg ccgagaccag 120
   aagtccctaa aaggaagagg cagcactctt ggtctggaca tcgaaacagc cactcatgca 180
   ggaaagcaga tagtggagcg aattctggaa gaggaatcag atgaggcact taaaatgacc 240
   atagcctctg ttcctacttc acgctactta actgacatga ctcttgatga gatgtcaaga 300
   gactggttca tgctcatgcc caagcagaaa gtaacaggct ccctatgtat aaggatggac 360
   caagcaatca tggataagaa catcatacta aaagcaaact ttagtgtgat tttcgaaagg 420
   ctggagacac taatactact tagagctttc accgaagaag gagcagtcgt tggcgaaatt 480
   tcaccattgc cttctcttcc aggacatact aatgaggatg tcaaaaatgc aattggggtc 540
   ctcatcggag gacttaaatg gaatgataac acagttagag tctctgaaac tctacagaga 600
   ttcgcttgga gaagcagtca tgagaatggg agaccttcat tccctccaaa gcagaaacga 660
   aaaatggcga gaacaattga gtcagaagtt tgaagaaata aggtggttga ttgaagaagt 720
   gagacataga ttgaaaaata cagaaaatag ttttgaacaa ataacattta tgcaagcctt 780
   acaactattg cttgaagtag aacaagagat aagaactttc tcgtttcagc ttatttaa 838
<210> 51
   <211> 838
   <212> DNA
   <213> Influenza A virus
<400> 51
   atggattcca acactgtgtc aagctttcag gtagactgtt ttctttggca tgtccgcaaa 60
   cgatttgcag accaagaact gggtgatgcc ccattccttg accggcttcg ccgagaccag 120
   aagtccctaa aaggaagagg cagcactctt ggtctggaca tcgaaacagc cactcgtgca 180
   ggaaagcaga tagtggagcg gattctggaa gaggagtcag atgaggcact taaaatgacc 240
   attgcctctg ttcctgcttc acgctactta actgacatga ctcttgatga gatgtcaaga 300
   gactggttca tgctcatgcc caagcagaaa gtaacaggct ccctatgtat aaggatggac 360
   caggcaatca tggataagaa catcatacta aaagcaaact ttagtgtgat tttcgaaagg 420
   ctggagacac taatactact tagagccttc accgaagaag gagcagtcgt tggcgaaatt 480
   tcaccattgc cttctcttcc aggacatact aatgaggatg tcaaaaatgc aattggggtc 540
   ctcatcggag gacttaaatg gaatgataat acagttagag tctctgaaac tctacagaga 600
   ttcgcttgga gaagcagtca tgagaatggg agaccttcat tccctccaaa gcagaaacga 660
   aaaatggcga gaacaattga gccagaagtt tgaagaaata agatggttga ttgaagaagt 720
   gcgacataga ttgaaaaata cagaaaatag ttttgaacaa ataacattta tgcaagcctt 780
   acaactattg cttgaagtag aacaagagat aagaactttc tcgtttcagc ttatttaa   838

## Description

This application claims priority to US patent application Serial No. 12/020,656 filed January 28, 2008.

### FIELD OF THE INVENTION

The present invention relates to a *Canidae* vaccine for use in reducing influenza viral shedding in a canine.

### BACKGROUND OF THE INVENTION

Respiratory diseases resembling influenza have infected thousands of dogs in the U.S. Recurrent outbreaks of severe respiratory disease characterized by coughing and fever have occurred in greyhounds at racing kennels. Pathological findings included severe pulmonary and plural hemorrhage, accurate to subacute erosive to hyperplastic tracheitis, bronchitis and bronchiolitis and bronchopneumonia. In 2004, eight greyhounds in Jacksonville, Florida were killed by an equine influenza virus that jumped the species barrier from horses to dogs.

Equine influenza is a disease of horses, and the virus is in the same group of viruses that cause flu in people. The disease is present in horse populations throughout Europe, North America and parts of Asia, with horses typically developing a fever and a dry hacking cough. In the early stages of the disease, horses are reluctant to eat or drink for several days, but usually recover in two to three weeks.

H3N8 subtypes of equine influenza were previously isolated from lungs of two dogs from Florida and one dog fromTexas who had died from the infection. Genetic sequence analyses and phylogenetic comparisons determined that all three canine isolates were closely related and evolved from contemporary strains of equine influenza H3N8.
Immunohistochemistry demonstrated influenza antigen in bronchial gland epithelial cells, bronchial and bronchiolar epithelial cells and in alveolar macrophages. Seroconversion to canine influenza virus was demonstrated by hemagglutination inhibition and microneutralization assays.

Molecular and antigenic analyses of three influenza viruses isolated from outbreaks of severe respiratory disease in racing greyhounds revealed that they are closely related to H3N8 equine influenza virus (see, e.g., Crawford et al., Science. 2005 Oct 21;310(5747):482-5. Epub 2005 Sep 26). Phylogenetic analysis indicated that the canine influenza virus genomes form a monophyletic group, consistent with a single interspecies virus transfer. Molecular changes in the hemagglutinin suggested adaptive evolution in the new host. The etiologic role of this virus in respiratory disease was supported by the temporal association of rising antibody titers with disease and by experimental inoculation studies. The geographic expansion of the infection and its persistence for several years indicate efficient transmission of canine influenza virus among greyhounds. Evidence of infection in pet dogs suggests that this infection may also become enzootic in this population.

In 2005, a mutated form of the Bird Flu (H3N8 Virus) was reported to have killed greyhounds in Massachusetts.

The document WO2007024947 describes a method of eliciting an immune response against influenza virus in a canine, comprising administering a formulation comprising an avipox expression vector comprising a polynucleotide encoding an influenza virus antigen and a pharmaceutically or veterinarily acceptable carrier, excipient or vehicle in an effective amount for eliciting an immune response.

The document by Karaca K et al (2007), American Journal of Veterinary Research, vol.68, pp.208-212 describes an evaluation of the ability of canarypox-vectored equine influenza virus vaccines to induce humoral immune responses against canine influenza viruses in dogs.

The document WO2007047938 describes administration to dogs of a cold-adapted equine/Kentucky/91 influenza virus.

Accordingly, there is a need for an effective vaccine against influenza in canines.

### SUMMARY OF THE INVENTION

The invention is as defined in the claims.

In one aspect, the present invention is a Canidae vaccine for use in reducing influenza viral shedding in a canine, wherein said vaccine comprises an effective amount of an expression vector, wherein the expression vector is a poxvirus, that contains and expresses in vivo in a canine a polynucleotide encoding equine influenza antigen comprising equine influenza H3 operatively linked to a promoter and optionally to an enhancer, and a pharmaceutically or veterinarily acceptable carrier, excipient, or vehicle.

The canine influenza vaccine described herein is a recombinant vaccine, advantageously, the vector is an avipox expression vector which may comprise a polynucleotide encoding an influenza antigen, epitope or immunogen. The influenza antigen, epitope or immunogen may be a hemagglutinin, matrix protein, neuraminidase, nonstructural protein, nucleoprotein, polymerase or any fragment thereof. An expression vector for use according to the invention is a poxvirus vector containing and expressing in vivo in a canine a polynucleotide encoding equine influenza antigen comprising equine influenza H3 operatively linked to a promoter.

As described herein, the canine influenza antigen, epitope or immunogen is derived from a canine infected with influenza. For example, influenza virus may be isolated from the broncho alveolar lavage and/or lung tissues of an affected dog. Isolation and characterization of the nucleotide sequence of the influenza virus infecting the dog may be done by routine experimentation by a person of ordinary skill in the art.

As described
herein, the canine influenza antigen, epitope or immunogen may be isolated from an equine influenza. The equine influenza may be an Ohio equine influenza, Kentucky equine influenza or a Newmarket equine influenza.

The avipox expression vector may be an attenuated avipox expression vector. In one embodiment, the avipox expression vector may be a canarypox vector, advantageously ALVAC, as recited in the claims.

The influenza antigen, epitope or immunogen may be a hemagglutinin, such as H3. The canarypox vector may be CP 2242, CP1529 or CP1533 or expressing the H3 of a canine influenza isolate vCP2328)

The present disclosure
also encompasses an inactivated influenza vaccine. The inactivated influenza vaccine desribed herein may be an inactivated canine influenza. The inactivated influenza vaccine further desribed herein may be an equine influenza, advantageously an Ohio equine influenza, Kentucky equine influenza or a Newmarket equine influenza. The vaccine may be inactivated with formalin or beta-propiolactone.

Described herein is a method of eliciting an immune response against influenza in a canine, which may comprise administering a formulation comprising any one of the above recombinant influenza vaccine or inactivated vaccine and a pharmaceutically or veterinarily acceptable carrier, excipient or vehicle in an effective amount for eliciting an immune response.
In an advantageous embodiment, an adjuvant may be added. The adjuvant may be aluminum hydroxide, alumimum phosphate, a carbomer or an oil-water-emulsion and optionally may comprise CpG. Advantageously, the vaccine is formulated so the administration may be subcutaneous or intramuscular.

Further described herein is a method of inducing an immune response against influenza in a canine, which may comprise administering a formulation comprising any one of the above recombinant influenza vaccine or inactivated vaccine and a pharmaceutically or veterinarily acceptable carrier, excipient or vehicle in an effective amount for inducing an immune response. In an advantageous embodiment, an adjuvant may be added. The adjuvant may be aluminum hydroxide, alumimum phosphate, a carbomer or an oil-water-emulsion, immunostimulating complex (ISCOM), and optionally may comprise CpG. Advantageously, the vaccine is formulated so administration may be subcutaneous or intramuscular.

Still further described herein is a kit for performing a method of eliciting or inducing an immune response which may comprise any one of the recombinant influenza vaccines or inactivated vaccines and instructions for performing the method.

It is noted that in this disclosure and particularly in the claims and/or paragraphs, terms such as "comprises", "comprised", "comprising" can mean "includes", "included", "including".

### BRIEF DESCRIPTION OF THE DRAWINGS

The following detailed description may best be understood in conjunction with the accompanying drawings, in which:
FIG. 1 illustrates the sequence of the insert in PJT004 (SEQ ID NO: 1);
FIG. 2 illustrates the sequence of the insert in PJT005 (SEQ ID NO: 2);
FIG. 3 illustrates a comparison of the amino acid sequence of EIV Ohio 03 strain HA to that of New Market strain H3 HA (SEQ ID NOS: 3 and 4);
FIG. 4A illustrates a construction of an ALVAC donor plasmid for generation of an ALVAC recombinant expressing codon optimized EIV H3 HA (Ohio 03);
FIG. 4B illustrates pALVAC C5 H6p-synthetic EIV H3 HA, pJY1571.1;
FIG. 5A illustrates a predicted amino acid sequence of product(s): EIV H3 HA;
FIG. 5B illustrates a nucleotide sequence of arms and insert with translation
FIG. 6 illustrates a vCP2242 Western blot analysis. A 1/1,000 dilution of pooled anti-EIV antibody was used for the analysis. Lane 1: 5 µl Fermentas Prestain protein marker, lane 2: 15 µl ALVAC cell pellet, lane 3: 15 µl vCP2242. cell pellet, lane 4: 15 µl vCP2242. cell pellet, lane 5: 15 µl vCP1533 cell pellet, lane 6: space, lane 7: 40 µl ALVAC supernatant, lane 8: 40 µl vCP2242. supernatant, lane 9: 40 µl vCP2242. supernatant and lane 10: 40 µl vCP1533 supernatant.
FIG. 7 illustrates the HI antibody responses to canine influenza virus (CIV) in dogs vaccinated with canarypox-vectored equine influenza virus (EIV) vaccines expressing either the hemagglutinin gene of A/equine/Kentucky/94 (vCP1529) or the hemagglutin gene of A2/equine/Ohio/03 (vCP2242). vCP2242 was administered by the subcutaneous (SC in FIG. 7) or the transdermal route (TD in FIG. 7) whereas vCP1529 was administered subcutaneously (SC in FIG. 7).
FIG. 8 illustrates the mean viral titers found in nasal swabs in control animals and vaccinated animals following intranasal challenge with influenza A/canine/NY/110659. Data = log10 virus isolation titers on MDCK cells.
FIG. 9 illustrates the mean body temperatures of control dogs and of dogs vaccinated subcutaneously twice at a 21-day interval with the hemagglutin gene of A2/equine/Ohio/03 (vCP2242) followed by challenge at day 43 with CIV-NY05 isolate (influenza A/canine/NY /110659).
FIG. 10 illustrates the mean body temperatures of control dogs (CON) and of dogs vaccinated subcutaneously twice at a 21-day interval with the hemagglutin gene of A2/equine/Ohio/03 (vCP2242) (VAC) followed by challenge at day 43 with CIV-NY05 isolate on days 42, 44, and 45. This figure is a box plot that has the mean (symbolized as a +) and the median (horizontal line), the 25^{th} and 75^{th} percentile (bottom and top of the box), minimum and maximum observations (whiskers), and outliers (small squares).
FIG. 11 illustrates the HI antibody responses to CIV HA antigen of A2/equine/Ohio/03 (vCP2242) followed by challenge at day 43 with CIV-NY05 isolate at three weeks post second vaccination and two weeks post challenge (Vac57), and in control dogs at two weeks post challenge (Cont57).
FIG. 12 illustrates the protective immune response of vCP2242 as characterized by the decreased time of virus shedding in the nasal cavity of dogs vaccinated subcutaneously twice at a 21-day interval with the hemagglutin gene of A2/equine/Ohio/03 (vCP2242) followed by challenge at day 43 with CIV-NY05 isolate as compared to control dogs.
FIG. 13 illustrates the the protective immune response of vCP2242 as characterized by the decreased duration of virus shedding in the nasal cavity of dogs vaccinated subcutaneously twice at a 21-day interval with the hemagglutin gene of A2/equine/Ohio/03 (vCP2242) followed by challenge at day 43 with CIV-NY05 isolate as compared to control dogs.

### DETAILED DESCRIPTION

The invention is as defined in the claims.

The present invention is based, in part, on Applicants' studies demonstrating a recombinant canarypox expressing equine influenza HA is immunogenic in dogs.

As described herein is any influenza antigen, epitope or immunogen that elicits an immunogenic response in an animal, advantageously a vertebrate, more advantageously a dog. The influenza antigen, epitope or immunogen described herein may be any influenza antigen, epitope or immunogen, such as a protein, peptide or fragment thereof, that elicits, induces or stimulates a response in an animal, advantageously a vertebrate, more advantageously a dog.

In an advantageous embodiment, the equine influenza H3 antigen, epitope or immunogen is derived from a canine infected with influenza. For example, influenza virus may be isolated from the broncho alveolar lavage and/or lung tissues of an affected dog. Isolation and characterization of the nucleotide sequence of the influenza infecting the dog may be done by routine experimentation by a person of ordinary skill in the art.

As also advantageously described herein, the equine influenza H3 antigen, epitope or immunogen may be derived from an equine infected with influenza or an equine influenza strain. Advantageously, the equine influenza strain is an Ohio equine influenza, Kentucky equine influenza strain or a Newmarket equine influenza strain. As described herein, the equine influenza H3 antigen, epitope or immunogen may be determined by one of ordinary skill of the art from the nucleotide sequences of Examples 4 and 5. Advantageously, the equine influenza antigen, epitope or immunogen is a hemagglutinin (HA) (e.g., HA precursor, HI, H2, protein, matrix protein (e.g., matrix protein M1 or M2), neuraminidase, nonstructural (NS) protein (e.g., NS1 or NS2), nucleoprotein (NP) and polymerase (e.g., PA polymerase, PB1 polymerase 1 or PB2 polymerase 2).

Examples of Kentucky equine influenza strains that may be used in methods described herein include, equine influenza strains A/eq/Kentucky/98 (see, e.g., Crouch et al., Vaccine. 2004 Dec 2;23(3):418-25), A/Equi 2 (Kentucky 81) (see, e.g., Short et al., J Vet Pharmacol Ther. 1986 Dec;9(4):426-32, Horner & Ledgard, N Z Vet J. 1988 Dec;36(4):205-6), A/equine/Kentucky/1/81 (Eq/Ky) (see, e.g., Breathnach et al., Vet Immunol Immunopathol. 2004 Apr;98(3-4):127-36), A/Equine/Kentucky/1/81 (H3N8) (see, e.g., Olsen et al., Vaccine. 1997 Jul;15(10):1149-56, Morley et al. Vet Microbiol. 1995 Jun;45(1):81-92, Ozaki et al., Vet Microbiol. 2001 Sep 20;82(2):111-9, Sugiura et al., J Virol Methods. 2001 Oct;98(1):1-8, see, e.g., Sugiura et al., J Virol Methods. 2001 Oct;98(1):1-8, Goto et al., J Vet Med Sci. 1993 Feb;55(1):33-7, Goto et al., J Vet Med Sci. 1993 Feb;55(1):33-7), A/Equine/Kentucky/1/91 (H3N8) (see, e.g., Youngner et al., Am J Vet Res. 2001 Aug;62(8):1290-4), A/Equine/Kentucky/1277/90 (Eq/Kentucky) (see, e.g., Webster & Thomas, Vaccine. 1993;11(10):987-93), A/Equine/Kentucky/2/91 (H3N8) (see, e.g., Donofrio et al., J Vet Diagn Invest. 1994 Jan;6(1):39-43), A/Equine/Kentucky/79 (H3N8) (see, e.g., Donofrio et al., J Vet Diagn Invest. 1994 Jan;6(1):39-43), A/equine/Kentucky/81 (see, e.g., Sugiura et al., J Virol Methods. 2001 Oct;98(1):1-8), A/equine/Kentucky/91 (H3N8) (see, e.g., Gross et al., Equine Vet J. 1998 Nov;30(6):489-97), A/equine-2/Kentucky/95 (H3N8) (see, e.g., Heldens et al., Vet J. 2004 Mar;167(2):150-7) and A/equine-2/Kentucky/98 (see, e.g., Chambers et al., Equine Vet J. 2001 Nov;33(7):630-6).

Examples of Newmarket equine influenza strains that may be used in methods described herein include, equine influenza strains A/eq/Newmarket/1/77 (see, e.g., Lindstrom et al., Arch Virol. 1998;143(8):1585-98), A/eq/Newmarket/5/03 (see, e.g., Edlund Toulemonde et al., Vet Rec. 2005 Mar 19;156(12):367-71), A/Equi 2 (H3N8), Newmarket 1/93 (see, e.g., Mohler et al., Biotechnol Bioeng. 2005 Apr 5;90(1):46-58, Nayak et al., J Chromatogr B Analyt Technol Biomed Life Sci. 2005 Jul 8), A/equi-2/Newmarket-1/93 (see, e.g., Heldens et al., J Immunol Methods. 2002 Jun 1;264(1-2):11-7), A/equine/Newmarket/2/93 (see, e.g., Wattrang et al., Viral Immunol. 2003;16(1):57-67), A/equine/Newmarket/79 (H3N8) (see, e.g., Duhaut & Dimmock, Virology. 2000 Sep 30;275(2):278-85, Noble & Dimmock, J Gen Virol. 1994 Dec;75 (Pt 12):3485-91, Duhaut & Dimmock, Virology. 1998 Sep 1;248(2):241-53, Hannant & Mumford, Vet Immunol Immunopathol. 1989 Jul;21(3-4):327-37, Hannant et al., Vet Microbiol. 1989 Apr;19(4):293-303, Hannant et al., Vet Rec. 1988 Feb 6;122(6): 125-8, Richards et al., Vet Immunol Immunopathol. 1992 Jun;33(1-2):129-43, Heldens et al., Vet J. 2004 Mar;167(2):150-7), A/equine/Newmarket/1/77 (H7N7) (see, e.g., Goto et al., J Vet Med Sci. 1993 Feb;55(1):33-7, Sugiura et al., J Virol Methods. 2001 Oct;98(1):1-8, Sugiura et al., J Virol Methods. 2001 Oct;98(1):1-8) and A/equine-2/Newmarket-2/93 (see, e.g., Heldens et al., Vet J. 2004 Mar; 167(2): 150-7).

Described herein are other equine influenza viruses that may be used, such asequine influenza virus A/eq/Miami/63 (H3N8) (see, e.g., van Maanen et al., Vet Microbiol. 2003 Jun 10;93(4):291-306), A/equi 1 (Prague strain) (see, e.g., Horner & Ledgard, N Z Vet J. 1988 Dec;36(4):205-6, Short et al., J Vet Pharmacol Ther. 1986 Dec;9(4):426-32), A/Equi 2 (Miami) (see, e.g., Short et al., J Vet Pharmacol Ther. 1986 Dec;9(4):426-32), A/equi-1/Prague/56 (Pr/56) (see, e.g., Heldens et al., J Immunol Methods. 2002 Jun 1;264(1-2):11-7), A/equi-2/Suffolk/89 (Suf/89) (see, e.g., Heldens et al., J Immunol Methods. 2002 Jun 1;264(1-2):11-7), A/Equine 2/Sussex/89 (H3N8) (see, e.g., Mumford et al., Vet Rec. 1994 Feb 12;134(7):158-62), A/equine/Sussex/89 (see, e.g., Wattrang et al., Viral Immunol. 2003;16(1):57-67), A/equine-2/Saskatoon/90 (see, e.g., Chambers et al., Equine Vet J. 2001 Nov;33(7):630-6), A/Equine/Prague/1/56 (H7N7) (see, e.g., Donofrio et al., J Vet Diagn Invest. 1994 Jan;6(1):39-43, Morley et al. Vet Microbiol. 1995 Jun;45(1):81-92),, A/equine/Miami/1/63 (H3N8) (see, e.g., Morley et al. Vet Microbiol. 1995 Jun;45(1):81-92, Ozaki et al., Vet Microbiol. 2001 Sep 20;82(2):111-9, Thomson et al., Vet Rec. 1977 May 28;100(22):465-8, Mumford et al., Epidemiol Infect. 1988 Jun;100(3):501-10, Donofrio et al., J Vet Diagn Invest. 1994 Jan;6(1):39-43, Mumford et al., J Hyg (Lond). 1983 Jun;90(3):385-95), A/Aichi/2/68 (H3N2) (see, e.g., Ozaki et al., Vet Microbiol. 2001 Sep 20;82(2):111-9), A/equine/Tokyo/2/71 (H3N8) (see, e.g., Goto et al., J Vet Med Sci. 1993 Feb;55(1):33-7), A/eq/LaPlata/1/88 (see, e.g., Lindstrom et al., Arch Virol. 1998;143(8):1585-98), A/Equine/Jilin/1/89 (Eq/Jilin) (see, e.g., Webster & Thomas, Vaccine. 1993;11(10):987-93), A/Equine/Alaska/1/91 (H3N8) (see, e.g., Webster & Thomas, Vaccine. 1993;11(10):987-93), A/equine/Saskatoon/1/91 (H3N8) (see, e.g., Morley et al. Vet Microbiol. 1995 Jun;45(1):81-92), A/equine/Rome/5/91 (H3N8) (see, e.g., Sugiura et al., J Virol Methods. 2001 Oct;98(1):1-8), A/equine/La Plata/1/93 (H3N8) (see, e.g., Ozaki et al., Vet Microbiol. 2001 Sep 20;82(2):111-9), A/equine/La Plata/1/93 (LP/93) (see, e.g., Sugiura et al., J Virol Methods. 2001 Oct;98(1):1-8), A/eq/Holland/1/95 (H3N8) (see, e.g., van Maanen et al., Vet Microbiol. 2003 Jun 10;93(4):291-306) and A/eq/Holland/2/95 (H3N8) (see, e.g., van Maanen et al., Vet Microbiol. 2003 Jun 10;93(4):291-306).

In another advantageous embodiment the equine influenza H3 antigen, epitope or immunogen may be derived from an equine infected with influenza or an equine influenza strain derived from a recent isolate.

As described herein, the influenza antigen, epitope or immunogen may also be isolated from any influenza strain such as avian H5N1 influenza virus, A/Hong Kong/156/97 (A/HK/156/97) (see, e.g., Leneva et al., Antimicrob Agents Chemother. 2001 Apr;45(4):1216-24), avian H7N1 influenza strain (see, e.g., Foni et al., New Microbiol. 2005 Jan;28(1):31-5), avian H9N2 influenza virus (see, e.g., Leneva et al., Antimicrob Agents Chemother. 2001 Apr;45(4):1216-24), avian influenza virus A/Chicken/HK/G9/97 (H9N2) (see, e.g., Leneva et al., Antimicrob Agents Chemother. 2001 Apr;45(4): 1216-24), avian influenza virus A/Quail/HK/G1/97 (H9N2) (see, e.g., Leneva et al., Antimicrob Agents Chemother. 2001 Apr;45(4):1216-24), avian influenza virus A/Teal/HK/W312/97 (H6N1) (see, e.g., Leneva et al., Antimicrob Agents Chemother. 2001 Apr;45(4): 1216-24), avian pandemic influenza A viruses of avian origin (see, e.g., Audsley & Tannock, Expert Opin Biol Ther. 2004 May;4(5):709-17), cold-adapted (ca) and temperature sensitive (ts) master donor strain, A/Leningrad/134/17/57 (H2N2) (see, e.g., Youil et al., Virus Res. 2004 Jun 15;102(2):165-76), equine influenza virus (A/Equi 2 (H3N8), Newmarket 1/93) (see, e.g., Mohler et al., Biotechnol Bioeng. 2005 Apr 5;90(1):46-58; Nayak et al., J Chromatogr B Analyt Technol Biomed Life Sci. 2005 Jul 8), equine-2 influenza virus (EIV; subtype H3N8) (see, e.g., Virology. 2001 Aug 15;287(1):202-13), equine-2 influenza virus, A/Equine/Kentucky/1/91 (H3N8) (see, e.g., Youngner et al., Am J Vet Res. 2001 Aug;62(8):1290-4), human influenza virus A(H3N2) isolates (see, e.g., Abed et al., J Infect Dis. 2002 Oct 15;186(8):1074-80), human influenza virus A/Memphis/1/71 (H3N2) (see, e.g., Suzuki et al., Biochem J. 1996 Sep 1;318 (Pt 2):389-93), human influenza virus A/Nanchang/933/95 (H3N2) virus (see, e.g., Scholtissek et al., J Virol. 2002 Feb;76(4):1781-6), human influenza virus A/PR/8/34 (H1N1) virus (see, e.g., Scholtissek et al., J Virol. 2002 Feb;76(4):1781-6), human influenza virus A/Singapore/57 (H2N2) virus (see, e.g., Scholtissek et al., J Virol. 2002 Feb;76(4):1781-6), influenza virus A (see, e.g., Chare et al., J Gen Virol. 2003 Oct;84(Pt 10):2691-703), influenza virus A PR/8/34 (PR8) virus (H1N1 subtype) (see, e.g., Mantani et al., Planta Med. 2001 Apr;67(3):240-3), influenza virus A/Aichi/2/68(H3N2) (see, e.g., Miyamoto et al., Antiviral Res. 1998 Aug;39(2):89-100), influenza virus A/Ann Arbor/6/60 cold-adapted virus (see, e.g., Treanor et al., J Virol. 1994 Dec;68(12):7684-8), influenza virus A/Beijing 32/92 (H3N2) (see, e.g., Zakay-Rones et al., J Altern Complement Med. 1995 Winter;1(4):361-9), influenza virus A/Charlottesville/31/95 (H1N1) (see, e.g., Gubareva et al., J Gen Virol. 2002 Nov;83(Pt 11):2683-92), influenza virus A/Kawasaki/86 (H1N1) virus (see, e.g., Staschke et al., Virology. 1998 Sep 1;248(2):264-74), influenza virus A/Korea/82 (H3N2) (see, e.g., Treanor et al., J Virol. 1994 Dec;68(12):7684-8), influenza virus A/Leningrad/134/57 (see, e.g., Egorov et al., J Virol. 1998 Aug;72(8):6437-41), influenza virus A/NWS/33 (H1N1) (see, e.g., Sidwell et al., Antiviral Res. 1998 Feb;37(2):107-20), influenza virus A/PR/8/34(H1N1) (see, e.g., Miyamoto et al., Antiviral Res. 1998 Aug;39(2):89-100), influenza virus A/PR8/34 (see, e.g., Nunes-Correia et al., Biochemistry. 1999 Jan 19;38(3):1095-101, Tree et al., Vaccine. 2001 May 14;19(25-26):3444-50), influenza virus A/Puerto Rico (PR)/8/34 (see, e.g., Egorov et al., J Virol. 1998 Aug;72(8):6437-41), influenza virus A/Puerto Rico/8-Mount Sinai (see, e.g., Mazanec et al., J Virol. 1995 Feb;69(2):1339-43), influenza virus A/Shangdong 9/93 (H3N2) (see, e.g., Zakay-Rones et al., J Altern Complement Med. 1995 Winter; 1(4):361-9, Sidwell et al., Antiviral Res. 1998 Feb;37(2):107-20), influenza virus A/Shingapol/1/57(H2N2) (see, e.g., Miyamoto et al., Antiviral Res. 1998 Aug;39(2):89-100), influenza virus A/Singapore 6/86 (H1N1) (see, e.g., Zakay-Rones et al., J Altern Complement Med. 1995 Winter;1(4):361-9), influenza virus A/Singapore/1/57 (H2N2) (see, e.g., Bantia et al., Antimicrob Agents Chemother. 1998 Apr;42(4):801-7), influenza virus A/Texas 36/91 (H1N1) (see, e.g., Zakay-Rones et al., J Altern Complement Med. 1995 Winter; 1(4):361-9), influenza virus A/Texas/36/91 (H1N1) virus (see, e.g., Gubareva et al., J Infect Dis. 2001 Feb 15;183(4):523-31, Halperin et al., Vaccine. 1998 Aug;16(13):1331-5), influenza virus A/Texas/36/91(H1N1) (see, e.g., Hayden et al., Antiviral Res. 1994 Oct;25(2):123-31), influenza virus A/Udorn/72 virus infection (see, e.g., Shimizu et al., Virology. 1999 Feb 15;254(2):213-9), influenza virus A/Victoria/3/75 (H3N2) (see, e.g., Sidwell et al., Antiviral Res. 1998 Feb;37(2):107-20), influenza virus A/Virginia/88(H3N2) (see, e.g., Hayden et al., Antiviral Res. 1994 Oct;25(2):123-31), influenza virus A/WSN/33 (H1N1) (see, e.g., Lu et al., Arch Virol. 2002;147(2):273-84), influenza virus A/WSN/33 (see, e.g., Gujuluva et al., Virology. 1994 Nov 1;204(2):491-505), influenza virus B (see, e.g., Chare et al., J Gen Virol. 2003 Oct;84(Pt 10):2691-703), influenza virus B/Ann Arbor 1/86 (see, e.g., Zakay-Rones et al., J Altern Complement Med. 1995 Winter;1(4):361-9), influenza virus B/Harbin/7/94 (see, e.g., Halperin et al., Vaccine. 1998 Aug;16(13):1331-5, influenza virus B/Hong Kong/5/72 (see, e.g., Sidwell et al., Antiviral Res. 1998 Feb;37(2):107-20), influenza virus B/Lee/40 (see, e.g., Miyamoto et al., Antiviral Res. 1998 Aug;39(2):89-100), influenza virus B/Victoria group (see, e.g., Nakagawa et al., J Virol Methods. 1999 Apr;79(1):113-20), influenza virus B/Yamagata 16/88 (see, e.g., Zakay-Rones et al., J Altern Complement Med. 1995 Winter;1(4):361-9), influenza virus B/Yamagata group (see, e.g., Nakagawa et al., J Virol Methods. 1999 Apr;79(1):113-20), influenza virus B/Yamanashi/166/98 (see, e.g., Hoffmann et al., Proc Natl Acad Sci USA. 2002 Aug 20;99(17):11411-6), influenza virus C (see, e.g., Chare et al., J Gen Virol. 2003 Oct;84(Pt 10):2691-703), influenza virus strain A/Equi/2/Kildare/89 (see, e.g., Quinlivan et al., J Clin Microbiol. 2004 Feb;42(2):759-63), influenza virus type B/Panama 45/90 (see, e.g., Zakay-Rones et al., J Altern Complement Med. 1995 Winter; 1(4):361-9), live, cold-adapted, temperature-sensitive (ca/ts) Russian influenza A vaccines (see, e.g., Palker et al., Virus Res. 2004 Oct;105(2):183-94), Madin Darby Canine Kidney (MDCK)-derived cell line (see, e.g., Halperin et al., Vaccine. 2002 Jan 15;20(7-8):1240-7), mouse-adapted influenza virus A/Guizhou/54/89 (H3N2 subtype) (see, e.g., Nagai et al., Biol Pharm Bull. 1995 Feb;18(2):295-9), mouse-adapted influenza virus A/PR/8/34 (A/PR8) (see, e.g., Nagai et al., Antiviral Res. 1995 Jan;26(1):11-25), mouse-adapted influenza virus B/Ibaraki/2/85 (see, e.g., Nagai et al., Biol Pharm Bull. 1995 Feb;18(2):295-9), Russian live attenuated influenza vaccine donor strains A/Leningrad/134/17/57, A/Leningrad/134/47/57 and B/USSR/60/69 (see, e.g., Audsley & Tannock, J Virol Methods. 2005 Feb;123(2):187-93), swine H1 and H3 influenza viruses (see, e.g., Gambaryan et al., Virus Res. 2005 Jul 1), swine influenza A viruses (see, e.g., Landolt et al., Am J Vet Res. 2005 Jan;66(1):119-24), swine influenza virus (SIV) (see, e.g., Clavijo et al., Can J Vet Res. 2002 Apr;66(2): 117-21), swine influenza virus A/Sw/Ger 2/81 (see, e.g., Zakay-Rones et al., J Altern Complement Med. 1995 Winter;1(4):361-9), swine influenza virus A/Sw/Ger 8533/91 (see, e.g., Zakay-Rones et al., J Altern Complement Med. 1995 Winter;1(4):361-9), turkey influenza virus A/Tur/Ger 3/91 (see, e.g., Zakay-Rones et al., J Altern Complement Med. 1995 Winter;1(4):361-9) and turkey influenza virus A/turkey/Minnesota/833/80 (H4N2) (see, e.g., Gubareva et al., J Virol. 1997 May;71(5):3385-90).

As used herein, the term "antigen" or "immunogen" means a substance that induces a specific immune response in a host animal. The antigen may comprise a whole organism, killed, attenuated or live; a subunit or portion of an organism; a recombinant vector containing an insert with immunogenic properties; a piece or fragment of DNA capable of inducing an immune response upon presentation to a host animal; a protein, a polypeptide, a peptide, an epitope, a hapten, or any combination thereof. Alternately, the immunogen or antigen may comprise a toxin or antitoxin.

The term "immunogenic protein or peptide" as used herein also refers includes peptides and polypeptides that are immunologically active in the sense that once administered to the host, it is able to evoke an immune response of the humoral and/or cellular type directed against the protein. Preferably the protein fragment is such that it has substantially the same immunological activity as the total protein. Thus, a protein fragment according to the invention comprises or consists essentially of or consists of at least one epitope or antigenic determinant. The term epitope relates to a protein site able to induce an immune reaction of the humoral type (B cells) and/or cellular type (T cells).

The term "immunogenic protein or peptide" further contemplates deletions, additions and substitutions to the sequence, so long as the polypeptide functions to produce an immunological response as defined herein. In this regard, particularly preferred substitutions will generally be conservative in nature, *i.e.,* those substitutions that take place within a family of amino acids. For example, amino acids are generally divided into four families: (1) acidic--aspartate and glutamate; (2) basic--lysine, arginine, histidine; (3) non-polar--alanine, valine, leucine, isoleucine, proline, phenylalanine, methionine, tryptophan; and (4) uncharged polar--glycine, asparagine, glutamine, cystine, serine threonine, tyrosine. Phenylalanine, tryptophan, and tyrosine are sometimes classified as aromatic amino acids. It is reasonably predictable that an isolated replacement of leucine with isoleucine or valine, or vice versa; an aspartate with a glutamate or vice versa; a threonine with a serine or vice versa; or a similar conservative replacement of an amino acid with a structurally related amino acid, will not have a major effect on the biological activity. Proteins having substantially the same amino acid sequence as the reference molecule but possessing minor amino acid substitutions that do not substantially affect the immunogenicity of the protein are, therefore, within the definition of the reference polypeptide.

The term "epitope" refers to the site on an antigen or hapten to which specific B cells and/or T cells respond. The term is also used interchangeably with "antigenic determinant" or "antigenic determinant site". Antibodies that recognize the same epitope can be identified in a simple immunoassay showing the ability of one antibody to block the binding of another antibody to a target antigen.

An "immunological response" to a composition or vaccine is the development in the host of a cellular and/or antibody-mediated immune response to a composition or vaccine of interest. Usually, an "immunological response" includes but is not limited to one or more of the following effects: the production of antibodies, B cells, helper T cells, and/or cytotoxic T cells, directed specifically to an antigen or antigens included in the composition or vaccine of interest. Preferably, the host will display either a therapeutic or protective immunological response such that resistance to new infection will be enhanced and/or the clinical severity of the disease reduced. Such protection will be demonstrated by either a reduction or lack of symptoms normally displayed by an infected host, a quicker recovery time and/or a lowered viral titer in the infected host.

The terms "immunogenic" protein or polypeptide as used herein also refers to an amino acid sequence which elicits an immunological response as described above. An "immunogenic" protein or polypeptide, as used herein, includes the full-length sequence of the protein, analogs thereof, or immunogenic fragments thereof. By "immunogenic fragment" is meant a fragment of a protein which includes one or more epitopes and thus elicits the immunological response described above. Such fragments can be identified using any number of epitope mapping techniques, well known in the art. See, *e.g.,* Epitope Mapping Protocols in Methods in Molecular Biology, Vol. 66 (Glenn E. Morris, Ed., 1996) Humana Press, Totowa, N.J. For example, linear epitopes may be determined by *e.g.,* concurrently synthesizing large numbers of peptides on solid supports, the peptides corresponding to portions of the protein molecule, and reacting the peptides with antibodies while the peptides are still attached to the supports. Such techniques are known in the art and described in, *e.g.,* U.S. Pat. No. 4,708,871; Geysen et al. (1984) Proc. Natl. Acad. Sci. USA 81:3998-4002; Geysen et al. (1986) Molec. Immunol. 23:709-715. Similarly, conformational epitopes are readily identified by determining spatial conformation of amino acids such as by, *e.g.,* x-ray crystallography and 2-dimensional nuclear magnetic resonance. See, *e.g.,* Epitope Mapping Protocols, *supra.* Methods especially applicable to the proteins of *T. parva* are fully described in the PCT Application Serial No. PCT/US2004/022605.

Synthetic antigens are also included within the definition, for example, polyepitopes, flanking epitopes, and other recombinant or synthetically derived antigens. See, *e.g.,* Bergmann et al. (1993) Eur. J. Immunol. 23:2777-2781; Bergmann et al. (1996) J. Immunol. 157:3242-3249; Suhrbier, A. (1997) Immunol. and Cell Biol. 75:402-408; Gardner et al. (1998) 12th World AIDS Conference, Geneva, Switzerland, Jun. 28-Jul. 3, 1998. Immunogenic fragments, for purposes of the present invention, will usually include at least about 3 amino acids, preferably at least about 5 amino acids, more preferably at least about 10-15 amino acids, and most preferably 25 or more amino acids, of the molecule. There is no critical upper limit to the length of the fragment, which could comprise nearly the full-length of the protein sequence, or even a fusion protein comprising at least one epitope of the protein.

Accordingly, a minimum structure of a polynucleotide expressing an epitope is that it comprises or consists essentially of or consists of nucleotides to encode an epitope or antigenic determinant of an influenza protein or polyprotein. A polynucleotide encoding a fragment of the total protein or polyprotein, more advantageously, comprises or consists essentially of or consists of a minimum of 21 nucleotides, advantageously at least 42 nucleotides, and preferably at least 57, 87 or 150 consecutive or contiguous nucleotides of the sequence encoding the total protein or polyprotein. Epitope determination procedures, such as, generating overlapping peptide libraries (Hemmer B. et al., Immunology Today, 1998, 19 (4), 163-168), Pepscan (Geysen et al., (1984) Proc. Nat. Acad. Sci. USA, 81, 3998-4002; Geysen et al., (1985) Proc. Nat. Acad. Sci. USA, 82, 178-182; Van der Zee R. et al., (1989) Eur. J. Immunol., 19, 43-47; Geysen H.M., (1990) Southeast Asian J. Trop. Med. Public Health, 21, 523-533; Multipin.RTM. Peptide Synthesis Kits de Chiron) and algorithms (De Groot A. et al., (1999) Nature Biotechnology, 17, 533-561), and in PCT Application Serial No. PCT/US2004/022605, can be used in the practice of the invention, without undue experimentation. Other documents cited may also be consulted for methods for determining epitopes of an immunogen or antigen and thus nucleic acid molecules that encode such epitopes.

A "polynucleotide" is a polymeric form of nucleotides of any length, which contain deoxyribonucleotides, ribonucleotides, and analogs in any combination. Polynucleotides may have three-dimensional structure, and may perform any function, known or unknown. The term "polynucleotide" includes double-, single-stranded, and triple-helical molecules. Unless otherwise specified or required, any embodiment of the invention described herein that is a polynucleotide encompasses both the double stranded form and each of two complementary forms known or predicted to make up the double stranded form of either the DNA, RNA or hybrid molecule.

The following are non-limiting examples of polynucleotides: a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes and primers. A polynucleotide may comprise modified nucleotides, such as methylated nucleotides and nucleotide analogs, uracyl, other sugars and linking groups such as fluororibose and thiolate, and nucleotide branches. The sequence of nucleotides may be further modified after polymerization, such as by conjugation, with a labeling component. Other types of modifications included in this definition are caps, substitution of one or more of the naturally occurring nucleotides with an analog, and introduction of means for attaching the polynucleotide to proteins, metal ions, labeling components, other polynucleotides or solid support. The polynucleotides can be obtained by chemical synthesis or derived from a microorganism.

The invention further comprises a complementary strand to a polynucleotide encoding an influenza antigen, epitope or immunogen. The complementary strand can be polymeric and of any length, and can contain deoxyribonucleotides, ribonucleotides, and analogs in any combination.

The terms "protein", "peptide", "polypeptide" and "polypeptide fragment" are used interchangeably herein to refer to polymers of amino acid residues of any length. The polymer can be linear or branched, it may comprise modified amino acids or amino acid analogs, and it may be interrupted by chemical moieties other than amino acids. The terms also encompass an amino acid polymer that has been modified naturally or by intervention; for example disulfide bond formation, glycosylation, lipidation, acetylation, phosphorylation, or any other manipulation or modification, such as conjugation with a labeling or bioactive component.

An "isolated" polynucleotide or polypeptide is one that is substantially free of the materials with which it is associated in its native environment. By substantially free, is meant at least 50%, advantageously at least 70%, more advantageously at least 80%, and even more advantageously at least 90% free of these materials.

Hybridization reactions can be performed under conditions of different "stringency." Conditions that increase stringency of a hybridization reaction are well known. See for example, "Molecular Cloning: A Laboratory Manual", second edition (Sambrook et al. 1989). Examples of relevant conditions include (in order of increasing stringency): incubation temperatures of 25°C, 37°C, 50°C, and 68°C; buffer concentrations of 10 x SSC, 6 x SSC, 1 x SSC, 0.1 x SSC (where SSC is 0.15 M NaCl and 15 mM citrate buffer) and their equivalent using other buffer systems; formamide concentrations of 0%, 25%, 50%, and 75%; incubation times from 5 minutes to 24 hours; 1, 2 or more washing steps; wash incubation times of 1, 2, or 15 minutes; and wash solutions of 6 x SSC, 1 x SSC, 0.1 x SSC, or deionized water.

Described herein are polynucleotides encoding functionally equivalent variants and derivatives of the influenza polypeptides and functionally equivalent fragments thereof which may enhance, decrease or not significantly affect properties of the polypeptides encoded thereby. These functionally equivalent variants, derivatives, and fragments display the ability to retain influenza activity. For instance, changes in a DNA sequence that do not change the encoded amino acid sequence, as well as those that result in conservative substitutions of amino acid residues, one or a few amino acid deletions or additions, and substitution of amino acid residues by amino acid analogs are those which will not significantly affect properties of the encoded polypeptide. Conservative amino acid substitutions are glycine/alanine; valine/isoleucine/leucine; asparagine/glutamine; aspartic acid/glutamic acid; serine/threonine/methionine; lysine/arginine; and phenylalanine/tyrosine/tryptophan. Described herein, the variants have at least 50%, at least 55%, at least 60%, at least 65%, at least 70%, at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98% or at least 99% homology or identity to the influenza polynucleotide or polypeptide of interest.

For the purposes of the present invention, sequence identity or homology is determined by comparing the sequences when aligned so as to maximize overlap and identity while minimizing sequence gaps. In particular, sequence identity may be determined using any of a number of mathematical algorithms. A nonlimiting example of a mathematical algorithm used for comparison of two sequences is the algorithm of Karlin & Altschul, Proc. Natl. Acad. Sci. USA 1990; 87: 2264-2268, modified as in Karlin & Altschul, Proc. Natl. Acad. Sci. USA 1993;90: 5873-5877.

Another example of a mathematical algorithm used for comparison of sequences is the algorithm of Myers & Miller, CABIOS 1988;4: 11-17. Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used. Yet another useful algorithm for identifying regions of local sequence similarity and alignment is the FASTA algorithm as described in Pearson & Lipman, Proc. Natl. Acad. Sci. USA 1988; 85: 2444-2448.

Advantageous for use according to the present invention is the WU-BLAST (Washington University BLAST) version 2.0 software. WU-BLAST version 2.0 executable programs for several UNIX platforms can be downloaded from ftp ://blast.wustl.edu/blast/executables. This program is based on WU-BLAST version 1.4, which in turn is based on the public domain NCBI-BLAST version 1.4 (Altschul & Gish, 1996, Local alignment statistics, Doolittle ed., Methods in Enzymology 266: 460-480; Altschul et al., Journal of Molecular Biology 1990; 215: 403-410; Gish & States, 1993;Nature Genetics 3: 266-272; Karlin & Altschul, 1993;Proc. Natl. Acad. Sci. USA 90: 5873-5877).

In general, comparison of amino acid sequences is accomplished by aligning an amino acid sequence of a polypeptide of a known structure with the amino acid sequence of a the polypeptide of unknown structure. Amino acids in the sequences are then compared and groups of amino acids that are homologous are grouped together. This method detects conserved regions of the polypeptides and accounts for amino acid insertions and deletions. Homology between amino acid sequences can be determined by using commercially available algorithms (see also the description of homology above). In addition to those otherwise mentioned herein, mention is made too of the programs BLAST, gapped BLAST, BLASTN, BLASTP, and PSI-BLAST, provided by the National Center for Biotechnology Information. These programs are widely used in the art for this purpose and can align homologous regions of two amino acid sequences.

In all search programs in the suite the gapped alignment routines are integral to the database search itself. Gapping can be turned off if desired. The default penalty (Q) for a gap of length one is Q=9 for proteins and BLASTP, and Q=10 for BLASTN, but may be changed to any integer. The default per-residue penalty for extending a gap (R) is R=2 for proteins and BLASTP, and R=10 for BLASTN, but may be changed to any integer. Any combination of values for Q and R can be used in order to align sequences so as to maximize overlap and identity while minimizing sequence gaps. The default amino acid comparison matrix is BLOSUM62, but other amino acid comparison matrices such as PAM can be utilized.

Alternatively or additionally, the term "homology " or "identity", for instance, with respect to a nucleotide or amino acid sequence, can indicate a quantitative measure of homology between two sequences. The percent sequence homology can be calculated as
(N*_{ref}* - N*_{dif}*)^{∗}100/N*_{ref}*, wherein N*_{dif}* is the total number of non-identical residues in the two sequences when aligned and wherein N*_{ref}* is the number of residues in one of the sequences. Hence, the DNA sequence AGTCAGTC will have a sequence identity of 75% with the sequence AATCAATC (N*_{ref}* = 8; *N_{dif}*=2).

Alternatively or additionally, "homology" or "identity" with respect to sequences can refer to the number of positions with identical nucleotides or amino acids divided by the number of nucleotides or amino acids in the shorter of the two sequences wherein alignment of the two sequences can be determined in accordance with the Wilbur and Lipman algorithm (Wilbur & Lipman, Proc Natl Acad Sci USA 1983; 80:726), for instance, using a window size of 20 nucleotides, a word length of 4 nucleotides, and a gap penalty of 4, and computer-assisted analysis and interpretation of the sequence data including alignment can be conveniently performed using commercially available programs (e.g., Intelligenetics™ Suite, Intelligenetics Inc. CA). When RNA sequences are said to be similar, or have a degree of sequence identity or homology with DNA sequences, thymidine (T) in the DNA sequence is considered equal to uracil (U) in the RNA sequence. Thus, RNA sequences are within the scope of the invention and can be derived from DNA sequences, by thymidine (T) in the DNA sequence being considered equal to uracil (U) in RNA sequences.

And, without undue experimentation, the skilled artisan can consult with many other programs or references for determining percent homology.

Described herein is the influenza polynucleotides contained in a vector molecule or an expression vector and operably linked to a promoter element and optionally to an enhancer.

The vector is a poxvirus, particularly a vaccinia virus or an avipox virus, such as fowlpox virus and canarypox virus. Advantageously, the virus is a canarypox virus. Advantageous canarypox strains may be an attenuated strain. The vector can express at least one epitope from Kentucky strains, Newmarket strains and/or Ohio strains. Advantageous canarypox constructs include, but are not limited to, vCP 1529, vCP 1533 and vCP 2242. Recombinant avipox viruses (see, e.g., U.S. Patent Nos. 5,505,941 and 5,756,103), such as an attenuated recombinant canarypox virus, for instance ALVAC, or an attenuated fowlpox virus, for instance TROVAC, are especially advantageous. In one advantageous embodiment, the recombinant ALVAC vaccine described by Edlund Toulemonde et al., Vet Rec. 2005 Mar 19;156(12):367-71 may be used as a canine influenza vaccine. Other viruses that may be used in methods described herein include vaccinia viruses, such as an attenuated vaccinia virus, for instance NYVAC, adenoviruses, such as canine adenoviruses (CAV), and herpesviruses, such as canine herpesvirus (CHV) or a feline herpesvirus (FHV).

A "vector" refers to a recombinant DNA or RNA plasmid or virus that comprises a heterologous polynucleotide to be delivered to a target cell, either in vitro or in vivo. The heterologous polynucleotide may comprise a sequence of interest for purposes of therapy, and may optionally be in the form of an expression cassette. As used herein, a vector needs not be capable of replication in the ultimate target cell or subject. The term includes cloning vectors also included are viral vectors.

The term "recombinant" means a polynucleotide semisynthetic, or synthetic origin which either does not occur in nature or is linked to another polynucleotide in an arrangement not found in nature.

"Heterologous" means derived from a genetically distinct entity from the rest of the entity to which it is being compared. For example, a polynucleotide, may be placed by genetic engineering techniques into a plasmid or vector derived from a different source, and is a heterologous polynucleotide. A promoter removed from its native coding sequence and operatively linked to a coding sequence other than the native sequence is a heterologous promoter.

The polynucleotides for use according to the invention may comprise additional sequences, such as additional encoding sequences within the same transcription unit, controlling elements such as promoters, ribosome binding sites, polyadenylation sites, additional transcription units under control of the same or a different promoter, sequences that permit cloning, expression, homologous recombination, and transformation of a host cell

Elements for the expression of an influenza antigen, epitope or immunogen are advantageously present in an inventive vector. In minimum manner, this comprises, consists essentially of, or consists of an initiation codon (ATG), a stop codon and a promoter, and optionally also a polyadenylation sequence for certain vectors such as plasmid and certain viral vectors, e.g., viral vectors other than poxviruses. When the polynucleotide encodes a polyprotein fragment, e.g. an influenza peptide, advantageously, in the vector, an ATG is placed at 5' of the reading frame and a stop codon is placed at 3'. Other elements for controlling expression may be present, such as enhancer sequences, stabilizing sequences, such as intron and signal sequences permitting the secretion of the protein.

Methods described herein for making and/or administering a vector or recombinants or plasmid for expression of gene products of genes of the invention either *in vivo* or *in vitro* can be any desired method, e.g., a method which is by or analogous to the methods disclosed in, or disclosed in documents cited in: U.S. Patent Nos. 4,603,112; 4,769,330; 4,394,448; 4,722,848; 4,745,051; 4,769,331; 4,945,050; 5,494,807; 5,514,375; 5,744,140; 5,744,141; 5,756,103; 5,762,938; 5,766,599; 5,990,091; 5,174,993; 5,505,941; 5,338,683; 5,494,807; 5,591,639; 5,589,466; 5,677,178; 5,591,439; 5,552,143; 5,580,859; 6,130,066; 6,004,777; 6,130,066; 6,497,883; 6,464,984; 6,451,770; 6,391,314; 6,387,376; 6,376,473; 6,368,603; 6,348,196; 6,306,400; 6,228,846; 6,221,362; 6,217,883; 6,207,166; 6,207,165; 6,159,477; 6,153,199; 6,090,393; 6,074,649; 6,045,803; 6,033,670; 6,485,729; 6,103,526; 6,224,882; 6,312,682; 6,348,450 and 6; 312,683; U.S. patent application Serial No. 920,197, filed October 16,1986; WO 90/01543; WO91/11525; WO 94/16716; WO 96/39491; WO 98/33510; EP 265785; EP 0 370 573; Andreansky et al., Proc. Natl. Acad. Sci. USA 1996;93:11313-11318; Ballay et al., EMBO J. 1993;4:3861-65; Felgner et al., J. Biol. Chem. 1994;269:2550-2561; Frolov et al., Proc. Natl. Acad. Sci. USA 1996;93:11371-11377; Graham, Tibtech 1990;8:85-87; Grunhaus et al., Sem. Virol. 1992;3:237-52; Ju et al., Diabetologia 1998;41:736-739; Kitson et al., J. Virol. 1991;65:3068-3075; McClements et al., Proc. Natl. Acad. Sci. USA 1996;93:11414-11420; Moss, Proc. Natl. Acad. Sci. USA 1996;93:11341-11348; Paoletti, Proc. Natl. Acad. Sci. USA 1996;93:11349-11353; Pennock et al., Mol. Cell. Biol. 1984;4:399-406; Richardson (Ed), Methods in Molecular Biology 1995;39, "Baculovirus Expression Protocols," Humana Press Inc.; Smith et al. (1983) Mol. Cell. Biol. 1983;3:2156-2165; Robertson et al., Proc. Natl. Acad. Sci. USA 1996;93:11334-11340; Robinson et al., Sem. Immunol. 1997;9:271; and Roizman, Proc. Natl. Acad. Sci. USA 1996;93:11307-11312. Thus, the vector described herein can be any suitable recombinant virus or virus vector, such as a poxvirus (e.g., vaccinia virus, avipox virus, canarypox virus, fowlpox virus, raccoonpox virus, swinepox virus, etc.), adenovirus (e.g., human adenovirus, canine adenovirus), herpesvirus (e.g. canine herpesvirus), baculovirus, retrovirus, etc. or the vector can be a plasmid. The herein cited documents, in addition to providing examples of vectors , can also provide sources for non-influenza peptides or fragments thereof to be expressed by vector or vectors in, or included in, the compositions desrcibed herein.

The present disclosure also relates to preparations comprising vectors, such as expression vectors, e.g., therapeutic compositions. Described herein, the preparations can comprise, consist essentially of, or consist of one or more vectors, e.g., expression vectors, such as *in vivo* expression vectors, comprising, consisting essentially or consisting of (and advantageously expressing) one or more of influenza antigens, epitopes or immunogens. Advantageously described herein, the vector contains and expresses a polynucleotide that includes, consists essentially of, or consists of a polynucleotide coding for (and advantageously expressing) an influenza antigen, epitope or immunogen, in a pharmaceutically or veterinarily acceptable carrier, excipient or vehicle. Thus, described herein, the other vector or vectors in the preparation comprises, consists essentially of or consists of a polynucleotide that encodes, and under appropriate circumstances the vector expresses one or more other proteins of an influenza antigen, epitope or immunogen (e.g., hemagglutinin, neuraminidase, nucleoprotein) or a fragment thereof.

Also described herein, the vector or vectors in the preparation comprise, or consist essentially of, or consist of polynucleotide(s) encoding one or more proteins or fragment(s) thereof of an influenza antigen, epitope or immunogen, the vector or vectors expressing the polynucleotide(s). The inventive preparation advantageously comprises, consists essentially of, or consists of, at least two vectors comprising, consisting essentially of, or consisting of, and advantageously also expressing, advantageously *in vivo* under appropriate conditions or suitable conditions or in a suitable host cell, polynucleotides from different canine influenza isolates encoding the same proteins and/or for different proteins, but advantageously the same proteins. Preparations containing one or more vectors containing, consisting essentially of or consisting of polynucleotides encoding, and advantageously expressing, advantageously *in vivo,* an influenza antigen, fusion protein or an epitope thereof. Further described herein are mixtures of vectors that contain, consist essentially of, or consist of coding for, and express, different influenza antigens, epitopes or immunogens, e.g., an influenza antigen, epitope or immunogen from different species such as, but not limited to, humans, , pigs, , in addition to avian species including chicken, ducks and geese. Described herein, the expression vector is a viral vector, in particular an *in vivo* expression vector. As advantageously described herein, the expression vector is an adenovirus vector. Advantageously described herein, the adenovirus is a human Ad5 vector, an E1-deleted and/ or an E3-deleted adenovirus.

The viral vector for use according to the invention is a poxvirus, e.g. a vaccinia virus or an attenuated vaccinia virus, (for instance, MVA, a modified Ankara strain obtained after more than 570 passages of the Ankara vaccine strain on chicken embryo fibroblasts; *see* Stickl & Hochstein-Mintzel, Munch. Med. Wschr., 1971, 113, 1149-1153; Sutter et al., Proc. Natl. Acad. Sci. U.S.A., 1992, 89, 10847-10851; available as ATCC VR-1508; or NYVAC, *see* U.S. Patent No. 5,494,807, for instance, Examples 1 to 6 and *et seq* of U.S. Patent No. 5,494,807 which discuss the construction of NYVAC, as well as variations of NYVAC with additional ORFs deleted from the Copenhagen strain vaccinia virus genome, as well as the insertion of heterologous coding nucleic acid molecules into sites of this recombinant, and also, the use of matched promoters; see also WO96/40241), an avipox virus or an attenuated avipox virus (e.g., canarypox, fowlpox, dovepox, pigeonpox, quailpox, ALVAC or TROVAC; see, e.g., U.S. Patent No. 5,505,941, 5,494,807), swinepox, raccoonpox, camelpox, or myxomatosis virus.

According to another embodiment of the invention, the poxvirus vector is a canarypox virus or a fowlpox virus vector, advantageously an attenuated canarypox virus or fowlpox virus. In this regard, is made to the canarypox available from the ATCC under access number VR-111. Attenuated canarypox viruses are described in U.S. Patent No. 5,756,103 (ALVAC) and WO01/05934. Numerous fowlpox virus vaccination strains are also available, e.g. the DIFTOSEC CT strain marketed by MERIAL and the NOBILIS VARIOLE vaccine marketed by INTERVET; and, reference is also made to U.S. Patent No. 5,766,599 which pertains to the attenuated fowlpox strain TROVAC.

For information on the method to generate recombinants thereof and how to administer recombinants thereof, the skilled artisan can refer documents cited herein and to WO90/12882, e.g., as to vaccinia virus mention is made of U.S. Patents Nos. 4,769,330, 4,722,848, 4,603,112, 5,110,587, 5,494,807, and 5,762,938 *inter alia;* as to fowlpox, mention is made of U.S. Patents Nos. 5,174,993, 5,505,941 and US-5,766,599 *inter alia;* as to canarypox mentionis made of U.S. Patent No. 5,756,103 *inter alia*; as to swinepox mention is made of U.S. Patent No. 5,382,425 *inter alia;* and, as to raccoonpox, mention is made of WO00/03030 *inter alia.*

When the expression vector is a vaccinia virus, insertion site or sites for the polynucleotide or polynucleotides to be expressed are advantageously at the thymidine kinase (TK) gene or insertion site, the hemagglutinin (HA) gene or insertion site, the region encoding the inclusion body of the A type (ATI); see also documents cited herein, especially those pertaining to vaccinia virus. In the case of canarypox, advantageously the insertion site or sites are ORF(s) C3, C5 and/or C6; see also documents cited herein, especially those pertaining to canarypox virus. In the case of fowlpox, advantageously the insertion site or sites are ORFs F7 and/or F8; see also documents cited herein, especially those pertaining to fowlpox virus. The insertion site or sites for MVA virus area advantageously as in various publications, including Carroll M. W. et al., Vaccine, 1997, 15 (4), 387-394; Stittelaar K. J. et al., J. Virol., 2000, 74 (9), 4236-4243; Sutter G. et al., 1994, Vaccine, 12 (11), 1032-1040; and, in this regard it is also noted that the complete MVA genome is described in Antoine G., Virology, 1998, 244, 365-396, which enables the skilled artisan to use other insertion sites or other promoters.

Advantageously, the polynucleotide to be expressed is inserted under the control of a specific poxvirus promoter, e.g., the vaccinia promoter 7.5 kDa (Cochran et al., J. Virology, 1985, 54, 30-35), the vaccinia promoter I3L (Riviere et al., J. Virology, 1992, 66, 3424-3434), the vaccinia promoter HA (Shida, Virology, 1986, 150, 451-457), the cowpox promoter ATI (Funahashi et al., J. Gen. Virol., 1988, 69, 35-47), the vaccinia promoter H6 (Taylor J. et al., Vaccine, 1988, 6, 504-508; Guo P. et al. J. Virol., 1989, 63, 4189-4198; Perkus M. et al., J. Virol., 1989, 63, 3829-3836), *inter alia.*

As described herein the viral vector is an adenovirus, such as a human adenovirus (HAV) or a canine adenovirus (CAV).

As described herein the viral vector is a human adenovirus, in particular a serotype 5 adenovirus, rendered incompetent for replication by a deletion in the E1 region of the viral genome, in particular from about nucleotide 459 to about nucleotide 3510 by reference to the sequence of the hAd5 disclosed in Genbank under the accession number M73260 and in the referenced publication J. Chroboczek et al Virol. 1992, 186, 280-285. The deleted adenovirus is propagated in E1-expressing 293 (F. Graham et al J. Gen. Virol. 1977, 36, 59-72) or PER cells, in particular PER.C6 (F. Falloux et al Human Gene Therapy 1998, 9, 1909-1917). The human adenovirus can be deleted in the E3 region, in particular from about nucleotide 28592 to about nucleotide 30470. The deletion in the E1 region can be done in combination with a deletion in the E3 region (*see, e.g.* J. Shriver et al. Nature, 2002, 415, 331-335, F. Graham et al Methods in Molecular Biology Vol .7: Gene Transfer and Expression Protocols Edited by E. Murray, The Human Press Inc, 1991, p 109-128; Y. Ilan et al Proc. Natl. Acad. Sci. 1997, 94, 2587-2592; US6,133,028; US6,692,956; S. Tripathy et al Proc. Natl. Acad. Sci. 1994, 91, 11557-11561; B. Tapnell Adv. Drug Deliv. Rev.1993, 12, 185-199;X. Danthinne et al Gene Thrapy 2000, 7, 1707-1714; K. Berkner Bio Techniques 1988, 6, 616-629; K. Berkner et al Nucl. Acid Res. 1983, 11, 6003-6020; C. Chavier et al J. Virol. 1996, 70, 4805-4810). The insertion sites can be the E1 and/or E3 loci (region) eventually after a partial or complete deletion of the E1 and/or E3 regions. Advantageously, when the expression vector is an adenovirus, the polynucleotide to be expressed is inserted under the control of a promoter functional in eukaryotic cells, such as a strong promoter, preferably a cytomegalovirus immediate-early gene promoter (CMV-IE promoter), in particular the enhancer / promoter region from about nucleotide -734 to about nucleotide +7 in M. Boshart et al Cell 1985, 41, 521-530 or the enhancer / promoter region from the pCI vector from Promega Corp. The CMV-IE promoter is advantageously of murine or human origin. The promoter of the elongation factor 1α can also be used. A muscle specific promoter can also be used (X. Li et al Nat. Biotechnol. 1999, 17, 241-245). Strong promoters are also discussed herein in relation to plasmid vectors. In one embodiment, a splicing sequence can be located downstream of the enhancer / promoter region. For example, the intron 1 isolated from the CMV-IE gene (R. Stenberg et al J. Virol. 1984, 49, 190), the intron isolated from the rabbit or human β-globin gene, in particular the intron 2 from the b-globin gene, the intron isolated from the immunoglobulin gene, a splicing sequence from the SV40 early gene or the chimeric intron sequence isolated from the pCI vector from Promege Corp. comprising the human β-globin gene donor sequence fused to the mouse immunoglobulin acceptor sequence (from about nucleotide 890 to about nucleotide 1022 in Genbank under the accession number CVU47120). A poly(A) sequence and terminator sequence can be inserted downstream the polynucleotide to be expressed, e.g. a bovine growth hormone gene, in particular from about nucleotide 2339 to about nucleotide 2550 in Genbank under the accession number BOVGHRH, a rabbit β-globin gene or a SV40 late gene polyadenylation signal.

As also described herein the viral vector is a canine adenovirus, in particular a CAV-2 (see, e.g. L. Fischer et al. Vaccine, 2002, 20, 3485-3497; U.S. Patent No. 5,529,780; U.S. Patent No. 5,688,920; PCT Application No. WO95/14102). For CAV, the insertion sites can be in the E3 region and /or in the region located between the E4 region and the right ITR region (see U.S. Patent No. 6,090,393; U.S. Patent No. 6,156,567). Described herein the insert is under the control of a promoter, such as a cytomegalovirus immediate-early gene promoter (CMV-IE promoter) or a promoter already described for a human adenovirus vector. A poly(A) sequence and terminator sequence can be inserted downstream the polynucleotide to be expressed, e.g. a bovine growth hormone gene or a rabbit β-globin gene polyadenylation signal.

As further described herein the viral vector is a herpesvirus such as a canine herpesvirus (CHV) or a feline herpesvirus (FHV). For CHV, the insertion sites may be in particular in the thymidine kinase gene, in the ORF3, or in the UL43 ORF (see U.S. Patent No. 6,159,477). In one embodiement the polynucleotide to be expressed is inserted under the control of a promoter functional in eukaryotic cells, advantageously a CMV-IE promoter (murine or human). A poly(A) sequence and terminator sequence can be inserted downstream the polynucleotide to be expressed, e.g. bovine growth hormone or a rabbit β-globin gene polyadenylation signal.

Yet further described herein, the expression vector is a plasmid vector or a DNA plasmid vector, in particular an *in vivo* expression vector. In a specific, non-limiting example, the pVR1020 or 1012 plasmid (VICAL Inc.; Luke C. et al., Journal of Infectious Diseases, 1997, 175, 91-97; Hartikka J. et al., Human Gene Therapy, 1996, 7, 1205-1217, see, e.g., U.S. Patent Nos. 5,846,946 and 6,451,769) can be utilized as a vector for the insertion of a polynucleotide sequence. The pVR1020 plasmid is derived from pVR1012 and contains the human tPA signal sequence. As described herein the human tPA signal comprises from amino acid M(1) to amino acid S(23) in Genbank under the accession number HUMTPA14. As also described herein, the plasmid utilized as a vector for the insertion of a polynucleotide sequence can contain the signal peptide sequence of equine IGF1 from amino acid M(24) to amino acid A(48) in Genbank under the accession number U28070. Additional information on DNA plasmids which may be consulted or employed in the practice are found, for example, in U.S. Patent Nos. 6,852,705; 6,818,628; 6,586,412; 6,576,243; 6,558,674; 6,464,984; 6,451,770; 6,376,473 and 6,221,362.

The term plasmid covers any DNA transcription unit comprising a polynucleotide according to the invention and the elements necessary for its *in vivo* expression in a cell or cells of the desired host or target; and, in this regard, it is noted that a supercoiled or non-supercoiled, circular plasmid, as well as a linear form, are intended to be within the scope of the invention.

Each plasmid comprises or contains or consists essentially of, in addition to the polynucleotide encoding an influenza antigen, epitope or immunogen, optionally fused with a heterologous peptide sequence, variant, analog or fragment, operably linked to a promoter or under the control of a promoter or dependent upon a promoter. In general, it is advantageous to employ a strong promoter functional in eukaryotic cells. The preferred strong promoter is the immediate early cytomegalovirus promoter (CMV-IE) of human or murine origin, or optionally having another origin such as the rat or guinea pig. The CMV-IE promoter can comprise the actual promoter part, which may or may not be associated with the enhancer part. Reference can be made to EP-A-260 148, EP-A-323 597, U.S. Patents Nos. 5,168,062, 5,385,839, and 4,968,615, as well as to PCT Application No WO87/03905. The CMV-IE promoter is advantageously a human CMV-IE (Boshart M. et al., Cell., 1985, 41, 521-530) or murine CMV-IE.

In more general terms, the promoter has either a viral or a cellular origin. A strong viral promoter other than CMV-IE that may be usefully employed in the practice of the invention is the early/late promoter of the SV40 virus or the LTR promoter of the Rous sarcoma virus. A strong cellular promoter that may be usefully employed in the practice of the invention is the promoter of a gene of the cytoskeleton, such as e.g. the desmin promoter (Kwissa M. et al., Vaccine, 2000, 18, 2337-2344), or the actin promoter (Miyazaki J. et al., Gene, 1989, 79, 269-277).

Functional sub fragments of these promoters, i.e., portions of these promoters that maintain an adequate promoting activity, are included within the present invention, e.g. truncated CMV-IE promoters according to PCT Application No. WO98/00166 or U.S. Patent No. 6,156,567 can be used in the practice of the invention. A promoter in the practice of the invention consequently includes derivatives and sub fragments of a full-length promoter that maintain an adequate promoting activity and hence function as a promoter, preferably promoting activity substantially similar to that of the actual or full-length promoter from which the derivative or sub fragment is derived, e.g., akin to the activity of the truncated CMV-IE promoters of U.S. Patent No. 6,156,567 to the activity of full-length CMV-IE promoters. Thus, a CMV-IE promoter in the practice of the invention can comprise or consist essentially of or consist of the promoter portion of the full-length promoter and/or the enhancer portion of the full-length promoter, as well as derivatives and sub fragments.

Preferably, the plasmids comprise or consist essentially of other expression control elements. It is particularly advantageous to incorporate stabilizing sequence(s), e.g., intron sequence(s), preferably the first intron of the hCMV-IE (PCT Application No. WO89/01036), the intron II of the rabbit β-globin gene (van Ooyen et al., Science, 1979, 206, 337-344).

As to the polyadenylation signal (polyA) for the plasmids and viral vectors other than poxviruses, use can more be made of the poly(A) signal of the bovine growth hormone (bGH) gene (*see* U.S. Patent No. 5,122,458), or the poly(A) signal of the rabbit β-globin gene or the poly(A) signal of the SV40 virus.

As also described herein, the expression vectors are expression vectors used for the *in vitro* expression of proteins in an appropriate cell system. The expressed proteins can be harvested in or from the culture supernatant after, or not after secretion (if there is no secretion a cell lysis typically occurs or is performed), optionally concentrated by concentration methods such as ultrafiltration and/or purified by purification means, such as affinity, ion exchange or gel filtration-type chromatography methods.

A "host cell" denotes a prokaryotic or eukaryotic cell that has been genetically altered, or is capable of being genetically altered by administration of an exogenous polynucleotide, such as a recombinant plasmid or vector. When referring to genetically altered cells, the term refers both to the originally altered cell and to the progeny thereof. Advantageous host cells include, but are not limited to, baby hamster kidney (BHK) cells, colon carcinoma (Caco-2) cells, COS7 cells, MCF-7 cells, MCF-10A cells, Madin-Darby canine kidney (MDCK) lines, mink lung (Mv1Lu) cells, MRC-5 cells, U937 cells and VERO cells. Polynucleotides comprising a desired sequence can be inserted into a suitable cloning or expression vector, and the vector in turn can be introduced into a suitable host cell for replication and amplification. Polynucleotides can be introduced into host cells by any means known in the art. The vectors containing the polynucleotides of interest can be introduced into the host cell by any of a number of appropriate means, including direct uptake, endocytosis, transfection, f-mating, electroporation, transfection employing calcium chloride, rubidium chloride, calcium phosphate, DEAE-dextran, or other substances; microprojectile bombardment; lipofection; and infection (where the vector is infectious, for instance, a retroviral vector). The choice of introducing vectors or polynucleotides will often depend on features of the host cell.

As desribed herein is the administration of a therapeutically effective amount of a formulation for the delivery and expression of an influenza antigen, epitope or immunogen in a target cell. Determination of the therapeutically effective amount is routine experimentation for one of ordinary skill in the art.
As described herein, the formulation comprises an expression vector comprising a polynucleotide that expresses an influenza antigen, epitope or immunogen and a pharmaceutically or veterinarily acceptable carrier, vehicle or excipient. In an advantageous embodiment, the pharmaceutically or veterinarily acceptable carrier, vehicle or excipient facilitates transfection and/or improves preservation of the vector or protein.

The pharmaceutically or veterinarily acceptable carriers or vehicles or excipients are well known to the one skilled in the art. For example, a pharmaceutically or veterinarily acceptable carrier or vehicle or excipient can be a 0.9% NaCl (e.g., saline) solution or a phosphate buffer. Other pharmaceutically or veterinarily acceptable carrier or vehicle or excipients that can be used for methods of this invention include, but are not limited to, poly-(L-glutamate) or polyvinylpyrrolidone. The pharmaceutically or veterinarily acceptable carrier or vehicle or excipients may be any compound or combination of compounds facilitating the administration of the vector (or protein expressed from an inventive vector *in vitro*); advantageously, the carrier, vehicle or excipient may facilitate transfection and/or improve preservation of the vector (or protein). Doses and dose volumes are herein discussed in the general description and can also be determined by the skilled artisan from this disclosure read in conjunction with the knowledge in the art, without any undue experimentation.

The cationic lipids containing a quaternary ammonium salt which are advantageously but not exclusively suitable for plasmids, are advantageously those having the following formula: 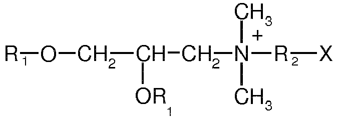 in which R₁ is a saturated or unsaturated straight-chain aliphatic radical having 12 to 18 carbon atoms, R₂ is another aliphatic radical containing 2 or 3 carbon atoms and X is an amine or hydroxyl group, e.g. the DMRIE. In another embodiment the cationic lipid can be associated with a neutral lipid, e.g. the DOPE.

Among these cationic lipids, preference is given to DMRIE (N-(2-hydroxyethyl)-N,N-dimethyl-2,3-bis(tetradecyloxy)-1-propane ammonium; WO96/34109), advantageously associated with a neutral lipid, advantageously DOPE (dioleoyl-phosphatidyl-ethanol amine; Behr J. P., 1994, Bioconjugate Chemistry, 5, 382-389), to form DMRIE-DOPE.

Advantageously, the plasmid mixture with the adjuvant is formed extemporaneously and advantageously contemporaneously with administration of the preparation or shortly before administration of the preparation; for instance, shortly before or prior to administration, the plasmid-adjuvant mixture is formed, advantageously so as to give enough time prior to administration for the mixture to form a complex, e.g. between about 10 and about 60 minutes prior to administration, such as approximately 30 minutes prior to administration.

When DOPE is present, the DMRIE:DOPE molar ratio is advantageously about 95: about 5 to about 5:about 95, more advantageously about 1: about 1, e.g., 1:1.

The DMRIE or DMRIE-DOPE adjuvant:plasmid weight ratio can be between about 50: about 1 and about 1: about 10, such as about 10: about 1 and about 1:about 5, and advantageously about 1: about 1 and about 1: about 2, e.g., 1:1 and 1:2.

Also described are inactivated canine influenza vaccines. As used herein, the term "inactivated vaccine" means a vaccine composition containing an infectious organism or pathogen that is no longer capable of replication or growth. Inactivation may be accomplished by a variety of methods sufficient to prevent replication or growth of the organism while maintaining its immunogenicity.

The inactivated vaccine may be an inactivated form of an isolate of an influenza virus from an affected dog. The virus may be isolated from the alveoli or lung of an affected dog. In another aspect of the disclosure, the inactivated vaccine may be an inactivated equine influenza H3. In an aspect of the disclosure, the equine influenza H3 is a Kentucky or Newmarket equine influenza. The inactivated vaccine described herein may be an inactivated version of any one of the influenza strains described above.

An inactivated vaccine may be prepared as well from the harvested culture fluid. The virus may be produced either by inoculation of 10-11-day embryonated eggs (J. Violay et al US6,048,537) or by inoculation of BHK-21 cell culture (C. Ross et al Archiv. Für die gesamte Virusforschung 1970, 30, 82-88; T. Tolstova et al Acta Virol. 1966, 10, 315-321; Ho. Merten et al Adv. Exp. Med. Biol. 1996, 397, 141-151), of MDCK cell culture (J. Tree et al Vaccine 2001, 19, 3444-3450; Y. Ghendon et al Vaccine 2005, 23, 4678-4684; R. Brands et al Dev. Biol. Stand. 1999, 98, 93-100; R. Youil et al J Virol. Methods 2004, 120, 23-31), of Vero cell culture (O. Kistner et al Vaccine 1998, 16, 960-968; E. Govorkova et al J. Virol. 1996, 70, 5519-5524). The allantoic fluid or the cell culture supernatant can be clarified by low centrifugation and/or filtration. The virus can be concentrated by ultrafiltration and can be purified by zonal centrifugation on sucrose gradient (J. Violay et al US6,048,537; O. Kistner et al idem), by gel filtration (D. Nayak et al J. Chromatogr. B Analyt. Technol. Biomed. Life Sci. 2005, 823, 75-81; S. Tomita et al Kitasato Arch. Exp. Med. 1971, 44, 185-196).

Inactivation may be achieved by treating the viruses by any of the methods commonly employed to make inactivated vaccines. These methods include but are not limited to formaldehyde treatment (O. Kistner et al idem; A. Garcia et al Avian Diseases 1998, 42, 248-256), betapropriolactone treatment (B. Bdowsky et al Vaccine 1991, 9, 398-402 and Vaccine 1993, 11, 343-348; N. Keverin et al Arch. Virol. 2000, 145, 1059-1066), ethylene-imine treatment (D. Swayne et al Avian Diseases 2001, 45, 355-365), treatment with organic solvents, treatment with detergents, treatment with Tween-ether or treatment with Triton X-100 (J. Vilay et al idem) for allantoic fluid . For the inactivation the concentration can be about 0.01-0.2 % w/v for the formaldehyde; about 0.03-0.2 % w/v for the betapropiolactone; about 0.5-20 mM for ethyleneimine. The methods recited herein serve as art-known examples for inactivating virus. Inactivated virus vaccines are usually administered mixed with an adjuvant. The inactivated vaccine can be administered to the animal by any of a plurality of methods which include but are not limited to inoculation intramuscularly, intradermally, or subcutaneously, spraying, ocularly, nasally, orally, or in ovo.

The immunogenic compositions and vaccines for use according to the invention may comprise or consist essentially of one or more adjuvants. Suitable adjuvants for use in the practice of the present invention are (1) polymers of acrylic or methacrylic acid, maleic anhydride and alkenyl derivative polymers, (2) immunostimulating sequences (ISS), such as oligodeoxyribonucleotide sequences having one ore more non-methylated CpG units (Klinman et al., Proc. Natl. Acad. Sci., USA, 1996, 93, 2879-2883; WO98/16247), (3) an oil in water emulsion, such as the SPT emulsion described on p 147 of "Vaccine Design, The Subunit and Adjuvant Approach" published by M. Powell, M. Newman, Plenum Press 1995, and the emulsion MF59 described on p 183 of the same work, (4) cation lipids containing a quaternary ammonium salt, e.g., DDA (5) cytokines, (6) aluminum hydroxide or aluminum phosphate, (7) saponin or (8) other adjuvants discussed in any document cited, or (9) any combinations or mixtures thereof, or (10) immunostimulating complexes (ISCOMs) that are open cage-like complexes built up by cholesterols, lipids, immunogen, and saponins from the bark of the tree Quillaia saponaria Molina (Sjolander et al., J. Leukocyte Biol., 1998, 64 (6), 713-723).

The oil in water emulsion (3), which is especially appropriate for viral vectors, can be based on: light liquid paraffin oil (European pharmacopoeia type), isoprenoid oil such as squalane, squalene, oil resulting from the oligomerization of alkenes, e.g. isobutene or decene, esters of acids or alcohols having a straight-chain alkyl group, such as vegetable oils, ethyl oleate, propylene glycol, di(caprylate/caprate), glycerol tri(caprylate/caprate) and propylene glycol dioleate, or esters of branched, fatty alcohols or acids, especially isostearic acid esters.
The oil is used in combination with emulsifiers to form an emulsion. The emulsifiers may be nonionic surfactants, such as: esters of on the one hand sorbitan, mannide (e.g. anhydromannitol oleate), glycerol, polyglycerol or propylene glycol and on the other hand oleic, isostearic, ricinoleic or hydroxystearic acids, said esters being optionally ethoxylated, or polyoxypropylene-polyoxyethylene copolymer blocks, such as Pluronic, e.g., L121.

The type (1) immunostimulating complex matrix type (ISCOM™ matrix) adjuvants are advantageous choices according to the invention, especially since it has been shown that this class of adjuvants are capable of up-regulating MHC Class II expression by APCs. An ISCOM™. matrix consists of (optionally fractionated) saponins (triterpenoids) from Quillaja saponaria, cholesterol, and phospholipid. When admixed with the immunogenic protein, the resulting particulate formulation is what is known as an ISCOM particle where the saponin constitutes 60-70% w/w, the cholesterol and phospholipid 10-15% w/w, and the protein 10-15% w/w. Details relating to composition and use of immunostimulating complexes can e.g. be found in Morein B et al., 1995, Clin. Immunother. 3: 461-475 as well as Barr IG and Mitchell GF, 1996, Immunol. and Cell Biol. 74: 8-25 provide useful instructions for the preparation of complete immunostimulating complexes.

Among the type (1) adjuvant polymers, preference is given to polymers of crosslinked acrylic or methacrylic acid, especially crosslinked by polyalkenyl ethers of sugars or polyalcohols. These compounds are known under the name carbomer (Pharmeuropa, vol. 8, no. 2, June 1996). One skilled in the art can also refer to U.S. Patent No. 2,909,462, which provides such acrylic polymers crosslinked by a polyhydroxyl compound having at least three hydroxyl groups, preferably no more than eight such groups, the hydrogen atoms of at least three hydroxyl groups being replaced by unsaturated, aliphatic radicals having at least two carbon atoms. The preferred radicals are those containing 2 to 4 carbon atoms, e.g. vinyls, allyls and other ethylenically unsaturated groups. The unsaturated radicals can also contain other substituents, such as methyl. Products sold under the name Carbopol (BF Goodrich, Ohio, USA) are especially suitable. They are crosslinked by allyl saccharose or by allyl pentaerythritol. Among them, reference is made to Carbopol 974P, 934P and 971P.

As to the maleic anhydride-alkenyl derivative copolymers, preference is given to EMA (Monsanto), which are straight-chain or crosslinked ethylene-maleic anhydride copolymers and they are, for example, crosslinked by divinyl ether. Reference is also made to J. Fields et al., Nature 186: 778-780, June 4, 1960.

With regard to structure, the acrylic or methacrylic acid polymers and EMA are preferably formed by basic units having the following formula: 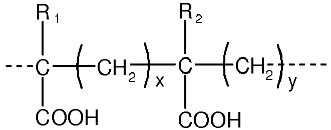 in which:
- R₁ and R₂, which can be the same or different, represent H or CH₃
- x = 0 or 1, preferably x = 1
- y = 1 or 2, with x + y = 2.

For EMA, x = 0 and y = 2 and for carbomers x = y = 1.

These polymers are soluble in water or physiological salt solution (20 g/l NaCl) and the pH can be adjusted to 7.3 to 7.4, e.g., by soda (NaOH), to provide the adjuvant solution in which the expression vector(s) can be incorporated. The polymer concentration in the final vaccine composition can range between 0.01 and 1.5% w/v, advantageously 0.05 to 1% w/v and preferably 0.1 to 0.4% w/v.

The cytokine or cytokines (5) can be in protein form in the immunogenic or vaccine composition, or can be co-expressed in the host with the immunogen or immunogens or epitope(s) thereof. Preference is given to the co-expression of the cytokine or cytokines, either by the same vector as that expressing the immunogen or immunogens or epitope(s) thereof, or by a separate vector therefor.

The disclosure comprehends preparing such combination compositions; for instance by admixing the active components, advantageously together and with an adjuvant, carrier, cytokine, and/or diluent.

Cytokines that may be used in the present invention include, but are not limited to, granulocyte colony stimulating factor (G-CSF), granulocyte/macrophage colony stimulating factor (GM-CSF), interferon α (IFN α), interferon β (IFN β), interferon γ, (IFN γ), interleukin-1α (IL-1α), interleukin-1β (IL-1β), interleukin-2 (IL-2), interleukin-3 (IL-3), interleukin-4 (IL-4), interleukin-5 (IL-5), interleukin-6 (IL-6), interleukin-7 (IL-7), interleukin-8 (IL-8), interleukin-9 (IL-9), interleukin-10 (IL-10), interleukin-11 (IL-11), interleukin-12 (IL-12), tumor necrosis factor α (TNF α), tumor necrosis factor β (TNF β), and transforming growth factor β (TGF β). It is understood that cytokines can be co-administered and/or sequentially administered with the immunogenic or vaccine composition for use according to the present invention. Thus, for instance, the vaccine for use according to the instant invention can also contain an exogenous nucleic acid molecule that expresses in vivo a suitable cytokine, e.g., a cytokine matched to this host to be vaccinated or in which an immunological response is to be elicited (for instance, a canine cytokine for preparations to be administered to dogs).

Advantageously, the pharmaceutical and/or therapeutic compositions and/or formulations according for use according to the invention comprise or consist essentially of or consist of an effective quantity to elicit a therapeutic response of one or more expression vectors and/or polypeptides as discussed herein; and, an effective quantity can be determined from this disclosure, including the documents incorporated herein, and the knowledge in the art, without undue experimentation.

In the case of therapeutic and/or pharmaceutical compositions based on a plasmid vector, a dose can comprise, consist essentially of or consist of, in general terms, about in 1 µg to about 2000 µg, advantageously about 50 µg to about 1000 µg and more advantageously from about 100 µg to about 800 µg of plasmid expressing the influenza antigen, epitope or immunogen. When the therapeutic and/or pharmaceutical compositions based on a plasmid vector is administered with electroporation the dose of plasmid is generally between about 0.1 µg and 1mg, advantageously between about 1 µg and 100 µg, advantageously between about 2 µg and 50 µg. The dose volumes can be between about 0.1 and about 2 ml, advantageously between about 0.2 and about 1 ml. These doses and dose volumes are suitable for the treatment of canines and other mammalian target species such as equines and felines.

The therapeutic and/or pharmaceutical composition contains per dose from about 10⁴ to about 10¹¹, advantageously from about 10⁵ to about 10¹⁰ and more advantageously from about 10⁶ to about 10⁹ viral particles of recombinant adenovirus expressing an influenza antigen, epitope or immunogen. In the case of therapeutic and/or pharmaceutical compositions based on a poxvirus, a dose can be between about 10² pfu and about 10⁹ pfu. The pharmaceutical composition contains per dose from about 10⁵ to 10⁹, advantageously from about 10⁶ to 10⁸ pfu of poxvirus or herpesvirus recombinant expressing the influenza antigen, epitope or immunogen.

The dose volume of compositions for target species that are mammals, e.g., the dose volume of canine compositions, based on viral vectors, e.g., non-poxvirus-viral-vector-based compositions, is generally between about 0.1 to about 2.0 ml, preferably between about 0.1 to about 1.0 ml, and more preferably between about 0.5 ml to about 1.0 ml.

With inactivated compositions of the virus or organism or pathogen produced on the new cell culture, the animal may be administered approximately 10⁴-10⁹ equivalent CCID₅₀ (titer before inactivation), advantageously approximately 10⁵-10⁸ equivalent CCID₅₀ in a single dosage unit. Alternately, embryonated chicken eggs may provide a substrate for pathogen propagation, in which case the unit of measure is median egg infectious dose (EID₅₀). The volume of one single dosage unit can be between 0.2 ml and 5.0 ml and advantageously between 0.5 ml and 2.0 ml and more advantageously about 2.0 ml. One or more administrations can be done; e.g. with two injections at 2-4 weeks interval, and advantageously with a boost about 3 weeks after the first injection.

In an advantageous embodiment, an animal, advantageously a dog, is vaccinated with two doses of inactivated vaccine at about 3 to 4 week intervals via the subcutaneous route, although an intramuscular route is also contemplated. Blood samples may be collected on the day of the first and/or second vaccination and about 2 to 4 weeks after the second vaccination to determine the levels of anti-influenza virus specific antibodies by methods known to one of skill in the art, for example, virus neutralization, hemagglutination inhibition, ELISA or single radial heamolysis (SRH) tests.

The efficacy of the inactivated vaccines may be tested about 2 to 4 weeks after the second immunization by challenging animals, advantageously dogs, with a virulent strain of influenza, advantageously the influenza H3N8 strain. The animal may be challenged by spray, intra-nasally, intra-tracheally and/or orally. The challenge viral may be about 10⁵⁻⁸ EID50 in a volume depending upon the route of administration. For example, if the administration is by spray, a virus suspension is aerosolized to generate about 1 to 100 µm droplets, if the administration is intra-nasal, intra-tracheal or oral, the volume of the challenge virus is about 0.5 ml, 1-2 ml and 5-10 ml, respectively. Animals may be observed daily for 14 days following challenge for clinical signs, for example, fever, cough, nasal, ocular discharge, respiratory distress, anorexia and lethargy. In addition, groups of animals may be euthanized and evaluated for pathological findings of pulmonary and pleural hemorrhage, tracheitis, bronchitis, broncholilitis, and bronchopneumonia. Tracheal swabs may be collected from all animals post challenge days 1-14 for virus isolation. The presence or absence of viral antigens in respiratory tissues may be evaluated by immunohistochemistry, for example, on days 3, 7 and 10 post-challenge. Blood samples may be collected post-challenge (e.g., on days 7 and 14 post-challenge) and may be analyzed for the presence of anti-influenza H3N8 virus specific antibody.

It should be understood by one of skill in the art that the disclosure herein is provided by way of example. From the disclosure herein and the knowledge in the art, the skilled artisan can determine the number of administrations, the administration route, and the doses to be used for each injection protocol, without any undue experimentation.

The present invention contemplates at least one administration to an animal of an efficient amount of the therapeutic composition for use according to the invention. The animal may be male, female, pregnant female and newborn. As desrcibed herein, this administration may be via various routes including, intramuscular (IM), intradermal (ID) or subcutaneous (SC) injection or via intranasal or oral administration. The therapeutic composition for use according to the invention can also be administered by a needleless apparatus (as, for example with a Pigjet, Biojector or Vitajet apparatus (Bioject, Oregon, USA)). Another approach to administer plasmid compositions is to use electroporation (see, e.g. S. Tollefsen et al. Vaccine, 2002, 20, 3370-3378; S. Tollefsen et al. Scand. J. Immunol., 2003, 57, 229-238; S. Babiuk et al., Vaccine, 2002, 20, 3399-3408; PCT Application No. WO99/01158). In another embodiment, the therapeutic composition is delivered to the animal by gene gun or gold particle bombardment. As advantageously described herein, the animal is a vertebrate. According to the invention, the vertebrate is a dog.

Desrcibed herein is a method of eliciting an immune response against influenza in an animal, comprising administering a formulation for delivery and expression of a recombinant poxvirus influenza vaccine or inactivated influenza vaccine in an effective amount for eliciting an immune response. Further described herein is a method of inducing an immunological or protective response against influenza in an animal, comprising administering to the animal an effective amount of a formulation for delivery and expression of an influenza antigen, epitope or immunogen wherein the formulation comprises recombinant poxvirus influenza vaccine or inactivated influenza vaccine and a pharmaceutically or veterinarily acceptable carrier, vehicle or excipient.

Desrcibed herein is a method to elicit, induce or stimulate the immune response of an animal, advantageously a vertebrate According to the invention, the vertebrate is a dog but described herein the animal may also be a cat.

Also described herein is a kit for performing a method of inducing an immunological or protective response against influenza in an animal comprising a recombinant influenza poxvirus vaccine or an inactivated influenza vaccine and instructions for performing the method of delivery in an effective amount for eliciting an immune response in the animal.

The invention will now be further described by way of the following non-limiting examples.

### EXAMPLE 1: Vaccination Of Dogs With Canarypox Expressing H3 Genes

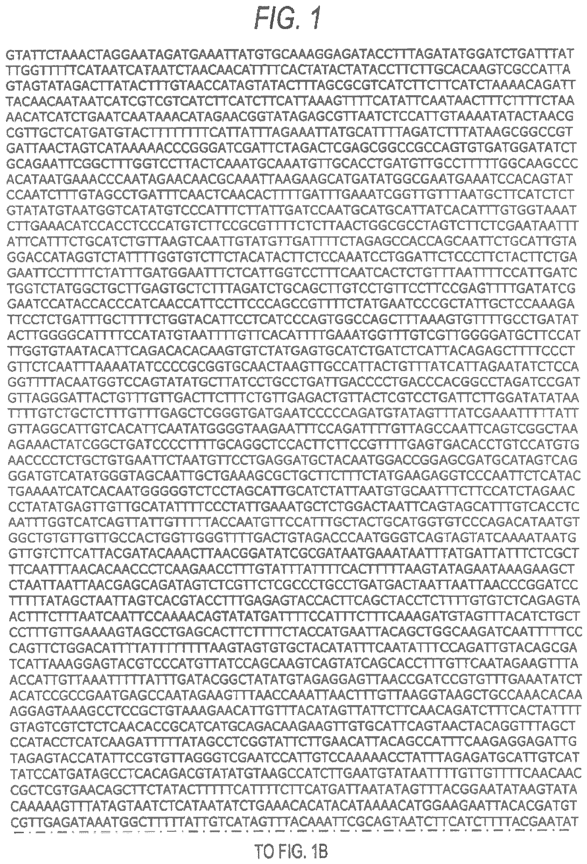

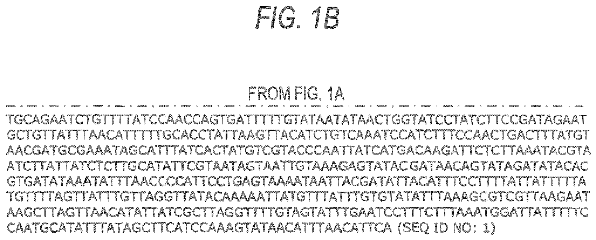

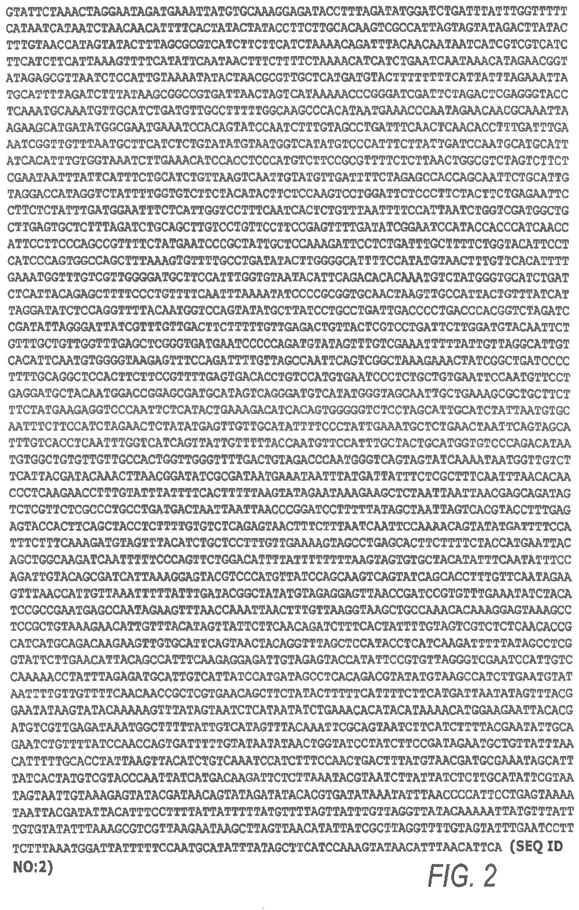

A study was conducted in which dogs were vaccinated dogs on days 0 and 21 with a canarypox expressing H3 genes from Kentucky (CP1529) and Newmarket (CP1533) equine influenza. The construction of CP1529 and CP1533 is described in Examples 1 (c5 locus), 4 and 5 of International Patent Publication WO99/44633,. The nucleotide sequence of the donor plasmids pJT004 and pJT005 are presented in FIGS. 1 and 2.

Sera was collected and tested against H3N8 influenza viruses and the other viruses. As shown in Table 1, canarypox expressing hemagglutinin H3 genes induced a substantial amount of antibodies which specifically reacted with H3N8 strains but not with H1N1 or H7N7. Importantly antibodies were detectable within two weeks after the first immunization.

Accordingly, a canarypox expressing an influenza HA gene is immunogenic in dogs.

**Table 1: Vaccination of dogs with canarypox expressing H3 hemaglutinin genes**

| **Antigen** | **Bleed** | **VACCINATED GROUP** | | | | | **CONTROL GROUP** | | | | |
|---|---|---|---|---|---|---|---|---|---|---|---|
| N/1/93 (H3N8) | D0 | 0 | 0 | 0 | 0 | 0 | 0 | 0 | 0 | 0 | 0 |
| | D14 | 0 | 63.8 | 69.9 | 31.4 | 96.7 | n.s. | n.s. | n.s. | n.s. | n.s. |
| | D21 | 104.1 | 91.4 | 121.5 | 74.6 | 121.5 | n.s. | n.s. | n.s. | n.s. | n.s. |
| | D36 | 106 | 96.7 | 111.7 | 69.9 | 123.6 | n.s. | n.s. | n.s. | n.s. | n.s. |
| | D51 | 76.2 | 55.1 | 74.6 | 38.3 | 69.9 | n.s. | n.s. | n.s. | n.s. | n.s. |
| N/2/93 (H3N8) | D0 | 0 | 0 | 0 | 0 | 0 | 0 | 0 | 0 | 0 | 0 |
| | D14 | 0 | 25.1 | 59.4 | 29.2 | 60.8 | n.s. | n.s. | n.s. | n.s. | n.s. |
| | D21 | 86.2 | 71.4 | 102.2 | 65.3 | 406 | n.s. | n.s. | n.s. | n.s. | n.s. |
| | D36 | 82.8 | 74.6 | 96.7 | 62.3 | 100.4 | n.s. | n.s. | n.s. | n.s. | n.s. |
| | D51 | 52.3 | 37.1 | 66.8 | 26.1 | 57.9 | n.s. | n.s. | n.s. | n.s. | n.s. |
| Pr/56 (H7N7) | D0 | 0 | 0 | 0 | 0 | 0 | 0 | 0 | 0 | 0 | 0 |
| | D14 | 0 | 19.3 | 0 | 24.1 | 0 | n.s. | n.s. | n.s. | n.s. | n.s. |
| | D21 | 0 | 0 | 0 | 0 | 0 | n.s. | n.s. | n.s. | n.s. | n.s. |
| | D36 | 0 | 0 | 0 | 0 | 0 | n.s. | n.s. | n.s. | n.s. | n.s. |
| | D51 | 0 | 0 | 0 | 0 | 0 | n.s. | n.s. | n.s. | n.s. | n.s. |
| PR8 (H1N1) | D0 | 0 | 0 | 0 | 0 | 0 | 0 | 0 | 0 | 0 | 0 |
| | D14 | 0 | 16.7 | 0 | 19.3 | 0 | n.s. | n.s. | n.s. | n.s. | n.s. |
| | D21 | 0 | 0 | 0 | 0 | 0 | n.s. | n.s. | n.s. | n.s. | n.s. |
| | D36 | 0 | 0 | 0 | 0 | 0 | n.s. | n.s. | n.s. | n.s. | n.s. |
| | D51 | 0 | 0 | 0 | 0 | 0 | 0 | 0 | 0 | 0 | 0 |

| | | | | | | | | | | | |
|---|---|---|---|---|---|---|---|---|---|---|---|
| n.s. = no sample | | | | | | | | | | | |

### EXAMPLE 2: Contruction of the donor plasmid pALVAC C5 H6p-synthetic EIV H3 HA, pJY1571.1)

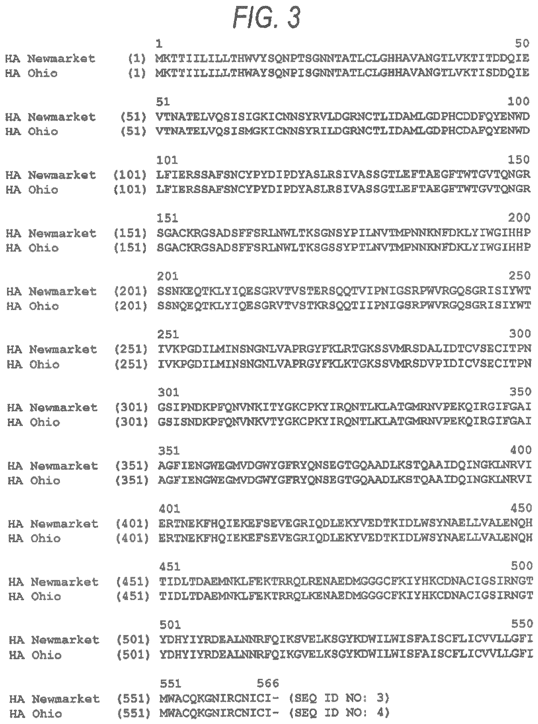

The HA gene was derived from equine influenza virus (EIV) H3N8 Ohio 03 strain isolated from a horse in 2003. The HA gene was synthetic with codon optimization for expression in mammlian cells. The amino acid sequence of EIV Ohio 03 strain HA was compared to that of New Market strain H3 HA and is presented in FIG. 3.

The purpose was the construction of an ALVAC donor plasmid for generation of an ALVAC recombinant expressing codon optimized EIV H3 HA (Ohio 03). The plasmid name was pALVAC C5 H6p-synthetic EIV H3 HA, pJY1571.1. The plasmid backbone is pALVAC C5 H6p. The promoter was a H6 promoter.

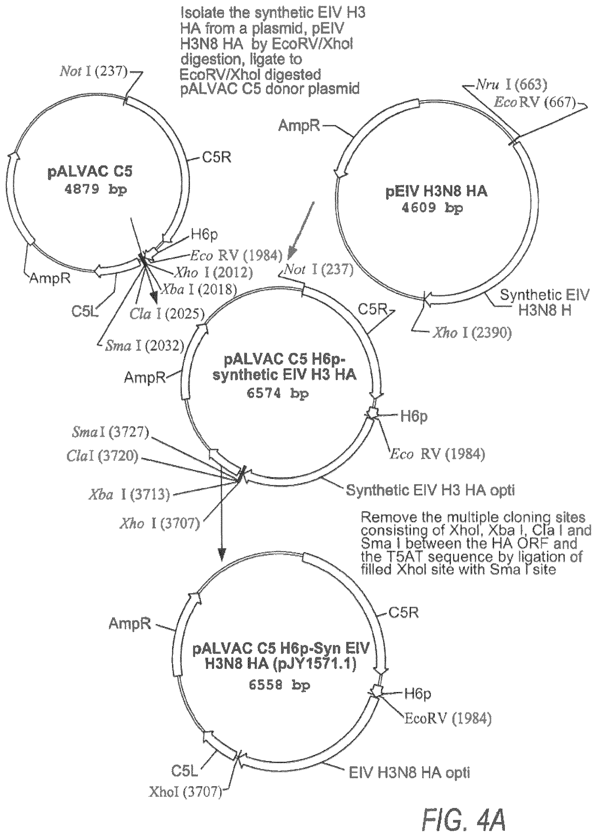

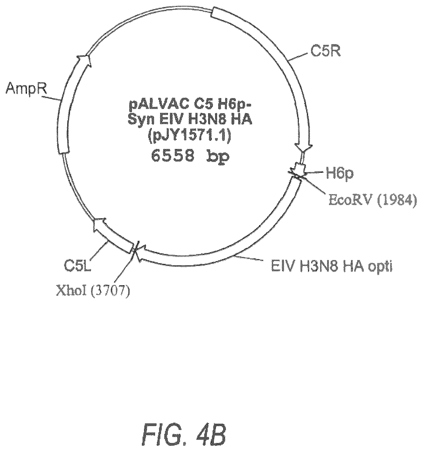

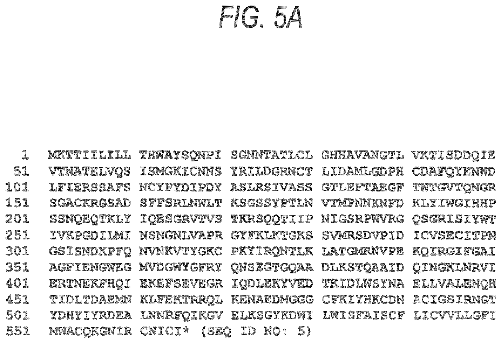

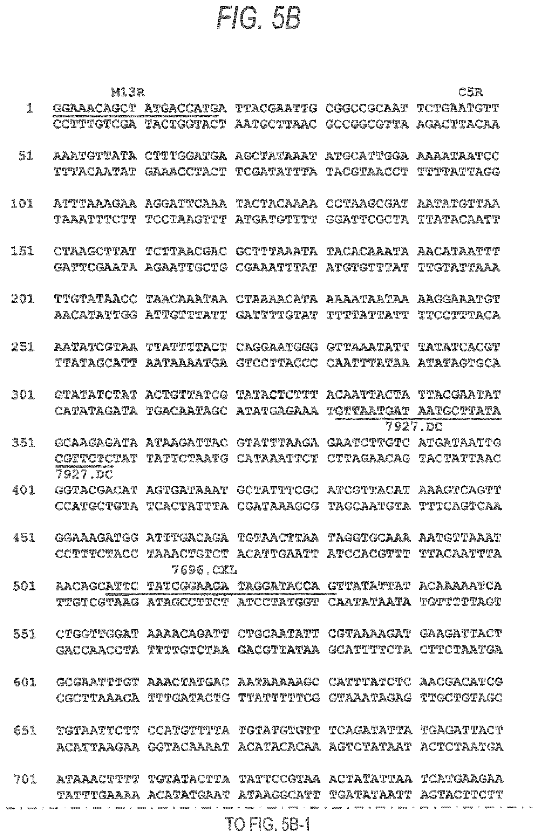

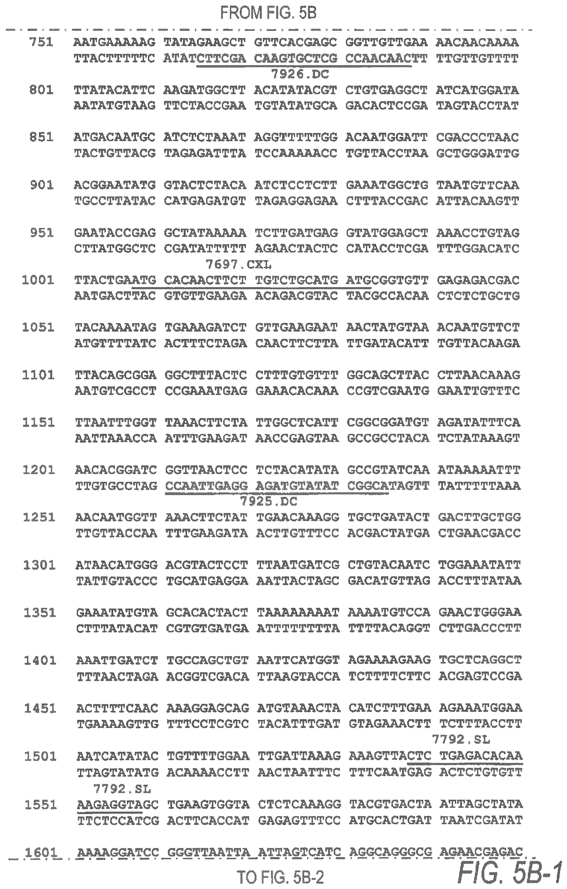

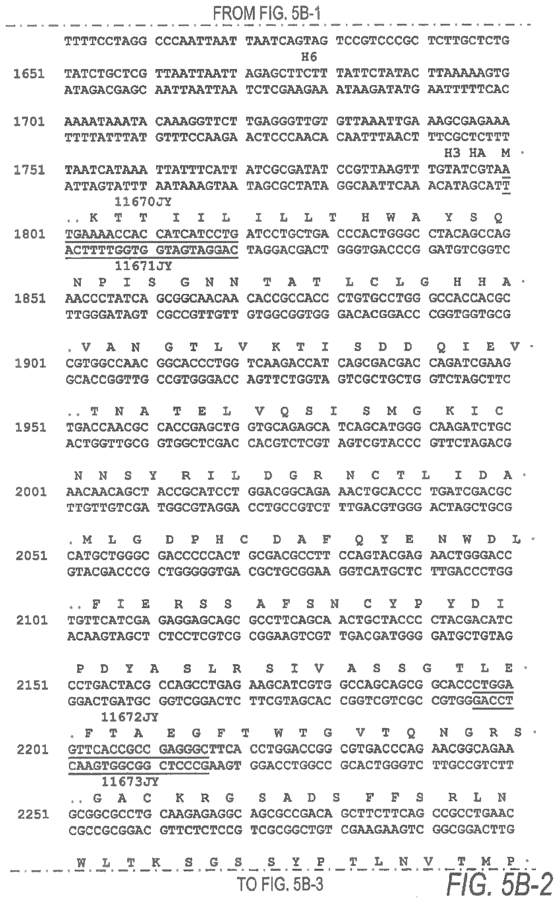

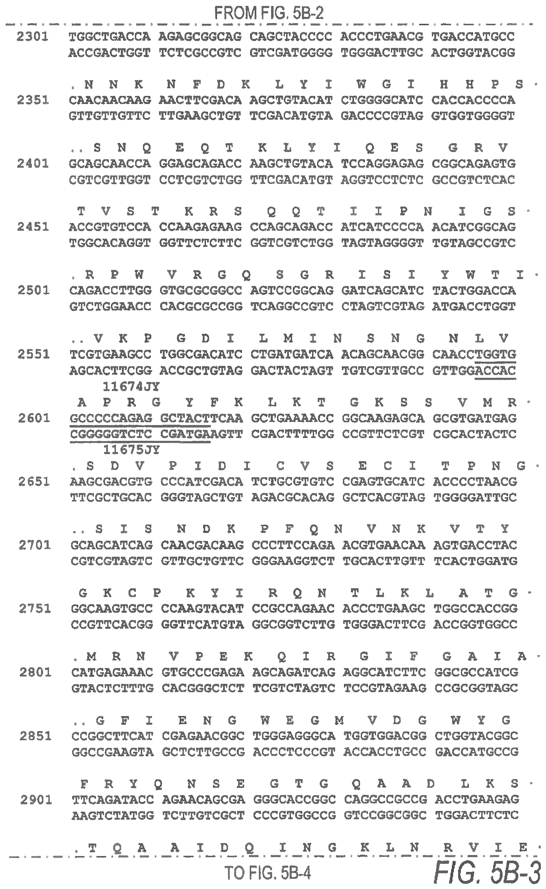

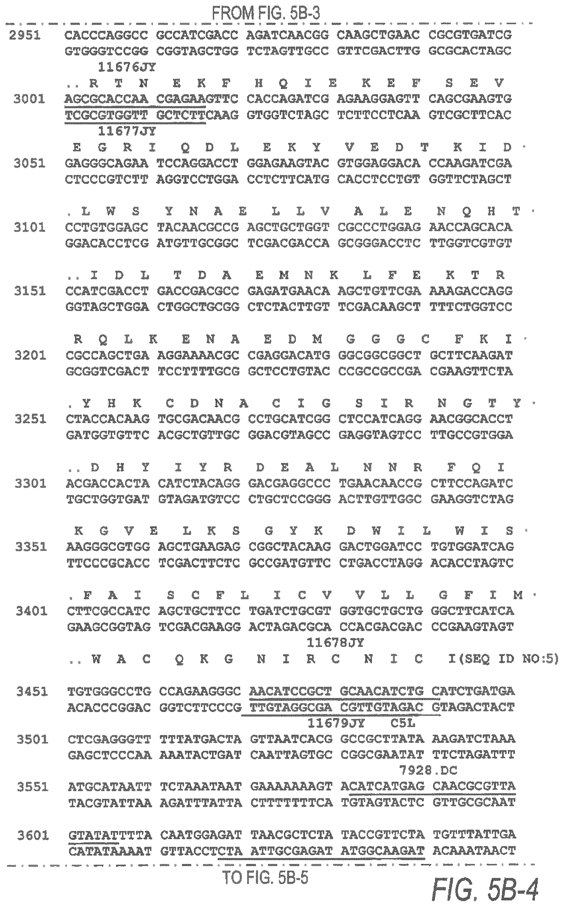

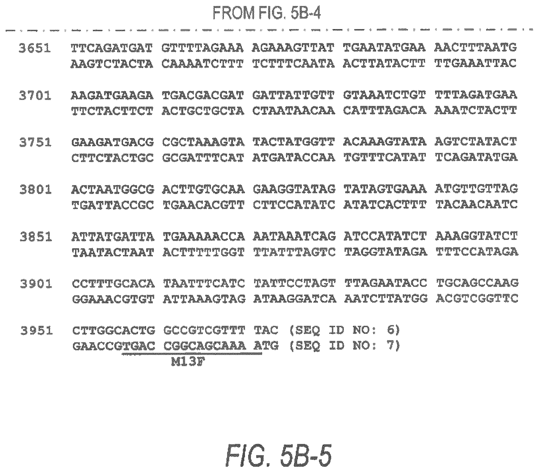

The description of plasmid construction was as follows and presented schematically in FIG. 4A. The synthetic EIV H3 HA (Ohio 03) was isolated from a plasmid pEIV H3N8 HA by EcoRV/Xhol digestion, and ligated to EcoRV/Xhol digested donor plasmid pALVAC C5 H6p to create pALVAC C5 H6p-Synthetic EIV H3 HA. In the resulting plasmid, there are multiple cloning sites consisting of Xhol, Xba I, Cla I and Sma I between the HA ORF and the T5AT sequence which serves as the transcription termination signal. To bring the HA ORF and the T5AT sequence close together, those cloning sites were then subsequently removed by ligation of re-filled Xhol site with Sma 1 site. The resulting plasmid pJY1571.1 was then sequenced and confirmed to contain the correct sequence. A diagram of the resulting plasmid is presented in FIG. 4B. The predicted amino acid sequence of EIV H3 HA is shown in FIG. 5A and the nucleotide sequence of arms and insert with translation is shown in FIG. 5B.

### EXAMPLE 3: Construction of the recombinant canarypox vCP2242

The purpose of this example was the generation and characterization of ALVAC recombinant containing EIV H3N8 codon optimized HA inserted at C5 loci of ALVAC (vCP2242). The parental virus was ALVAC, the donor plasmid was pJY1571.1, the insertion site was a C5 Locus, the promoter was a H6 promoter and cells for in vitro recombination were primary chicken embryo fibroblast cells (1°CEF).

The in vitro recombination (IVR) was performed by transfection of 1°CEF cells with 15 µg of Not I-linearized donor plasmid pJY1571.1. The transfected cells were subsequently infected with ALVAC as rescue virus at MOI of 10. After 24 hr, the transfected-infected cells were harvested, sonicated and used for recombinant virus screening.

Recombinant plaques were screened based on the plaque lift hybridization method using a 821 bp EIV syn HA specific probe labeled with horse radish peroxidase (HRP) according to the manufacturer's protocol. After four sequential rounds of plaque purification, the recombinant designated as vCP2242 was generated and confirmed by hybridization as 100% positive for the EIV syn HA insert and 100% negative for the C5 ORF.

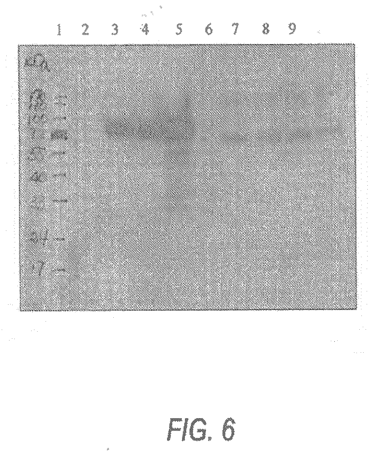

Expression analysis and sequence analysis were performed. Expression analysis was performed by Western blot. Primary CEF cells were infected with vCP2242 stock at MOI of 10 and incubated at 37 C for 26.5 hrs. The cells and culture supernatant were then harvested. Sample proteins were separated on a 10% SDS-PAGE gel, transferred to Immobilon nylon membrane, and probed with a pool of monoclonal mouse anti-EIV HA antibodies (anti Eq/AK/91: 124-1D9-1, 124-3E3-3, 124-4F3-2 and H3N8 A Eq/miami/63 pool at 1/1,000 dilution). Peroxidase-conjugated goat anti-mouse antiserum was used as a secondary antibody and the bands were visualized using luminol reagents. vCP2242 showed a protein expression profile with a 80 kDa protein expressed in cell pellet, but not in the culture medium as presented in FIG. 6.

Results of the sequence analysis demonstrated that the sequences of the EIV syn HA and C5L and C5R of ALVAC were correct.

### EXAMPLE 4: Kentucky Equine Influenza Nucleotide Sequences (Preparative Example)

DEFINITION Influenza A virus (A/equine/Kentucky/5/02(H3N8)) nonstructural protein gene, complete cds.
   ACCESSION AY855345; SEQ ID NO: 8
DEFINITION Influenza A virus (A/equine/Kentucky/5/02(H3N8)) matrix protein gene, complete cds.
   ACCESSION AY855344; SEQ ID NO: 9
DEFINITION Influenza A virus (A/equine/Kentucky/5/02(H3N8)) neuraminidase gene, complete cds.
   ACCESSION AY855343; SEQ ID NO: 10
DEFINITION Influenza A virus (A/equine/Kentucky/5/02(H3N8)) nucleoprotein gene, complete cds.
   ACCESSION AY855342; SEQ ID NO: 11
DEFINITION Influenza A virus (A/equine/Kentucky/5/02(H3N8)) hemagglutinin precursor, gene, complete cds.
   ACCESSION AY855341; SEQ ID NO: 12
DEFINITION Influenza A virus (A/equine/Kentucky/5/02(H3N8)) PA polymerase gene, complete cds.
   ACCESSION AY855340; SEQ ID NO: 13
DEFINITION Influenza A virus (A/equine/Kentucky/5/02(H3N8)) PB1 polymerase 1 gene, complete cds.
   ACCESSION AY855339; SEQ ID NO: 14
DEFINITION Influenza A virus (A/equine/Kentucky/5/02(H3N8)) PB2 polymerase 2 gene, complete cds.
   ACCESSION AY855338; SEQ ID NO: 15
DEFINITION Influenza A virus (A/equine/Kentucky/1/91(H3N8)) hemagglutinin precursor (HA) gene, complete cds.
   ACCESSION L39918; SEQ ID NO: 16
DEFINITION Influenza A virus (A/equine/Kentucky/1/92(H3N8)) hemagglutinin precursor (HA) gene, complete cds.
   ACCESSION L39917; SEQ ID NO: 17
DEFINITION Influenza A virus (A/equine/Kentucky/1/90(H3N8)) hemagglutinin precursor (HA) gene, complete cds.
   ACCESSION L39915; SEQ ID NO: 18
DEFINITION Influenza A virus (A/equine/Kentucky/1/94(H3N8)) hemagglutinin precursor (HA) gene, complete cds.
   ACCESSION L39914; SEQ ID NO: 19
DEFINITION Influenza A virus (A/equine/Kentucky/1/81(H3N8)) nucleoprotein (NP) gene, complete cds.
   ACCESSION AY291288; SEQ ID NO: 20
DEFINITION Influenza A virus (A/Equine/Kentucky/1/92(H3N8)) gene for hemagglutinin precursor, partial cds.
   ACCESSION D30683; SEQ ID NO: 21
DEFINITION Influenza A virus (A/equine/Kentucky/1/97(H3N8)) hemagglutinin precursor (HA1) mRNA, partial cds.
   ACCESSION AF197249; SEQ ID NO: 22
DEFINITION Influenza A virus (A/equine/Kentucky/1/96(H3N8)) hemagglutinin precursor (HA1) mRNA, partial cds.
   ACCESSION AF197248; SEQ ID NO: 23
DEFINITION Influenza A virus (A/equine/Kentucky/9/95(H3N8)) hemagglutinin precursor (HA1) mRNA, partial cds.
   ACCESSION AF197247; SEQ ID NO: 24
DEFINITION Influenza A virus (A/equine/Kentucky/1/98(H3N8)) hemagglutinin precursor (HA1) mRNA, partial cds.
   ACCESSION AF197241; SEQ ID NO: 25
DEFINITION Influenza A virus (A/eq/Kentucky/81(H3N8)) hemagglutinin mRNA, complete cds.
   ACCESSION U58195; SEQ ID NO: 26
DEFINITION Influenza A virus (A/equine/Kentucky/2/86 (H3N8)) membrane protein M1 and membrane protein M2 genes, complete cds.
   ACCESSION M63540; SEQ ID NO: 27
DEFINITION Influenza A virus isolate A/equine/Kentucky/76 nonstructural protein, complete cds.
   ACCESSION M80971; SEQ ID NO: 28
DEFINITION Influenza A virus (A/eq/Kentucky/92(H3N8)) matrix proteins M1 and M2 (M) gene, complete cds.
   ACCESSION AF001683; SEQ ID NO: 29
DEFINITION Influenza A virus (A/eq/Kentucky/81(H3N8)) matrix proteins M1 and M2 (M) gene, complete cds.
   ACCESSION AF001676; SEQ ID NO: 30
DEFINITION Influenza A virus (A/eq/Kentucky/92(H3N8)) nonstructural proteins NS1 and NS2 (NS) gene, complete cds.
   ACCESSION AF001671; SEQ ID NO: 31
DEFINITION Influenza A virus (A/eq/Kentucky/1/88(H3N8)) nonstructural proteins NS1 and NS2 (NS) gene, complete cds.
   ACCESSION AF001664; ; SEQ ID NO: 32
DEFINITION Influenza A/equine/Kentucky/2/86 (H3N8) nucleoprotein (seg 5) mRNA, complete cds.
   ACCESSION M30751; SEQ ID NO: 33
DEFINITION Influenza A/Equine/Kentucky/2/86 (H3N8), PB2 polymerase, complete cds.
   ACCESSION M73526 M36049; SEQ ID NO: 34
DEFINITION Influenza A/equine/Kentucky/1/87 (H3N8) hemagglutinin (HA) RNA (seg. 4), complete cds.
   ACCESSION M24728 J04336; SEQ ID NO: 35
DEFINITION Influenza A/equine/Kentucky/2/86 (H3N8) hemagglutinin (HA) RNA (seg. 4), complete cds.
   ACCESSION M24727 J04336; SEQ ID NO: 36

### EXAMPLE 5: Newmarket Equine Influenza Nucleotide Sequences (Preparative Example)

DEFINITION Influenza A virus (A/equine 2/Suffolk/89(H3N8)) NS1 gene.
   ACCESSION X80060; SEQ ID NO: 37
DEFINITION Influenza A virus (A/eq/Newmarket/93/(H3N8)) HAI gene for HAY subunit of haemagglutinin, genomic RNA.
   ACCESSION X85089; SEQ ID NO: 38
DEFINITION Influenza A virus (A/eq/Newmarket/93/(H3N8)) HAI gene for HAY subunit of haemagglutinin, genomic RNA.
   ACCESSION X85088; SEQ ID NO: 39
DEFINITION Influenza A virus (A/eq/Sussex/89/(H3N8)) HAI gene for HAY subunit of haemagglutinin, genomic RNA.
   ACCESSION X85090; SEQ ID NO: 40
DEFINITION Influenza A virus (A/eq/Lambourn/92/(H3N8)) HAI gene for HAY subunit of haemagglutinin, genomic RNA.
   ACCESSION X85087; SEQ ID NO: 41
DEFINITION Influenza A virus (A/eq/Ella/89/(H3N8)) HAI gene for HAY subunit of haemagglutinin, genomic RNA.
   ACCESSION X85086; SEQ ID NO: 42
DEFINITION Influenza A virus (A/eq/Arundel/91/(H3N8)) HAI gene for HAY subunit of haemagglutinin, genomic RNA.
   ACCESSION X85085; SEQ ID NO: 43
DEFINITION Influenza A virus (A/equine/Suffolk/89(H3N8)) HA gene for haemagglutinin.
   ACCESSION X68437; SEQ ID NO: 44
DEFINITION Influenza A virus (A/equine/Newmarket/1/77(H7N7)) gene for haemagglutinin.
   ACCESSION X62554; SEQ ID NO: 45
DEFINITION Influenza A virus HA partial gene for haemagglutinin, genomic RNA, strain A/equine/Newmarket-Bob Champion/89(H3N8).
   ACCESSION AJ223193; SEQ ID NO: 46
DEFINITION Influenza A virus (A/Equine/NewMarket/D64/79(H3N8)) gene for hemagglutinin precursor, partial cds.
   ACCESSION D30677; SEQ ID NO: 47
DEFINITION Influenza A virus (A/eq/Newmarket/1/77(H7N7)) matrix proteins M1 and M2 (M) gene, complete cds.
   ACCESSION AF001686; SEQ ID NO: 48
DEFINITION Influenza A virus (A/eq/Newmarket/79(H3N8)) matrix proteins M1 and M2 (M) gene, complete cds.
   ACCESSION AF001675; SEQ ID NO: 49
DEFINITION Influenza A virus (A/eq/Newmarket/1/77(H7N7)) nonstructural proteins NS1 and NS2 (NS) gene, complete cds.
   ACCESSION AF001663; SEQ ID NO: 50
DEFINITION Influenza A virus (A/eq/Newmarket/D63/79(H3N8)) nonstructural proteins NS1 and NS2 (NS) gene, complete cds.
   ACCESSION AF001662; SEQ ID NO: 51

### EXAMPLE 6: Efficacy of inactivated influenza virus vaccine in canines (Non-illustrative of the invention).

Vaccine active ingredient (AI) is produced either in 9-11 day-old embryonated chicken eggs or in continuous cell cultures such as MDCK cells.

Egg Production, Harvest and Purification. Vaccine AI production in embryonating chicken eggs is prepared by inoculation 0.1 - 0.25 ml of virus suspension containing 100-1000 EID50 (egg infectious dose) into the allantoic cavity. Allantoic fluid is harvested after incubation at 33-37 °C for 2-6 days and pooled. The virus pool is clarified by centrifugation at 1500 - 4000g for 5-15 minutes and filtered through a 20 µm filter (1). The virus maybe concentrated by ultra-filtration using a 300 KDa membrane with a maximun concentration factor of 20 (2).

Further the concentrated virus is optionally purified by zonal ultra-centrifugation on a sucrose gradient followed by filtration of the virus containing fraction through a 0.5 µm membrane. This step can be repeated. The virus containing fraction is diluted in a suitable buffer such as a phosphate buffer (3).

Further purification consists of treatment with Tween/ether at 0.1 % final concentration (w/v)) and 50% final concentration (w/w), respectively. The resulting solution is centrifuged and the aqueous phase is collected. This step can be repeated. The aqueous phase is treated with nitrogen to remove residual ether (4).

Cell Culture Production, Harvest and Purification. Active ingredient is prepared in cells (e.g. MDCK, BHK, PerC6 cells) by inoculation at a rate of MOI of 0.001-1 TCID50. Culture is harvested after incubation at 35-37C for 48-96 hrs when cytopathic effect (CPE) is approximately 50-90%. Culture is sonicated and centrifuged at 1500-4000g for 5-15 minutes (1). The virus is concentrated by ultra-filtration using a 300 KD membrane with a max. concentration factor of 20 (2).

The virus is optionally purified by exclusion chromatography (Sepharose 6 fast flow)(3).

Inactivation. Virus (fraction 1, 2, 3 or 4) are inactivated by addition of formalin or beta-propiolactone to a final concentration of 0.05 % (w/v) or 0.01 % (w/v), respectively and the suspension is held respectively at 5 °C for 16-18 hours or at 20°C for 25-36 hours with continuous agitation.

Active ingredient inactivation is confirmed by inoculation into embryonated chicken eggs or onto MDCK cells.

Preparation of Final Vaccine. Vaccine consists of the inactivated influenza virus and an adjuvant such as aluminium hydroxide, aluminum phosphate, Carbomer or oil-in-water emulsion, which may contain other immunostimulating components such as CpG (1-100 ug/dose). Vaccine typically contains 15-50 µg of hemagglutinin measured by single radial-immunodiffusion (SRD) or >400 haemagglutination Units

The vaccine is administered either by subcutaneous or intramuscular route. Two doses are administered at an interval of 2-5 weeks to pups from 4 weeks of age. Duration of immunity: 6-12 months after a primary vaccination course.

Vaccination/Challenge Protocol. Dogs (less than 4 weeks old) are vaccinated with 2 doses of inactivated vaccine at 3-4 weeks interval via the subcutaneous route. Blood samples are collected on the day of the first and second vaccination and 2-4 weeks after second vaccination to determine the levels of anti-influenza virus specific antibodies using virus neutralization, hemagglutination inhibition, ELISA or Single Radial Heamolysis (SRH) tests.

Two to 4 weeks after 2^{nd} immunization dogs are challenged with a virulent strain of influenza H3N8 to determine efficacy of the vaccines.

Vaccinated and control dogs are challenged by the following route:
By spray: animals are placed into an enclosed container or a mask is placed on the nose of the dogs and the virus suspension is aerosolized to generate 1-100 µM droplets. Animals are kept in the aerosolization chambers for 30 minutes to allow inhalation of the aerosol.

Alternativelly thy can be challenged by one the following routes:
Intra-nasally: 0.5 ml containing 10⁵⁻⁸ EID50 of challenge virus is dropped to each nostril by the aid of dropper.

Intra-tracheally: 1-2 ml containing 10⁵⁻⁸ EID₅₀ virus is directly instilled into the trachea.

Orally: 5- 10 ml containing 10⁵⁻⁸ EID50 of challenge virus is orally administered.

Animals are observed daily for 14 days following challenge for clinical signs including fever, cough, nasal, ocular discharge, respiratory distress, anorexia and lethargy. In addition, groups of animals are euthanized and evaluated for pathological findings of pulmonary and pleural hemorrhage, tracheitis, bronchitis, broncholilitis, and bronchopneumonia.

Tracheal swabs are collected from all animals post challenge days 1-14 for virus isolation.

Presence or absence of viral antigens in respiratory tissues is evaluated by immunohistochemistry on days 3, 7 and 10 post-challenge.

Blood samples are collected on days 7 and 14 post-challenge and are analyzed for the presence of anti-influenza H3N8 virus specific antibody.

### EXAMPLE 7: Vaccination of Dogs with Equine Influenza Vaccines to Induce a Humoral Immune Response Against CIV

A study was conducted in which 35dogs were randomly allocated into four groups of 9, 9, 9 and 8 dogs. None of the dogs had detectable antibodies to CIV, as determined by HI tests (below) at the commencement of the study. One group was immunized with vCP1529 (canarypox recombinant expressing HA gene from Kentucky equine influenza, A/eq/Kentucky/94) 10^{5.7} TCID₅₀/ 1 ml dose, which was administered subcutaneously, and two groups were immunized with vCP2242 (canarypox recombinant expressing HA gene from A/eq/Ohio/03), which was administered to one group subcutaneously (10^{5.7} TCID₅₀/ 1 ml dose) and to one group transdermally (10^{5.7} TCID₅₀/ 0.25 ml dose). Vaccinates received 2 doses of vaccine on Day 0 and Day 28. The fourth group was unvaccinated and served as a control. All dogs were examined for local reactions at the site of inoculation and for general clinical signs.

Blood samples were collected for serology on days -1, 7, 13 , 21, 28, 35, and 42 for all groups. A hemagglutination inhibition (HI) test was performed as described previously (Karaca et al., 2007). Briefly, sera were separated and were heat inactivated (56°C for 30 minutes). Sera were then treated with potassium periodate followed by incubation with turkey RBCs to remove nonspecific HAs and inhibitors. Four hemagglutination units of antigen were added to serial 2-fold dilutions of treated sera and incubated at 18° to 22°C for 30 minutes. Turkey RBCs were added, and plates were incubated for an additional 30 minutes. Wells were then examined for evidence of hemagglutination. The lowest dilution of serum tested was 1:8, and antibody titers corresponding to the reciprocal of the highest dilution that inhibited hemagglutination were presented as geometric mean titers (GMTs).

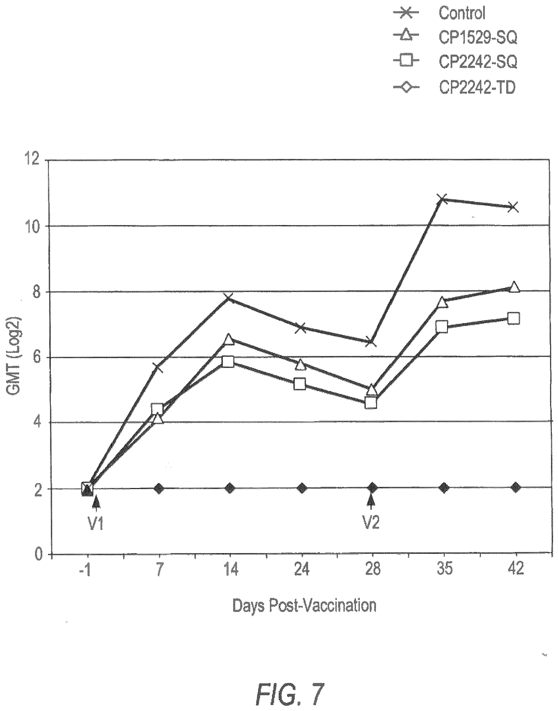

All dogs tolerated the inoculations well. None of the dogs had a systemic or local adverse reaction, regardless of the inoculum or route of administration. Antibody responses of dogs as measured by HI tests are shown in FIG. 7. On day -1, all dogs were seronegative (antibody titres <8). Control dogs remained seronegative throughout the duration of the study. All inoculated dogs had detectable antibodies against CIV on days 13 , 21, and 28 after the initial inoculation. Although there was no significant difference in antibody titers between subcutaneously inoculated groups, antibody titres of the transdermally inoculated group were significantly higher than the others after the first and second inoculations. (repeated measures ANOVA, p<0.01).

### EXAMPLE 8: Vaccination of Dogs with Equine Influenza Vaccines and challenge with a virulent Canine Influenza Isolate.

A study was conducted in which 33 dogs were randomly allocated to one of two groups. Dogs of group 1 (n=16) were not inoculated and served as a control. Dogs of group 2 (n=17) were inoculated subcutaneously on day 0 and day 28 with 10^{5.7} TCID₅₀ of CP2242 (canarypox recombinant expressing HA gene from A/eq/Ohio/03). All dogs were challenged on day 43 with 10^{7.0} TCID₅₀ ofCIV-NY05 first by syringe 0.5ml directly into the nares followed with challenge by aerosolization into a closed chamber for 5-15 minutes, with oxygen supplied using a commercially available nebulizer. All dogs were clinically observed pre and post challenge according to the timeline in Table 2.

**Table 2: Timeline**

| | | |
|---|---|---|
| **(VI = virus isolation; Bb = *Bordetella bronchiseptica* isolation)** | | |

| **Study Day** | **DPC*** | **ACTIVITY** |
|---|---|---|
| D-4 | N/ A | Baseline injection site observations |
| | | Nasal swab for VI and *Bb* |
| | | Baseline Blood for serology |
| D0 | | Vaccination # 1 |
| D1-4 | | Injection site observations |
| D7 | | Injection site observations |
| | | Weigh |
| D16-18 | | Baseline Injection site observations |
| D21 | | Baseline Injection site observations |
| | | Weigh |
| | | Nasal swabs for VI and *Bb* |
| | | Baseline Blood Collection for serology |
| | | Vaccination # 2 |
| D22-25 | | Injection site observations |
| D28 | | Injection site observations |
| | | Baseline Clinical observations |
| | | Nasal swabs for VI and *Bb* |
| | | Baseline Blood Collection for serology |
| D42 | | Baseline Clinical Observations (Weigh) |
| | | Nasal swabs for VI and *Bb* |
| | | Baseline Blood collection for serology |
| D43 | | Challenge |
| D44-48 | 1-5 | Clinical observations |
| | | Nasal swabs for VI and *Bb* |
| D49 | 6 | Clinical observations (Weigh) |
| | | Nasal swabs for VI and *Bb* |
| D50 | 7 | Clinical observations |
| | | Nasal swabs for VI and Bb |
| | | Blood for serology |
| D52-53 | 9-10 | Clinical observations |
| | | Nasal swabs for VI and Bb |
| D57 | 14 | Clinical observations (Weigh) |
| | | Nasal swabs for VI and Bb |
| | | Blood for serology |
| | | Euthanasia- End of Study |

| | | |
|---|---|---|
| * Days Post-Challenge | | |

The clinical signs that were recorded as absent or present include cough (spontaneous or inducible by mild tracheal palpation), ocular/nasal discharge (serous or purulent), rectal temperature (°F), depression/lethargy, dyspnea, and weight loss.

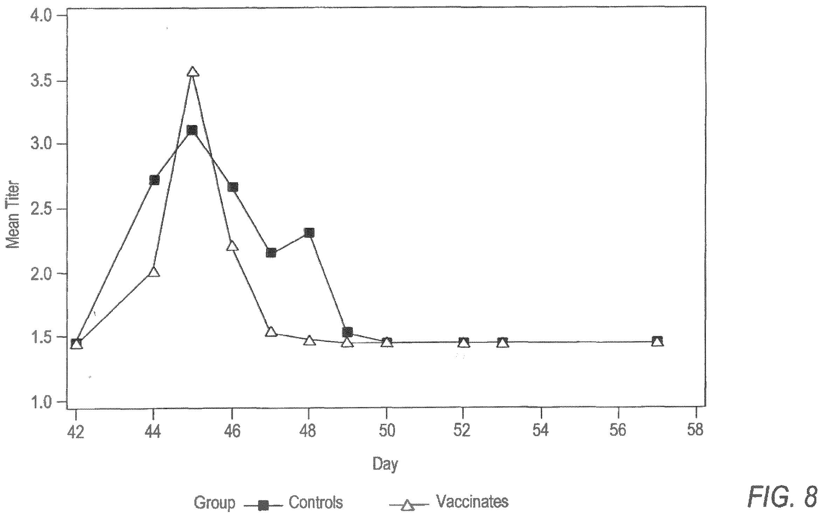

All dogs were evaluated for CIV viral load, body temperature, HI antibody responses to CIV HA antigen, and virus shedding. To determine CIV viral loads, nasal swabs were collected from all animals on days -4, 21, 28, and 42 pre-challenge samples and on days 44-50, 52, 53 and 57 (1-7, 9, 10 and 14 days post-challenge). Levels of virus were determined using a virus titration assay on Madine Darby canine kidney (MDCK) cells and the titer results were expressed as TCID₅₀. FIG. 8 illustrates describes the mean viral titers from swab extracts in controls and vaccinates. In this example, a TCID₅₀ of 1.45 is the minimum detectable titer, therefore a titer of 1.45 = no virus detected. The vaccinated animals stop shedding at Day 47 whereas control animals shed until Day 49. Controls had a significantly higher titer than vaccinates on Day 44 (p<0.0001), Day 46 (p=0.0016), Day 47 (p=0.0027) and Day 48 (p<0.0001).

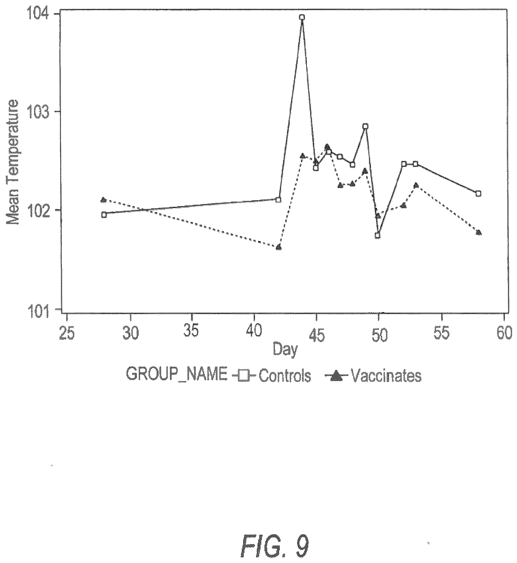

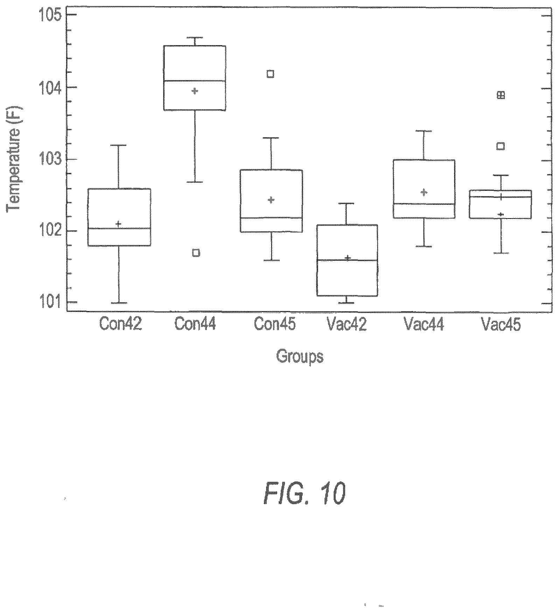

Body temperatures of dogs were evaluated throughout the study. The mean body temperatures of dogs vaccinated with vCP2242 were lower than the mean body temperatures of control dogs (FIGS. 9 and 10).

FIG. 9 shows the mean temperature over time from Day 28 to Day 57. Controls show an increase from a ∼102oF baseline to ∼104oF, 24 hours after challenge whereas vaccinate temperature increase from 102oF to ∼102.5oF (statistically significant difference on that day with p<0.0001). Controls also have a significantly higher mean body temperature on day 49 (p=0.048).

FIG. 10 is a boxplot for Days 42 (1 day pre-challenge), 44 and 45 (1 and 2 days post-challenge).

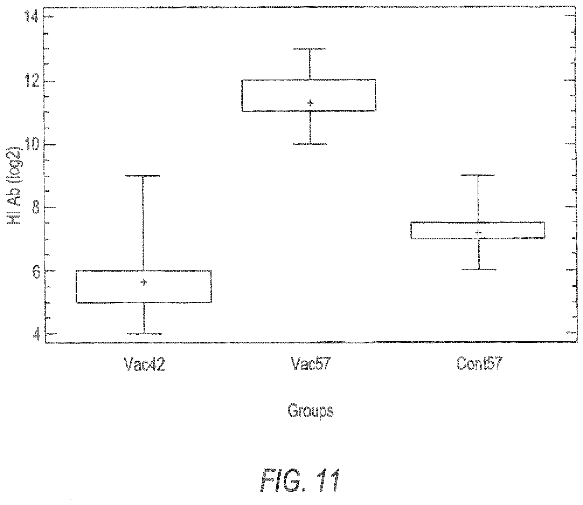

FIG. 11 shows the HI titers on Day 42 (1 day pre-challenge) and Day 57 (14 days post-challenge). A virulent CIV-NY05 challenge boosts the HI titers of dogs vaccinated with vCP2242. Although unvaccinated animals seroconverted after challenge, their mean Day 57 HI titer was significantly lower than vaccinates. This is an evidence of the efficient priming provided by the vaccine.

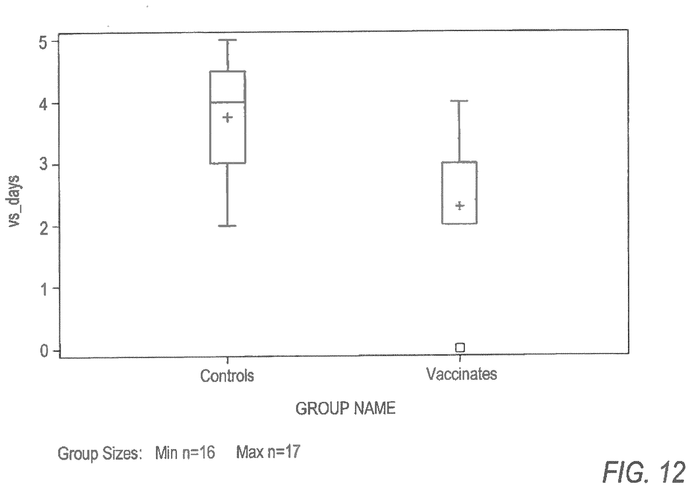

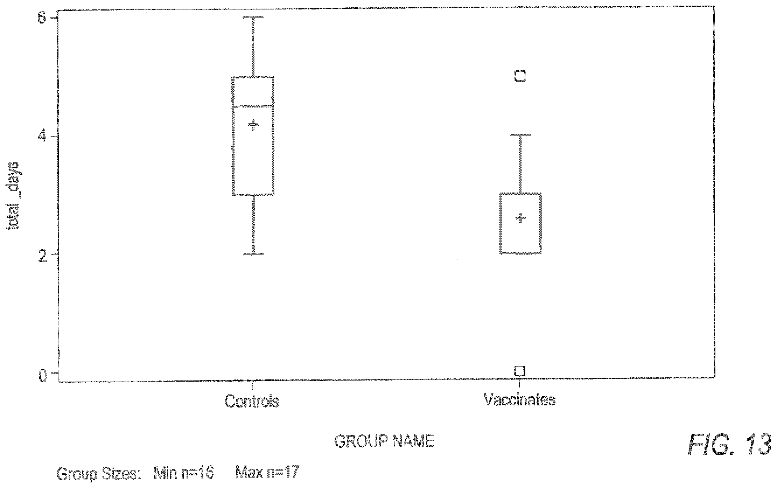

On average, dogs vaccinated with with HA antigen of A2/equine/Ohio/03 (vCP2242) followed by challenge at day 43 with CIV-NY05 isolate demonstrated a decreased number of shedding days as compared to control dogs (FIG. 12) as well as a decreased duration of virus shedding in the nasal cavity (FIG. 13).

FIG. 12 show the number of shedding days illustrating the decrease in number of shedding days in the vaccinates vs. controls.

FIG. 13 shows the decrease in the duration of virus shedding in the vaccinates.

## Claims

1. A *Canidae* vaccine for use in reducing influenza viral shedding in a canine, wherein said vaccine comprises an effective amount of an expression vector that contains and expresses *in vivo* in a canine a polynucleotide encoding equine influenza antigen comprising equine influenza H3 operatively linked to a promoter and optionally to an enhancer , wherein the expression vector is a poxvirus, and a pharmaceutically or veterinarily acceptable carrier, excipient, or vehicle.

2. The vaccine for the use of claim 1, wherein the vaccine further comprises an adjuvant.

3. The vaccine for the use of claim 2, wherein the adjuvant is a cationic lipid containing a quaternary ammonium salt.

4. The vaccine for the use of any one of claims 1 to 3, wherein the vaccine is formulated for subcutaneous or intramuscular administration.

5. The vaccine for the use of claim 1, wherein the polynucleotide comprises H3N8 codon optimized HA.

6. The vaccine for the use according to claim 1 or 5, wherein the polynucleotide is isolated from a canine infected with equine influenza.

7. The vaccine for the use according to claim 1 or 5, wherein the polynucleotide is isolated from an equine influenza virus isolate.

8. The vaccine for the use according to claim 7, wherein the equine influenza isolate is selected from the group consisting of an Ohio equine influenza virus isolate, a Kentucky equine influenza virus isolate, a Newmarket equine influenza virus isolate or mixtures thereof.

9. The vaccine for the use according to any of claims 1-8, wherein the poxvirus is an avipox virus.

10. The vaccine for the use according to claim 9, wherein the avipox virus is a canarypox virus.

11. The vaccine for the use according to claim 10, wherein the canarypox virus is ALVAC.

12. The vaccine for the use according to any one of claims 1 to 11 wherein said vaccine is for use in reducing the duration of influenza viral shedding.

13. The vaccine for the use according to any one of claims 1 to 11 wherein said vaccine is for use in reducing influenza viral shedding in the nasal cavity.

## Patentansprüche

1. *Canidae*-Impfstoff zur Verwendung beim Reduzieren der Influenza-Virenfreisetzung in einem Hund, wobei der Impfstoff eine wirksame Menge eines Expressionsvektors umfasst, der *in vivo* in einem Hund ein Polynucleotid enthält und exprimiert, das ein Pferde-Influenza-Antigen codiert, das Pferde-Influenza H3 umfasst, das mit einem Promotor und gegebenenfalls mit einem Enhancer funktionell verknüpft ist, wobei der Expressionsvektor ein Pockenvirus ist, und einen pharmazeutisch oder veterinärmedizinisch verträglichen Träger, Exzipienten oder ein pharmazeutisch oder veterinärmedizinisch verträgliches Vehikel umfasst.

2. Impfstoff zur Verwendung nach Anspruch 1, wobei der Impfstoff des Weiteren ein Adjuvans umfasst.

3. Impfstoff zur Verwendung nach Anspruch 2, wobei das Adjuvans ein kationisches Lipid ist, das quartäres Ammoniumsalz enthält.

4. Impfstoff zur Verwendung nach einem der Ansprüche 1 bis 3, wobei der Impfstoff zur subkutanen oder intramuskulären Verabreichung formuliert ist.

5. Impfstoff zur Verwendung nach Anspruch 1, wobei das Polynucleotid H3N8-Codon-optimiertes HA umfasst.

6. Impfstoff zur Verwendung nach Anspruch 1 oder 5, wobei das Polynucleotid aus einem Hund isoliert ist, der mit Pferde-Influenza infiziert ist.

7. Impfstoff zur Verwendung nach Anspruch 1 oder 5, wobei das Polynucleotid aus einem Pferde-Influenza-Virus-Isolat isoliert ist.

8. Impfstoff zur Verwendung nach Anspruch 7, wobei das Pferde-Influenza-Isolat ausgewählt ist aus der Gruppe bestehend aus einem Ohio-Pferde-Influenza-Virus-Isolat, einem Kentucky-Pferde-Influenza-Virus-Isolat, einem Newmarket-Pferde-Influenza-Virus-Isolat oder Gemischen davon.

9. Impfstoff zur Verwendung nach einem der Ansprüche 1 bis 8, wobei der Pockenvirus ein Avipoxvirus ist.

10. Impfstoff zur Verwendung nach Anspruch 9, wobei das Avipoxvirus ein Kanarienpockenvirus ist.

11. Impfstoff zur Verwendung nach Anspruch 10, wobei das Kanarienpockenvirus ALVAC ist.

12. Impfstoff zur Verwendung nach einem der Ansprüche 1 bis 11, wobei der Impfstoff zur Verwendung beim Reduzieren der Dauer der Influenza-Virenfreisetzung ist.

13. Impfstoff zur Verwendung nach einem der Ansprüche 1 bis 11, wobei der Impfstoff zur Verwendung beim Reduzieren der Influenza-Virenfreisetzung in der Nasenhöhle ist.

## Revendications

1. Vaccin pour *Canidae* pour une utilisation dans la réduction de l'excrétion du virus de la grippe chez un canidé, dans lequel ledit vaccin comprend une quantité efficace d'un vecteur d'expression qui contient et exprime *in vivo* chez un canidé un polynucléotide codant pour un antigène de grippe équine comprenant un H3 de grippe équine lié de manière fonctionnelle à un promoteur et facultativement à un amplificateur, dans lequel le vecteur d'expression est un poxvirus, et un support, excipient ou véhicule acceptable dans le domaine pharmaceutique ou vétérinaire.

2. Vaccin pour l'utilisation selon la revendication 1, dans lequel le vaccin comprend en outre un adjuvant.

3. Vaccin pour l'utilisation selon la revendication 2, dans lequel l'adjuvant est un lipide cationique contenant un sel d'ammonium quaternaire.

4. Vaccin pour l'utilisation selon l'une quelconque des revendications 1 à 3, dans lequel le vaccin est formulé pour une administration sous-cutanée ou intramusculaire.

5. Vaccin pour l'utilisation selon la revendication 1, dans lequel le polynucléotide comprend une HA du H3N8 codon-optimisée..

6. Vaccin pour l'utilisation selon la revendication 1 ou 5, dans lequel le polynucléotide est isolé à partir d'un canidé infecté par la grippe équine.

7. Vaccin pour l'utilisation selon la revendication 1 ou 5, dans lequel le polynucléotide est isolé à partir d'un isolat de virus de grippe équine.

8. Vaccin pour l'utilisation selon la revendication 7, dans lequel l'isolat de grippe équine est sélectionné dans le groupe constitué d'un isolat de virus de grippe équine de l'Ohio, d'un isolat de virus de grippe équine du Kentucky, d'un isolat de virus de grippe équine de Newmarket ou de mélanges de ceux-ci.

9. Vaccin pour l'utilisation selon l'une quelconque des revendications 1 à 8, dans lequel le poxvirus est un virus avipox.

10. Vaccin pour l'utilisation selon la revendication 9, dans lequel le virus avipox est un virus canarypox.

11. Vaccin pour l'utilisation selon la revendication 10, dans lequel le virus canarypox est ALVAC.

12. Vaccin pour l'utilisation selon l'une quelconque des revendications 1 à 11, dans lequel ledit vaccin est pour une utilisation dans la réduction de la durée d'excrétion du virus de la grippe.

13. Vaccin pour l'utilisation selon l'une quelconque des revendications 1 à 11, dans lequel ledit vaccin est pour une utilisation dans la réduction de l'excrétion du virus de la grippe dans la cavité nasale.
